(19) Europäisches Patentamt
European Patent Office
Office européen des brevets

(11) **EP 4 242 250 A1**

(12) **EUROPEAN PATENT APPLICATION**

(43) Date of publication:
**13.09.2023 Bulletin 2023/37**

(21) Application number: **22160866.4**

(22) Date of filing: **08.03.2022**

(51) International Patent Classification (IPC):
***C08G 65/336*** *(2006.01)* ***A61K 49/18*** *(2006.01)*
***A61K 51/06*** *(2006.01)* ***C09D 171/02*** *(2006.01)*

(52) Cooperative Patent Classification (CPC):
**C08G 65/336; A61K 51/065; A61K 51/1248; C09D 171/02**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Spago Nanomedical AB**
**223 63 Lund (SE)**

(72) Inventors:
- **AXELSSON, Oskar**
**243 32 HÖÖR (SE)**
- **EKENGARD, Erik**
**212 31 MALMÖ (SE)**
- **LIU, Yi-Chi**
**224 71 LUND (SE)**
- **LARSSON, Rikard**
**268 75 TÅGARP (SE)**
- **PARIS, Juraj**
**211 28 MALMÖ (SE)**

(74) Representative: **Ström & Gulliksson AB**
**P.O. Box 793**
**220 07 Lund (SE)**

(54) **COMPOUNDS FOR COATING OF NANOSTRUCTURES**

(57) The present disclosure relates to a compound according to Formula (I) and to a method for producing the compound as well as to the use of the compound as an intermediate in the production of coated nanostructures. The present disclosure also relates to such nanostructures and to the use of such nanostructures as carriers of radionuclides as well as pharmaceutical compositions comprising such nanostructures.

Fig. 13

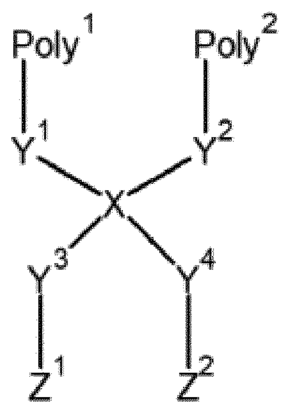

(I)

EP 4 242 250 A1

## Description

### TECHNICAL FIELD

The present disclosure relates to compounds suitable for coating of polymeric nanostructures with applications as intermediates in the production of nanostructures with applications in systemic radiotherapy and imaging.

### BACKGROUND

Polymer coatings, such as PEG-containing polymer coatings, can be used to prevent aggregation of and improve biocompatibility of nanostructures. While PEG remains the polymer of choice, other polymers, such as e.g. polyvinylpyrrolidones and polyamides, suitable for coating nanostructures for biomedical applications have been described.

WO2018130713 describes dipodal structures with one or more short PEG-chains. The structures have a cyclic core, such as an aromatic core, which proved to give rise to some impurities during large scale production.

In WO2021122859 this problem is described in detail and a method to remove the impurities is presented. However, this method includes several purification steps, which increases the production costs and may also influence the quality of the final pharmaceutical product comprising the coated nanostructures.

The object of the present disclosure is to overcome these problems.

### SUMMARY

According to a first aspect, the above and other objects are achieved in full or at least in part by a compound as defined in claim 1. According to this claim, the above object is achieved by a compound according to Formula (I):

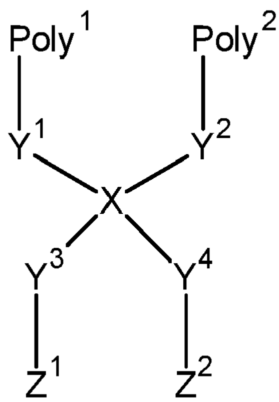

(I)

wherein $Poly^1$ and $Poly^2$ independently are selected from the group consisting of a hydrogen and a hydrophilic polymer group having a molecular weight of 400 to 10 000 Da; wherein at least one of $Poly^1$ or $Poly^2$ is a hydrophilic polymer group having a molecular weight of 400 to 10 000 Da. $Y^1$ and $Y^2$ are independently $-A(CH_2)_nB-$; wherein A is bound to X; B in $Y^1$ is bound to $Poly^1$; B in $Y^2$ is bound to $Poly^2$; n is an integer between 0 and 5; A is chosen from the group consisting of -O-, -S-, -NH-, -C(=O)O-, -C(=O)NH-, -O(C=O)-, -NH(C=O)- and a covalent bond; and B is chosen from the group consisting of -O-, -S-, -NH-, -C(=O)O-, -C(=O)NH-, -O(C=O)-, -NH(C=O)- and a covalent bond, provided that B is a covalent bond when the atom in $Poly^1$ or $Poly^2$ that is bound to $Y^1$ or $Y^2$ is not a carbon atom. $Y^3$ and $Y^4$ independently are $-E(CH_2)_m-$; wherein E is bound to X; m is an integer between 2 and 5; and E is independently chosen from the group consisting of -S-, -NH-, -C(=O)O-, -C(=O)NH-, -O(C=O)-, -NH(C=O)- and a covalent bond. X is chosen from the group consisting of structures $X^1$, $X^2$, $X^3$, $X^4$, and $X^5$

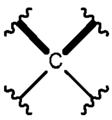

$X^1$

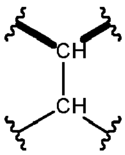

$X^2$

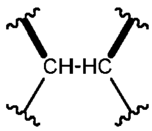

$X^3$

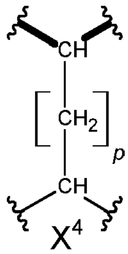

$X^4$

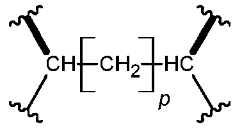

$X^5$

wherein bold bonds represent bonds to $Y^1$ or $Y^2$; non-bold bonds represent bonds to $Y^3$ or $Y^4$; and p is an integer between 1 and 5. $Z^1$ and $Z^2$ independently are $-SiR^1R^2R^3$, wherein $R^1$, $R^2$, and $R^3$ are independently chosen from the group consisting of chloride, bromide, iodide, lower alkoxy, aryloxy, carboxy, amino, and -NH-acyl.

These compounds (also referred to as coating precursors) can be applied as a layer on nanoparticle surfaces to extend the circulation of nanoparticles in the blood (plasma half-life) and allow a sufficient time to bind to cells in targeted tissues. Furthermore, such coated nanoparticles have an extended stability.

Such compounds have the advantage that, after synthesis of the compound, no extra purification steps are required, which is beneficial for the cost and quality of a pharmaceutical product comprising nanostructures coated with the coating precursor.

$Poly^1$ and $Poly^2$ may comprise ethylene glycol units. Alternatively, $Poly^1$ and $Poly^2$ may comprise other hydrophilic polymers, such as e.g. polyvinylpyrrolidones and polyamides. Such, polymers, although more chemically sensitive to e.g. hydrolysis, than polyethylene glycol, may be used in certain cases, since these groups generally are less immunogenic than polyethylene glycol.

$Poly^1$ and $Poly^2$ may comprise 10 to 200 ethylene glycol units. Several ethylene glycol units joined together is also referred to as a polyethylene glycol chain or a PEG chain. $Poly^1$ and $Poly^2$ may comprise 12 to 170 ethylene glycol units, such as between 15 and 120, such as 20 and 90, such as between 30 and 75, such as between 35 and 60, such as between 40 and 50, such as between 42 and 48. $Poly^1$ and $Poly^2$ may comprise 12 to 30 ethylene glycol units, such as between 20 and 50, such as between 30 and 60, such as between 50 and 90, such as between 60 and 120, such as between 75 and 170.

Preferably, $Poly^1$ and $Poly^2$ comprises 30 to 60 ethylene glycol units, even more preferably, 40 to 50 ethylene glycol units.

Optionally, $Poly^1$ and $Poly^2$, especially when comprising ethylene glycol units, may be terminated by a capping group, such as a lower alkyl group. Thus, in one embodiment, $Poly^1$ and $Poly^2$ comprise 30 to 60 ethylene glycol units, such as 40 to 50 ethylene glycol units, and is terminated by a capping group, the capping group being a methyl group or an ethyl group.

According to one embodiment, in both $Y^1$ and $Y^2$, A is a covalent bond, B is -O-, and n is an integer between 1 and 3. Such compounds are stable and synthetically accessible.

According to another embodiment, in both $Y^3$ and $Y^4$, E is a covalent bond. Such compounds are stable and synthetically accessible

According to a further embodiment, the hydrophilic polymer group comprises a PEG chain. Optionally, the PEG chain is terminated with a lower alkyl.

According to yet another embodiment, each hydrophilic polymer group comprises 20 to 150 ethylene glycol residues, preferably between 30 and 60, such as between 40 and 50, ethylene glycol groups. Such compounds are resistant to interactions with plasma proteins and have long plasma half-lives and are synthetically accessible.

According to a further embodiment, $Poly^1$ and $Poly^2$ are $\omega$-methyl-(ethyleneoxy)$_w$, wherein w is 20 to 150; in $Y^1$ and $Y^2$, A is a covalent bond, B is -O-, and n is 1; $Y^3$ and $Y^4$ are both $-CH_2-CH_2-CH_2-$; X is $X^1$; and $Z^1$ and $Z^2$ are independently chosen from the group consisting of triethoxysilyl and trimethoxysilyl. Such compounds are synthetically accessible, have a convenient reactivity when used as coatings, and yield coated nanoparticles with favorable in-vivo properties. Preferably, w is 30 to 60, end even more preferred, w is 40 to 50.

A second aspect of the present disclosure relates to a compound according to Formula (II):

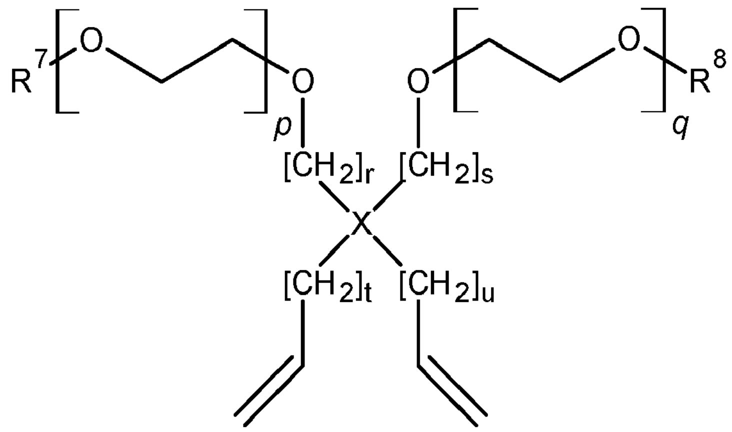

(II)

wherein $R^7$ and $R^8$ are independently chosen from the group consisting of lower alkyls; p is an integer between 20 and 150; q is an integer between 20 and 150; r is an integer between 1 and 3; s is an integer between 1 and 3; t is an integer between 0 and 3; u is an integer between 0 and 3. In Formula II) X is chosen from the group consisting of $X^1$, $X^2$, $X^3$, $X^4$, and $X^5$:

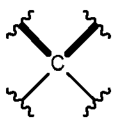

$X^1$

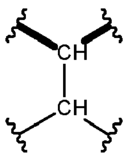

$X^2$

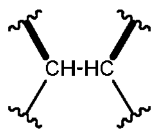

$X^3$

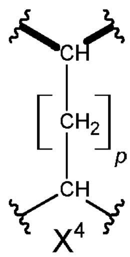

$X^4$

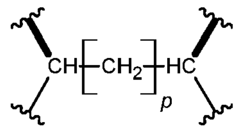

$X^5$

wherein bold bonds represent bonds to $[CH_2]_r$ and $[CH_2]_q$; non-bold bonds represent bonds to $[CH_2]_t$ and $[CH_2]_u$; and p is an integer between 1 and 5.

Preferably, p is an integer between 30 to 60, such as between 40 to 50.

Preferably, q is an integer between 30 to 60, such as between 40 to 50.

Preferably, the lower alkyls are methyl and/or ethyl.

In a third aspect, the present disclosure relates to the use of the compound according to Formula (II) as defined above in the production of a compound according to Formula (I) as defined above wherein the hydrophilic polymer group in Formula (I) comprises a PEG chain.

In a fourth aspect, the present disclosure relates to a method of purification of the compound according to Formula (II) as defined above, comprising the steps of:

a) providing an aqueous solution of an impure compound according to Formula (II) as defined above, wherein the aqueous solution comprises:

water in an amount of 7.5 to 16.5 times the total mass of the impure compound according to Formula (II) as defined above; and
NaCl in an amount of 6% to 9% (w/v) of the amount of water;

b) subjecting the aqueous solution of step a) to between 2 and 5 intermediate extractions, performed at a temperature between 40 °C and 70 °C, wherein each intermediate extraction comprises the steps of:

b1) optionally; adding a further portion of NaCl so that the total amount of NaCl added corresponds to an amount of NaCl less than 9% (w/v) of the amount of water in the aqueous solution of step a);
b2) extracting the aqueous solution with a carboxylate ester solvent; and
b3) removing the organic phase, thereby providing an aqueous phase;

c) adding NaCl to the aqueous phase from step b3) in an amount of at least 1 % of the amount of water in step a),

so that the total amount of NaCl corresponds to an amount of NaCl between 8% and 12% and subjecting the aqueous phase to between 2 and 5 product extractions, performed at a temperature between 40 °C and 70 °C, wherein each product extraction comprises the steps of:

c1) extracting the aqueous phase with a carboxylate ester solvent; and
c2) removing the organic phase;

d) pooling the organic phases from each step c2);
e) concentrating the pooled organic phases from step d), thereby obtaining a residue;
f) dissolving the residue from step e) in an aqueous buffer having a pH of between 6 and 9 to provide an aqueous phase;
g) subjecting the aqueous phase from step f) to 2 to 4 polishing extractions, wherein each polishing extraction comprises the steps of:

g1) extracting the aqueous phase from step f) with a chlorinated solvent; and
g2) removing the organic phase;

h) pooling the organic phases from each step g2); and
i) concentrating the pooled organic phases from step h), thereby obtaining a residue, comprising a diPEGylated diene according to Formula (II) as defined above, comprising less than 10 % (w/w) impurities.

A compound according to Formula (II) as defined above and purified according to the method described herein may be used to produce a compound according to Formula (I) having a high purity. Thus, no extra purification steps are required, which is beneficial for the cost and also of the quality of the produced coating precursor and of the nanostructures coated with the coating precursor.
The amount of impurities as stated above may be measured by HPLC ELSD.
The temperature in step b) may be between 50 °C to 70 °C, preferably, 55 °C to 65 °C, such as 60 °C. At these temperatures, it has been shown that the emulsion that forms at lower temperatures breaks and allows for a clean separation of the phases.
In step c1), the carboxylate ester may be ethyl acetate or isopropyl acetate. Such carboxylate esters are good solvents for the extracted species have boiling points that allows working at the desired temperature while still being practical to remove by evaporation.
After step d), the organic phases may be subjected to a step of drying prior to concentration (step e). This can be realized by a number of methods known to one skilled in the art, such as by drying over a drying agent such as magnesium sulphate or molecular sieves. A step of drying is desirable since the following step in the synthesis generally requires water-free conditions.
The aqueous buffer in step f) is preferably aqueous sodium bicarbonate, since it achieves the desired pH in a convenient and economical way.
In step g1), the chlorinated solvent may dichloromethane, chloroform, tetrachloroethane. Such chlorinated solvents are preferred since they have a high affinity for PEGylated species.
Preferably, the chlorinated solvent is dichloromethane.
In a fourth aspect, the present disclosure relates to a method for producing the compound according to Formula (I) as defined above, wherein both $Poly^1$ and $Poly^2$ comprise a PEG chain and wherein each PEG chain is terminated with a lower alkyl, the method comprising the steps of:

i) providing a diene according to Formula (II) as defined above, optionally purified as described above;
ii) contacting the diene with at least 30 equivalents of a hydrosilylation reagent having the structure $HSiR^1R^2R^3$, wherein $R^1$, $R^2$, and $R^3$ are independently chosen from the group consisting of lower alkoxy-groups, in the presence of a platinum catalyst and an aromatic hydrocarbon solvent at a temperature of between 10 and 35 °C;
iii) removing the excess of the hydrosilation reagent; and
iv) removing platinum from the product.

Thus, this method can be used for the synthesis of silylated dipodal structures comprising one or more poly(ethylene glycol) methyl ether chains.
Importantly, no extra purification steps are required, which is beneficial for the cost and also of the quality of the produced coating precursor and of the nanostructures coated with the coating precursor.
In step ii), the alkoxy-groups are preferably methoxy-groups and/or ethoxy-groups. Such compounds have a suitable reactivity when used to coat nanoparticles while still being stable enough to allow easy handling.

Preferably, the alkoxy-groups in step ii) are methoxy and/or ethoxy.

Preferably, step iii) is carried out after step ii) after a time period when the reaction has proceeded essentially to completion, such as 24 hours.

In a fifth aspect, the present disclosure relates to the use of a compound according to Formula (I) as described above or a compound according to Formula (II) as defined above, or a product of any one of the methods described above, as an intermediate in the production of coated nanostructures. Preferably, the compound used comprises 30 to 60, such as 50 to 50 ethylene glycol groups.

In a sixth aspect, the present disclosure relates to a globular nanostructure having a hydrodynamic diameter between 10 and 100 nm, wherein the nanostructure has a coating derived from a compound according to Formula (I) as described above or a compound according to Formula (II) as defined above, or a product of any one of the methods described above.

Such nanostructures may be used in the treatment of cancer and in imaging.

The hydrodynamic diameter may be 10 nm and 100 nm, such as between 12 nm and 80 nm, such as between 14 nm and 60 nm, such as between 16 nm 50 nm, such as between 18 nm and 45 nm, such as between 20 nm and 40 nm, such as between 25 nm and 35 nm.

The nanostructure may comprise chelating groups. The chelating groups may be bisphosphonates, preferably germinal bisphosphonates.

According to one embodiment, the globular further comprises a radionuclide. The radionuclide may be suitable for medicinal imaging, such as a radionuclide suitable for PET or a radionuclide suitable for SPECT, and/or a radionuclide suitable for radionuclide therapy.

The radionuclide may be $^{177}$Lu, $^{153}$Sm and/or $^{90}$Y.

In a seventh aspect, the present disclosure relates to a pharmaceutical composition comprising a plurality of globular nanostructures as described above. The nanostructures may comprise a radionuclide. Thus, both "empty" nanostructures, i.e. nanostructures not carrying a radionuclide and/or "loaded" nanostructures, i.e. nanostructures carrying a radionuclide, may be comprised in the pharmaceutical composition.

In an eighth aspect, the present disclosure relates to a pharmaceutical composition for use in the treatment of cancer or in imaging, wherein the pharmaceutical composition comprises a plurality of globular nanostructures comprising a radionuclide. The radionuclide may be suitable for medicinal imaging, such as a radionuclide suitable for PET or a radionuclide suitable for SPECT, and/or a radionuclide suitable for radionuclide therapy.

In a ninth aspect, the present disclosure relates to the use of a globular nanostructure according to the present disclosure as a carrier of radionuclides. The radionuclide may be suitable for medicinal imaging, such as a radionuclide suitable for PET or a radionuclide suitable for SPECT, and/or a radionuclide suitable for radionuclide therapy. Specifically, the radionuclide may be $^{177}$Lu, $^{153}$Sm and/or $^{90}$Y.

Other objectives, features and advantages of the present disclosure will appear from the following detailed description, from the experimental data, as well as from the attached claims. It is noted that the disclosure relates to all possible combinations of features.

Generally, all terms used in the claims are to be interpreted according to their ordinary meaning in the technical field, unless explicitly defined otherwise herein. All references to "a/an/the [component, means, step, etc.]" are to be interpreted openly as referring to at least one instance of said component, means, step, etc., unless explicitly stated otherwise. The steps of any method disclosed herein do not have to be performed in the exact order disclosed, unless explicitly stated.

As used herein, the term "comprising" and variations of that term are not intended to exclude other additives, components, integers or steps.

## DEFINITION OF TERMS

As used herein, a chemical bond terminated in a squiggly line perpendicular to the bond indicate that the bond connects the structure shown to some part of the structure of a chemical entity not shown in the structure. As illustration,

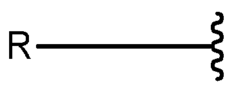

indicates that R is bound to some part of the structure of a chemical entity not shown in the structure.

The term "nanostructure" as used herein relates to an entity with a total size in the nanorange, i.e. up to 100 nm.

The term "globular" as used herein is meant to describe a shape such that the minor axis is no less than half of the major axis, i.e. the longest axis through the center (point of weight) of the structure is no more than twice the length of the shortest axis through the same point. For an explanatory illustration, not limiting this definition, see Fig. 1.

The term "globular nanostructure" as used herein relates to a nanostructure as discussed above having an essentially globular form or shape. This means that shapes such as flakes, rods, tubes, toroids, chains and ribbons are excluded.

The term "monomer" as used herein is a molecule that may bind covalently to other molecules of the same kind, (and optionally, other kinds) to form a polymer that is a macromolecule composed of several monomer residues.

The term "monomer residue" as used herein refers to the atoms derived from one monomer unit as incorporated into the larger polymer. Depending on how the monomers link up, all the atoms may be retained or some may be lost as the bonds were formed.

The term "polymer group" as used herein refers to a polymeric part of a chemical structure, that is, the group comprises multiple monomer residues. The monomer residues may be the same or different. The polymer group may be straight or branched. The ends of the polymer group may comprise different structure than the rest of the polymer group. Often it is clear to one skilled in the art how a polymer group binds to the rest of the chemical structure that the polymer group forms part of. For some polymer groups with the capacity to form bonds to the rest of the chemical structure that the polymer group forms part of in multiple ways it may be necessary to indicate how the polymer group is bound to the rest of the chemical structure.

The term "hydrophilic polymer" as used herein refers to a polymer or polymer group having repeating monomer units with a ratio of (oxygen + nitrogen / total number of non-hydrogen atoms) higher than or equal to 0.25.

The term "PEG" as used herein refer to the polymer polyethylene glycol, represented by the structure

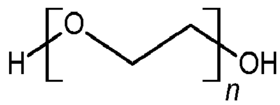

or to any polymer or polymer group comprising polyethylene glycol residues, described of the structure

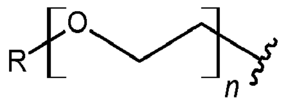

.

The term "mPEG" as used herein refer to the polymer polyethylene glycol methyl ether, represented by the structure

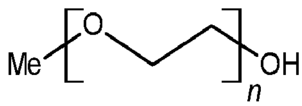

or to a polymer group of the structure

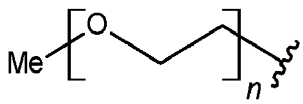

.

The term "alkyl" as used herein includes substituted and unsubstituted saturated straight -chain hydrocarbon group. The alkyl group may in the present text have 1-10 carbon atoms. Typical alkyl groups include, but are in no way limited to, methyl, ethyl, propyl, butyl, pentyl, hexyl and the like.

The term "alkoxy" as used herein refers to the formula -OR wherein R is a lower alkyl, e.g. methoxy, ethoxy, n-propoxy, 1-methyl ethoxy (isopropoxy), n-butoxy, iso-butoxy, sec-butoxy, tert-butoxy, amyloxy, isoamyloxy and the like. An alkoxy group in accordance with the present disclosure may be optionally substituted.

The term "coating" as used herein refers to a layer of material applied to a surface to confer to said surface some property or properties. Often the coating is covalently attached to the surface. Often the surface is the surface of a nanostructure. Often the properties conferred to the surface comprise inertness, such as bio-inertness. The word "coating" can also refer to the process of applying coating to a surface, and the word "coat" can be used as a verb with the meaning "apply coating (to)". Which form of the word "coating" is used in a specific instant is generally clear to one skilled in the art.

The term "organosilane" as used herein refers to organic compounds containing one or more carbon-silicon bond(s).

The term "alkoxysilane" as used herein refers to an organic group bonded to silicon through an oxygen atom with the formula Si-OR wherein R is a lower alkyl, e.g. methoxy, ethoxy, n-propoxy, 1 -methyl ethoxy (isopropoxy), n-butoxy, iso-butoxy, sec-butoxy, tert-butoxy, amyloxy, isoamyloxy and the like.

The term "hydrodynamic diameter" as used herein refers to the diameter of the hypothetical hard sphere that diffuses at the same speed as the nanostructure in solution, i.e. the diameter of the equivalent hard sphere as calculated from the diffusion coefficient, according to the Stokes-Einstein equation. The term is also known as "Stokes diameter" or "Stokes-Einstein diameter". Hydration and shape are included in the behavior of the sphere. The diffusion coefficient is in turn calculated from e.g. the time dependent light scattering data obtained by the Dynamic Light Scattering (DLS) technique. Other technical methods to measure the diffusion coefficient of nanostructures are known to one skilled in the art and may be used instead. In those cases, measurements need t be referenced to the DLS-measurement. As a comparison, bovine serum albumin is measured to have a hydrodynamic diameter of 6.9 nm by DLS in aqueous saline at pH 7 and room temperature. Depending on whether the number average volume average, or scattered intensity average is used, the calculated values may be somewhat different. The volume average is generally the most useful since it shows which nanostructure size the bulk if the material has. The average diameters referred to in this text refer to volume averages as measured at 25 °C in aqueous saline or in 8 % ethylene glycol (v/v) in saline, i.e. volume average hydrodynamic diameter.

The term "DLS" as used herein is an acronym for Dynamic Light Scattering, a particle sizing method, and may also be referred to as Photon Correlation Spectroscopy or Quasi-Elastic Light Scattering. The DLS sizes given as stated in the text and in the claims, if nothing else is specified, refers to the average of the volume weighted peak for a sample measured at 25 °C in aqueous solution with an ionic strength corresponding to 150 mM NaCl, also called saline, or in 8 % ethylene glycol (v/v) in saline.

Impurities in the product of any method described herein are, unless otherwise stated, given as impurities as measured by HPLC ELSD.

The term "HPLC ELSD" as used herein is an acronym for High Pressure Liquid Chromatography Evaporative Light Scattering Detector.

The term "dispersity" as used herein is a measure of the width of the size distribution of a sample of macromolecules or nanostructures. For most applications it is preferable to have as narrow a distribution of sizes as possible around the average i.e. a low dispersity. For a sample with completely uniform size the dispersity is 1 and for non-uniform samples it will be larger than 1. The dispersity can be calculated in different ways, either as the ratio of Mw/Mn or similarly $M_v/M_n$, where Mw is the weight-average molecular weight, $M_n$ is number-average molecular weight, and $M_v$ is the volume-average molecular weight. Herein we analogously define dispersity, $Đ_d$, for a sample of nanostructures as $Đ_d = d_v/d_n$ were $d_v$ is the volume-average diameter as measured by DLS and $d_n$ is the number average diameter as measured by DLS.

## BRIEF DESCRIPTION OF THE DRAWINGS

By way of example, embodiments of the present teaching will now be described with reference to the accompanying drawings, in which:

Fig. 1 is an illustration of a globular object, showing minor and major axels.
Fig. 2 is an illustration of the structure of a coated nanostructure, comprising a central part, an intermediate layer and a coating layer.
Fig. 3 shows a process scheme for the extractive purification process.
Fig. 4 shows a process scheme for the preliminary extraction.
Fig. 5 shows a process scheme for the intermediate extraction.
Fig. 6 shows a process scheme for the product extraction.
Fig. 7 shows a process scheme for the product polishing.
Fig. 8 is an illustration of extractive purification, showing movement of di-PEGylated diene through the extraction.
Fig. 9 shows an example composition of a quenched reaction mixture from the synthesis of di-PEGylated diene 9b.
Fig. 10 shows a HPLC-ELSD chromatogram of the reaction mixture from the synthesis of di-PEGylated diene 9 before, A, and after, B, extractive purification.
Fig. 11 is an illustration of a coating precursor reacted with the surface a nanostructure.
Fig. 12 is a graph of tumor growth for treatment (diamonds) and control (squares) groups from Example 17.
Fig. 13 shows a compound according to Formula (I).

## DETAILED DESCRIPTION

The present disclosure relates to chemical compounds, henceforth called "coating precursors", with the ability to form a dense coating layer on nanostructures. In particular, they are suitable to form dense coatings on nanostructures having silanol groups available on their surface.

The compounds according to the present disclosure provide significant improvements over the prior art in that they can be synthesized at large scale in high purity as shown in Example 3.

These compounds further provide significant improvements over prior art in forming the coating of nanostructures. As shown in Example 16, nanostructures coated with coating precursors according to the present disclosure have higher storage stability and more favorable in *vivo*-properties as compared to nanostructures coated with coating precursors outside the scope of the present disclosure.

Furthermore, nanostructures coated with coating precursors according to the present disclosure have been shown to have an effect in the treatment of tumors (see Example 17).

The coating precursors and the resulting coating comprise polymer groups. As is known to one skilled in the art, polymers synthesized by the vast majority of methods are not identical chemical entities, but rather comprise a plurality of polymer molecules with different degree of polymerization, crosslinking, and branching. The degree of polymerization can be expressed as the number of monomer residues in the polymer, or alternatively be expressed as chain length or by the molecular weight of the polymer. The polymer groups of the present disclosure are, unless otherwise specified, present as mixtures of polymer groups with different degrees of polymerization.

The coating precursors according to the present disclosure comprise reactive silane functionalities. This renders the coating precursors capable of forming bonds to surfaces comprising functional groups capable of forming bonds of the structure G-O-Si, where G represents an atom in the surface, such as a silicon atom, and Si represents a silicon atom from a coating precursor molecule.

When the degree of polymerization is given as a range it is understood by one skilled in the art that this signifies that a large portion, such as a majority of, such as more than 80 %, or such as more than 90 %, or such as more than 95 %, of the polymer molecules have degrees of polymerization falling within the given range. It is also understood that some small fraction of the polymer groups present can have degrees of polymerization that is outside of the given range, and that this might include both higher and lower degrees of polymerization than the given range. When the degree of polymerization is given as a single value it is understood, unless otherwise specified, that the value signifies the average or typical degree of polymerization.

***Coating precursors (compounds according to the present disclosure)***

The present disclosure relates to a compound according to Formula (I):

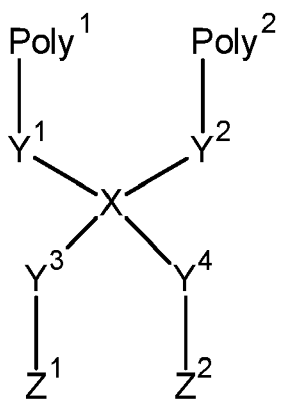

(I)

wherein

Poly$^1$ and Poly$^2$ independently are selected from the group consisting of a hydrogen and a hydrophilic polymer group having a molecular weight of 400 to 10 000 Da; wherein at least one of Poly$^1$ or Poly$^2$ is a hydrophilic polymer group having a molecular weight of 400 to 10 000 Da;

$Y^1$ and $Y^2$ independently are $-A(CH_2)_nB-$; wherein

A is bound to X;

B in $Y^1$ is bound to $Poly^1$;
B in $Y^2$ is bound to $Poly^2$;
n is an integer between 0 and 5;
A is chosen from the group consisting of -O-, -S-, -NH-, -C(=O)O-, -C(=O)NH-, -O(C=O)-, -NH(C=O)- and a covalent bond; and
B is chosen from the group consisting of -O-, -S-, -NH-, -C(=O)O-, -C(=O)NH-, -O(C=O)-, -NH(C=O)- and a covalent bond, provided that B is a covalent bond when the atom in $Poly^1$ or $Poly^2$ that is bound to $Y^1$ or $Y^2$ is not a carbon atom;

$Y^3$ and $Y^4$ independently are $-E(CH_2)_m-$; wherein

E is bound to X;
m is an integer between 2 and 5; and
E is independently chosen from the group consisting of -S-, -NH-, -C(=O)O-, -C(=O)NH-, -O(C=O)-, -NH(C=O)- and a covalent bond; and

X is chosen from the group consisting of structures $X^1$, $X^2$, $X^3$, $X^4$, and $X^5$

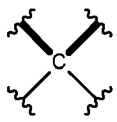
$X^1$

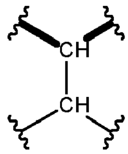
$X^2$

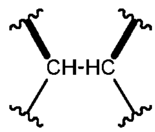
$X^3$

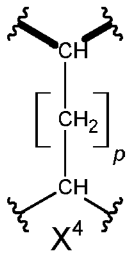
$X^4$

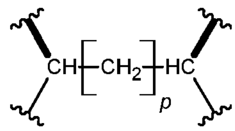
$X^5$

wherein

bold bonds represent bonds to $Y^1$ or $Y^2$;
non-bold bonds represent bonds to $Y^3$ or $Y^4$; and
p is an integer between 1 and 5;

and
$Z^1$ and $Z^2$ independently are $-SiR^1R^2R^3$, wherein
$R^1$, $R^2$, and $R^3$ are independently chosen from the group consisting of chloride, bromide, iodide, lower alkoxy, aryloxy, carboxy, amino, and -NH-acyl.

If, when considering the structure above, two or more covalent bonds are connected in series, they should together be interpreted as one bond.

## *$Poly^1$ and* $Poly^2$

$Poly^1$ and/or $Poly^2$ may comprise a capping group suitable for further derivatization, such groups include carboxylic acids, amines, activated esters, azides, thiols, N-succinimides, epoxides, double bonds, and alkynes.
$Poly^1$ and/or $Poly^2$ may comprise poly-ethers.
Preferably, $Poly^1$ and/or $Poly^2$ comprise polyethylene glycol. $Poly^1$ and $Poly^2$ may comprise, on average, 10-200 ethylene glycol units, such as between 12 and 30, or between 20 and 50, or between 30 and 60, or between 50 and 90, or between 60 and 120, or between 75 and 170.
The polyethylene glycol chain may be terminated by a hydroxyl group or by a lower alkyl group, such as methyl or ethyl.
Alternatively, the polyethylene glycol chain may be terminated by a carboxylate ester group, such as a $-CH_2-C(O)-O-$*t*Bu group, an amide group.
In a preferred embodiment, each polyethylene glycol chain contains between 20 and 150 ethylene glycol residues and each polyethylene glycol chain is terminated by a methyl group.
In a particular embodiment, $Poly^1$ and $Poly^2$ comprise polyethylene glycol, and each polyethylene glycol chain is terminated by a methyl group, and the number of ethylene glycol residues in each polymer group is between 12 and

170, such as between 12 and 30, or between 20 and 50, or between 30 and 60, or between 40 and 50, or between 50 and 90, or between 60 and 120, or between 75 and 170.

Specifically, $Poly^1$ and $Poly^2$ may comprise polyethylene glycol, wherein each polyethylene glycol chain is terminated by a methyl group, and the average number of ethylene glycol residues in each polymer group is between 42 and 48.

$Poly^1$ and/or $Poly^2$ may also comprise polyethylene glycol in combination with one or more polymers as a block co-polymer. Thus, $Poly^1$ and/or $Poly^2$ may comprise a polymer group derived from the copolymerization of ethylene oxide and other monomers.

In some embodiments, $Poly^1$ and/or $Poly^2$ comprise polyoxazolines, such as polymethyloxazolines or polyethyloxazolines.

In some embodiments, $Poly^1$ and $Poly^2$ comprise polymethyloxazolines, each polymer group terminated with a lower alkyl group and each polymer group comprising between 12 and 120 monomer residues.

In some embodiments, $Poly^1$ and $Poly^2$ comprise polyethyloxazolines, each polymer group terminated with a lower alkyl group and each polymer group comprising between 10 and 100 monomer residues.

In some embodiments, $Poly^1$ and/or $Poly^2$ comprises polyglycerols.

In some embodiments, $Poly^1$ and $Poly^2$ comprise polyglycerols, and the polyglycerols are linear or essentially linear.

In some embodiments, $Poly^1$ and $Poly^2$ comprise polyglycerols, and the polyglycerols are branched, such as hyperbranched.

In some embodiments, $Poly^1$ and/or $Poly^2$ comprise polyvinylpyrrolidones.

In some embodiments, $Poly^1$ and/or $Poly^2$ comprise polyamides.

In some embodiments, $Poly^1$ and $Poly^2$ comprise polyglycine.

In some embodiments, $Poly^1$ and $Poly^2$ comprise polymer groups of the structure -[NH-CH(O-{mPEG})-C(O)]-, wherein mPEG is methyl-terminated polyethylene glycol,

In some embodiments, $Poly^1$ and $Poly^2$ comprise peptoids.

### *$Y^1$ and $Y^2$*

$Y^1$ and $Y^2$ may be identical or different.

In a specific embodiment, A is a covalent bond in both $Y^1$ and $Y^2$, and B is a covalent bond or -O-, and n is 2.

Preferably, A is a covalent bond in both $Y^1$ and $Y^2$, and B is -O-, and n is 1.

In another embodiment, in $Y^1$ A is a covalent bond and B is a covalent bond or -O-, and n is 1; and in $Y^2$ A is a covalent bond and B is a covalent bond or -O- and n is 2.

In yet another embodiment, in $Y^1$ A is a covalent bond and B is a covalent bond or -O- and n is between 1 and 3; and in $Y^2$ A and B are covalent bonds and n is 0, and $Poly^2$ is H.

In a yet a further embodiment, in one or both of $Y^1$ and $Y^2$, A is a covalent bond and B is -O-, -S-, -N-, -C(=O)O-, -C(=O)NH-, -O(C=O)-,or -NH(C=O)-.

In yet another embodiment, in one of $Y^1$ or $Y^2$ A is a covalent bond and in the other of $Y^1$ or $Y^2$ A is -O-, -S-, -N-, -C(=O)O-, -C(=O)NH-, -O(C=O)-,or -NH(C=O)-.

In yet another embodiment, in both of $Y^1$ and $Y^2$ A is -O-, -S-, -N-, -C(=O)O-, -C(=O)NH-, -O(C=O)-, or -NH(C=O)-; and X is $X^3$ or $X^5$.

It is also conceivable for $Y^1$ and/or $Y^2$ to have structures other than those described above. For example, the alkyl part of $Y^1$ and/or $Y^2$ could be further substituted, such as by the alkyl part of $Y^1$ and/or $Y^2$ being a branched alkyl, or substituted with groups such as F or methoxy.

### *$Y^3$ and $Y^4$*

$Y^3$ and $Y^4$ may be identical or different. Specifically, m may be the same or different in $Y^3$ and $Y^4$.

In one embodiment, E is a covalent bond and m is between 2 and 5 in both of $Y^3$ and $Y^4$.

In a specific embodiment E is a covalent bond and m is 3 in both of $Y^3$ and $Y^4$.

In another specific embodiment E is a covalent bond and m is 4 in both of $Y^3$ and $Y^4$.

In yet another specific embodiment E is a covalent bond and m is 5 in both of $Y^3$ and $Y^4$.

Thus, preferably E is a covalent bond in both $Y^3$ and $Y^4$ and m may be the same or different in $Y^3$ and $Y^4$. For example, in a specific embodiment, E is a covalent bond in both $Y^3$ and $Y^4$, and m is between 2 and 5 in $Y^3$, and m is between 2 and 5 in $Y^4$, but not identical the value of m for $Y^3$.

In some embodiments, for one of $Y^3$ and $Y^4$, E is -S-, -NH-, -C(=O)O-, -C(=O)NH-, -O(C=O)-, or -NH(C=O)-, and for the other of $Y^3$ and $Y^4$ E is a covalent bond.

In other embodiments, for both of $Y^3$ and $Y^4$, E is -S-, -NH-, -C(=O)O-, -C(=O)NH-, -O(C=O)-, or -NH(C=O)-;

and X is $X^3$ or $X^5$.

It is also conceivable for the alkyl part of $Y^3$ and/or $Y^4$ to be further substituted, such as by the alkyl part of $Y^3$ and/or $Y^4$ being a branched alkyl, or substituted with groups such as F or methoxy.

It is also conceivable for the alkyl part of $Y^3$ and/or $Y^4$ to contain heteroatoms, such as O or S.

*$Z^1$ and $Z^2$*

In one embodiment, $R^1$, $R^2$, and $R^3$ are halogens in both of $Z^1$ and $Z^2$. The halogen may be Cl and/or Br.

Thus, in a specific embodiment, $R^1$, $R^2$, and $R^3$ are Cl in both of $Z^1$ and $Z^2$.

In another specific embodiment, $R^1$, $R^2$, and $R^3$ are Br in both of $Z^1$ and $Z^2$.

In one embodiment, $R^1$, $R^2$, and $R^3$ are lower alkoxy groups in both of $Z^1$ and $Z^2$. Preferably, the lower alkoxy group is methoxy, ethoxy and/or iso-propyloxy.

Thus, in a specific embodiment, $R^1$, $R^2$, and $R^3$ are methoxy in both of $Z^1$ and $Z^2$.

In in another specific embodiment, $R^1$, $R^2$, and $R^3$ are ethoxy in both of $Z^1$ and $Z^2$.

In a further specific embodiment $R^1$, $R^2$, and $R^3$ are iso-propyloxy in both of $Z^1$ and $Z^2$.

Alternatively, the R-groups on $Z^1$ and $Z^2$ are mixtures of Cl and lower alkoxy.

*Specific coating precursors*

Some non-limiting examples of specific coating precursors are listed below.

Specific combinations of $Y^1$ and $Y^2$ and $Poly^1$ and $Poly^2$

In some embodiments, for both of $Y^1$ and $Y^2$ A is a covalent bond and B is a covalent bond or -O- and n is 1 or 2; and $Poly^1$ and $Poly^2$ comprise poly ethers. The poly ethers are as described above. Preferably, the polyether is a polyethylene glycol.

In some embodiments, for both of $Y^1$ and $Y^2$ A is a covalent bond and B is -O- and n is 1 or 2 and $Poly^1$ and $Poly^2$ comprise polyethylene glycol, each polyethylene glycol chain containing between 20 and 150 ethylene glycol residues and each PEG chain is terminated by a methyl group.

In a preferred embodiment, for both of $Y^1$ and $Y^2$ A is a covalent bond and B is -O- and n is 1, and $Poly^1$ and $Poly^2$ comprises polyethylene glycol, and each polyethylene glycol chain is terminated by a methyl group, and the number of ethylene glycol residues in each polymer group is between 12 and 30, or between 20 and 50, or between 30 and 60, or between 50 and 90, or between 60 and 120, or between 75 and 170.

In one embodiment, for $Y^1$ A is a covalent bond and B is -O- and n is between 1 and 3, and for $Y^2$ A and B are covalent bonds and n is 0, and $Poly^1$ is polyethylene glycol, comprising between 20 and 150 ethylene glycol residues, and terminated by a methyl group, and $Poly^2$ is H.

It is also conceivable for the linkage between either or both of $Y^1$ and $Y^2$ and $Poly^1$ or $Poly^2$ to have other structures than those described above. Such alternative structures might comprise a triazole linkage.

Specific combinations of $Y^1$ and $Y^2$ and $Y^3$ and $Y^4$

In one embodiment, for both of $Y^1$ and $Y^2$ A is a covalent bond and B is -O- and n is 1 or 2, and for both of $Y^3$ and $Y^4$ E is a covalent bond, and m is between 2 and 4.

In a preferred embodiment, for both of $Y^1$ and $Y^2$ A is a covalent bond and B is -O- and n is 1, and for both of $Y^3$ and $Y^4$ E is a covalent bond and m is 3.

Specific combinations of $Y^1$ and $Y^2$, X, $Poly^1$ and $Poly^2$

In one embodiment, for both of $Y^1$ and $Y^2$ A is a covalent bond and B is -O- and n is 1 or 2; X is $X^1$ or $X^2$; $Poly^1$ and $Poly^2$ comprise polyethylene glycol, and each polyethylene glycol chain is terminated by a methyl group, and the number of ethylene glycol residues in each polymer group is between 12 and 30, or between 20 and 50, or between 30 and 60, or between 50 and 90, or between 60 and 120, or between 75 and 170.

In another embodiment, for $Y^1$ and $Y^2$ A is -O-, -S-, -C(=O)O-, or -O(C=O)-, and n is 0 or 2 to 5; X is $X^3$ or $X^5$; $Poly^1$ and $Poly^2$ comprise polyethylene glycol, and each PEG chain is terminated by a methyl group.

In a further embodiment, for $Y^1$ and $Y^2$ A is -C(=O)O- or -O(C=O)-, and n is 0; X is $X^1$, $X^2$ or $X^4$; $Poly^1$ and $Poly^2$ comprise polyethylene glycol, and each PEG chain is terminated by a methyl group.

Specific combinations of $Y^3$ and $Y^4$ and X

In one embodiment, for $Y^3$ and $Y^4$ E is -S-, -N-, -C(=O)O-, -C(=O)NH-, -O(C=O)-, or -NH(C=O)-; and X is $X^3$ or $X^5$.
In another embodiment, for both of $Y^3$ and $Y^4$ E is a covalent bond and m is 3 and X is $X^1$, $X^2$ or $X^4$.

Specific combinations of $Y^1$ and $Y^2$, X, and $Y^3$ and $Y^4$

In one embodiment, for $Y^1$ A is a covalent bond and B is a covalent bond or -O-, n is 1; for $Y^2$ A and B are covalent bonds, n is 0; X is $X^1$; for $Y^3$ E is a covalent bond and m is 3; and for $Y^4$ E is a covalent bond and m is 3.
In another embodiment, for $Y^1$ A is a covalent bond and B is -O-, n is 1; for $Y^2$ A is a covalent bond and B is -O-, n is 1; X is $X^4$ and p is 1; for $Y^3$ E is a covalent bond and m is 3; and for $Y^4$ E is a covalent bond and m is 3.
In a further embodiment, for $Y^1$ A is a covalent bond and B is -O-, n is 1; for $Y^2$ A is a covalent bond and B is -O-, n is 1; X is $X^1$; for $Y^3$ E is a covalent bond and m is 5; and for $Y^4$ E is a covalent bond and m is 5.
In yet another embodiment, for $Y^1$ A is a covalent bond and B is -O-, n is 1; for $Y^2$ A is a covalent bond and B is -O-, n is 1; X is $X^1$; for $Y^3$ E is a covalent bond and m is 5; and for $Y^4$ E is a covalent bond and m is 4.
In a further embodiment, for $Y^1$ A is a covalent bond and B is -O-, n is 1; for $Y^2$ A is a covalent bond and B is -O-, n is 1; X is $X^1$; for $Y^3$ E is a covalent bond and m is 2; and for $Y^4$ E is a covalent bond and m is 2.
In yet another embodiment, for $Y^1$ A is a covalent bond and B is -O-, n is 0; for $Y^2$ A is a covalent bond and B is -O-, n is 0; X is $X^3$; for $Y^3$ E is a covalent bond and m is 2; and for $Y^4$ E is a covalent bond and m is 2.
In one embodiment, for $Y^1$ A is a covalent bond and B is -O-, n is 1; for $Y^2$ A is a covalent bond and B is -O-, n is 1; X is $X^4$ and p is 1; for $Y^3$ E is a covalent bond and m is 4; and for $Y^4$ E is a covalent bond and m is 4.
In a further embodiment, for $Y^1$ A is a covalent bond and B is -O-, n is 1; for $Y^2$ A is a covalent bond and B is -O-, n is 1; X is $X^4$ and p is 1; for $Y^3$ E is a covalent bond and m is 2; and for $Y^4$ E is a covalent bond and m is 2.
In a preferred embodiment, for $Y^1$ A is a covalent bond and B is -O-, n is 1; for $Y^2$ A is a covalent bond and B is -O-, n is 1; X is $X^1$; for $Y^3$ E is a covalent bond and m is 3; and for $Y^4$ E is a covalent bond and m is 3.

Specific combinations of $Y^1$ and $Y^2$, X, $Y^3$ and $Y^4$, and $Poly^1$ and $Poly^2$

In one embodiment, for $Y^1$ A is a covalent bond and B is -O-, n is 1; for $Y^2$ A and B are covalent bonds, n is 0; X is $X^1$; for $Y^3$ E is a covalent bond and m is 3; and for $Y^4$ E is a covalent bond and m is 3; and $Poly^1$ comprises polyethylene glycol, containing between 20 and 150 ethylene glycol residues and terminated by a methyl group; and $Poly^2$ is H.
In another embodiment, for $Y^1$ A is a covalent bond and B is -O-, n is 1 to 3; for $Y^2$ A is a covalent bond and B is -O-, n is 1 to 3 ; X is $X^4$ and p is 1 to 3; for $Y^3$ E is a covalent bond and m is 3 or 4; and for $Y^4$ E is a covalent bond and m is 3 or 4; and $Poly^1$ comprises polyethylene glycol, containing between 20 and 150 ethylene glycol residues and terminated by a methyl group; and $Poly^2$ comprises polyethylene glycol, containing between 20 and 150 ethylene glycol residues and terminated by a methyl group.
In a further embodiment, for $Y^1$ A is a covalent bond and B is -O-, n is 1; for $Y^2$ A is a covalent bond and B is -O-, n is 1; X is $X^1$; for $Y^3$ E is a covalent bond and m is between 2 and 5; and for $Y^4$ E is a covalent bond and m is between 2 and 5; and $Poly^1$ comprises polyethylene glycol, containing between 20 and 150 ethylene glycol residues and terminated by a methyl group; and $Poly^2$ comprises polyethylene glycol, containing between 20 and 150 ethylene glycol residues and terminated by a methyl group.
In yet another embodiment, for $Y^1$ A is a covalent bond and B is -O-, n is 0; for $Y^2$ A is a covalent bond and B is -O-, n is 0; X is $X^3$; for $Y^3$ E is a covalent bond and m is between 2 and 4; and for $Y^4$ E is a covalent bond and m is between 2 and 4; and $Poly^1$ comprises polyethylene glycol, containing between 20 and 150 ethylene glycol residues and terminated by a methyl group; and $Poly^2$ comprises polyethylene glycol, containing between 20 and 150 ethylene glycol residues and terminated by a methyl group.
In a further embodiment, for $Y^1$ A is a covalent bond and B is -O-, n is 1; for $Y^2$ A is a covalent bond and B is -O-, n is 1; X is $X^4$ and p is 1 to 3; for $Y^3$ E is a covalent bond and m is between 2 and 4; and for $Y^4$ E is a covalent bond and m is between 2 and 4; and $Poly^1$ comprises polyethylene glycol, containing between 20 and 150 ethylene glycol residues and terminated by a methyl group; and $Poly^2$ comprises polyethylene glycol, containing between 20 and 150 ethylene glycol residues and terminated by a methyl group.
In a preferred embodiment, for $Y^1$ A is a covalent bond and B is -O-, n is 1; for $Y^2$ A is a covalent bond and B is -O-, n is 1; X is $X^1$; for $Y^3$ E is a covalent bond and m is 3; and for $Y^4$ E is a covalent bond and m is 3; and $Poly^1$ comprises polyethylene glycol, containing between 20 and 150 ethylene glycol residues and terminated by a methyl group; and $Poly^2$ comprises polyethylene glycol, containing between 20 and 150 ethylene glycol residues and terminated by a methyl group.
In another preferred embodiment, for $Y^1$ A is a covalent bond and B is -O-, n is 1; for $Y^2$ A is a covalent bond

and B is -O-, n is 1; X is $X^1$; for $Y^3$ E is a covalent bond and m is 3; and for $Y^4$ E is a covalent bond and m is 3; and $Poly^1$ and $Poly^2$ comprises polyethylene glycol, and each PEG chain is terminated by a methyl group, and the number of ethylene glycol residues in each polymer group is between 12 and 30, or between 20 and 50, or between 30 and 60, or between 50 and 90, or between 60 and 120, or between 75 and 170.

In a further preferred embodiment, for $Y^1$ A is a covalent bond and B is -O-, n is 1; for $Y^2$ A is a covalent bond and B is -O-, n is 1; X is $X^1$; for $Y^3$ E is a covalent bond and m is 3; and for $Y^4$ E is a covalent bond and m is 3; and $Poly^1$ and $Poly^2$ comprises polyethylene glycol, and each PEG chain is terminated by a methyl group, and the average number of ethylene glycol residues in each polymer group is between 42 and 48.

Specific combinations of $Y^1$ and $Y^2$, X, $Y^3$ and $Y^4$, $Poly^1$ and $Poly^2$, $Z^1$ and $Z^2$

In one embodiment, for $Y^1$ A is a covalent bond and B is -O-, n is 1; for $Y^2$ A and B are covalent bonds, n is 0; X is $X^1$; for $Y^3$ E is a covalent bond and m is 3; and for $Y^4$ E is a covalent bond and m is 3; and $Poly^1$ comprises polyethylene glycol, containing between 20 and 150 ethylene glycol residues and terminated by a methyl group; and $Poly^2$ is H; and for $Z^1$ $R^1$, $R^2$, and $R^3$ are chloride or lower alkoxy, such as methoxy or ethoxy; and for $Z^2$ $R^1$, $R^2$, and $R^3$ are chloride or lower alkoxy, such as methoxy or ethoxy.

In another embodiment, for $Y^1$ A is a covalent bond and B is -O-, n is 1 to 3; for $Y^2$ A is a covalent bond and B is -O-, n is 1 to 3 ; X is $X^4$ and p is 1 to 3; for $Y^3$ E is a covalent bond and m is 3 or 4; and for $Y^4$ E is a covalent bond and m is 3 or 4; and $Poly^1$ comprises polyethylene glycol, containing between 20 and 150 ethylene glycol residues and terminated by a methyl group; and $Poly^2$ comprises polyethylene glycol, containing between 20 and 150 ethylene glycol residues and terminated by a methyl group; and for $Z^1$ $R^1$, $R^2$, and $R^3$ are chloride or lower alkoxy, such as methoxy or ethoxy; and $Z^2$ $R^1$, $R^2$, and $R^3$ are chloride or lower alkoxy, such as methoxy or ethoxy.

In a further embodiment, for $Y^1$ A is a covalent bond and B is -O-, n is 1; for $Y^2$ A is a covalent bond and B is -O-, n is 1; X is $X^1$; for $Y^3$ E is a covalent bond and m is between 2 and 5; and for $Y^4$ E is a covalent bond and m is between 2 and 5; and $Poly^1$ comprises polyethylene glycol, containing between 20 and 150 ethylene glycol residues and terminated by a methyl group; and $Poly^2$ comprises polyethylene glycol, containing between 20 and 150 ethylene glycol residues and terminated by a methyl group; and for $Z^1$ $R^1$, $R^2$, and $R^3$ are chloride or lower alkoxy, such as methoxy or ethoxy; and for $Z^2$ $R^1$, $R^2$, and $R^3$ are chloride or lower alkoxy, such as methoxy or ethoxy.

In yet another embodiment, for $Y^1$ A is a covalent bond and B is -O-, n is 0; for $Y^2$ A is a covalent bond and B is -O-, n is 0; X is $X^3$; for $Y^3$ E is a covalent bond and m is between 2 and 4; and for $Y^4$ E is a covalent bond and m is between 2 and 4; and $Poly^1$ comprises polyethylene glycol, containing between 20 and 150 ethylene glycol residues and terminated by a methyl group; and $Poly^2$ comprises polyethylene glycol, containing between 20 and 150 ethylene glycol residues and terminated by a methyl group; and for $Z^1$ $R^1$, $R^2$, and $R^3$ are chloride or lower alkoxy, such as methoxy or ethoxy; and for $Z^2$ $R^1$, $R^2$, and $R^3$ are chloride or lower alkoxy, such as methoxy or ethoxy.

In a further embodiment, for $Y^1$ A is a covalent bond and B is -O-, n is 1; for $Y^2$ A is a covalent bond and B is -O-, n is 1; X is $X^4$ and p is 1 to 3; for $Y^3$ E is a covalent bond and m is between 2 and 4; and for $Y^4$ E is a covalent bond and m is between 2 and 4; and $Poly^1$ comprises polyethylene glycol, containing between 20 and 150 ethylene glycol residues and terminated by a methyl group; and $Poly^2$ comprises polyethylene glycol, containing between 20 and 150 ethylene glycol residues and terminated by a methyl group; and for $Z^1$ $R^1$, $R^2$, and $R^3$ are chloride or lower alkoxy, such as methoxy or ethoxy; and for $Z^2$ $R^1$, $R^2$, and $R^3$ are chloride or lower alkoxy, such as methoxy or ethoxy.

In a preferred embodiment, for $Y^1$ A is a covalent bond and B is -O-, n is 1; for $Y^2$ A is a covalent bond and B is -O-, n is 1; X is $X^1$; for $Y^3$ E is a covalent bond and m is 3; and for $Y^4$ E is a covalent bond and m is 3; and $Poly^1$ comprises polyethylene glycol, containing between 20 and 150 ethylene glycol residues and terminated by a methyl group; and $Poly^2$ comprises polyethylene glycol, containing between 20 and 150 ethylene glycol residues and terminated by a methyl group; and for $Z^1$ $R^1$, $R^2$, and $R^3$ are chloride or lower alkoxy, such as methoxy or ethoxy; and for $Z^2$ $R^1$, $R^2$, and $R^3$ are chloride or lower alkoxy, such as methoxy or ethoxy.

In another preferred embodiment, for $Y^1$ A is a covalent bond and B is -O-, n is 1; for $Y^2$ A is a covalent bond and B is -O-, n is 1; X is $X^1$; for $Y^3$ E is a covalent bond and m is 3; and for $Y^4$ E is a covalent bond and m is 3; and $Poly^1$ and $Poly^2$ comprises polyethylene glycol, and each polyethylene glycol chain is terminated by a methyl group, and the number of ethylene glycol residues in each polymer group is between 12 and 30, or between 20 and 50, or between 30 and 60, or between 50 and 90, or between 60 and 120, or between 75 and 170; and for $Z^1$ $R^1$, $R^2$, and $R^3$ are chloride or lower alkoxy, such as methoxy or ethoxy; and for $Z^2$ $R^1$, $R^2$, and $R^3$ are chloride or lower alkoxy, such as methoxy or ethoxy.

In a further preferred embodiment, for $Y^1$ A is a covalent bond and B is -O-, n is 1; for $Y^2$ A is a covalent bond and B is -O-, n is 1; X is $X^1$; for $Y^3$ E is a covalent bond and m is 3; and for $Y^4$ E is a covalent bond and m is 3; and $Poly^1$ and $Poly^2$ comprises polyethylene glycol, and each PEG chain is terminated by a methyl group, and the average number of ethylene glycol residues in each polymer group is around 45, such as between 42 and 48; and for $Z^1$ $R^1$, $R^2$, and $R^3$ are chloride or lower alkoxy, such as methoxy or ethoxy; and for $Z^2$ $R^1$, $R^2$, and $R^3$ are chloride or lower alkoxy, such

as methoxy or ethoxy.

In one embodiment, for $Y^1$ A and B are covalent bonds, n is 1; for $Y^2$ A and B are covalent bonds, n is 1; X is $X^1$; for $Y^3$ E is a covalent bond and m is 3; and for $Y^4$ E is a covalent bond and m is 3; and $Poly^1$ and $Poly^2$ comprises polyoxazolines, such as polymethyloxazolines or polyethyloxazolines , and each polymer chain is terminated by a lower alkyl group, such as a methyl or ethyl group; and for $Z^1$ $R^1$, $R^2$, and $R^3$ are chloride or lower alkoxy, such as methoxy or ethoxy; and for $Z^2$ $R^1$, $R^2$, and $R^3$ are chloride or lower alkoxy, such as methoxy or ethoxy.

In one preferred embodiment, for $Y^1$ A is a covalent bond and B is - O-, n is 1; for $Y^2$ A is a covalent bond and B is -O-, n is 1; X is $X^1$; for $Y^3$ E is a covalent bond and m is 3; and for $Y^4$ E is a covalent bond and m is 3; and $Poly^1$ and $Poly^2$ are polyethylene glycol, and each polyethylene glycol chain is terminated by a methyl group, and the average number of ethylene glycol residues in each polymer group is around 45, such as between 42 and 48; and for $Z^1$ and $Z^2$ $R^1$, $R^2$, and $R^3$ are ethoxy.

In another preferred embodiment, for $Y^1$ A is a covalent bond and B is -O-, n is 1; for $Y^2$ A is a covalent bond and B is -O-, n is 1; X is $X^1$; for $Y^3$ E is a covalent bond and m is 3; and for $Y^4$ E is a covalent bond and m is 3; and $Poly^1$ and $Poly^2$ are polyethylene glycol, and each polyethylene glycol chain is terminated by a methyl group, and the average number of ethylene glycol residues in each polymer group is between 65 and 72; and for $Z^1$ and $Z^2$ $R^1$, $R^2$, and $R^3$ are ethoxy.

***Method of producing the precursors***

The present disclosure also relates to a method of producing the coating precursors.

The method is useful over a variety of scales, such as from small scale laboratory scale to kilogram production scale and larger, offering a clear advantage over alternative methods suitable only at some limited scale.

When coating precursors of the present disclosure are used to coat nanostructures (described in more detail below), coated nanostructures having e.g. a longer plasma half-life and better storage stability are produced if the coating precursors have a high purity, such as a purity of at least 90 %, than when the coating precursors have a lower purity.

Advantageously, the present method generates coating precursors of a purity such that when the coating precursors are used to coat nanostructures, the resulting coated nanostructures are of high quality with desirable *in vivo* properties and desirable storage stability.

Due to the nature of the coating precursors, purification of the finished coating precursors is difficult. Many commonly employed methods for the purification of chemical compounds are not suitable for the coating precursors of the current disclosure.

As the compounds comprise polymer groups, and the polymer groups are generally present as a multitude of chain lengths, purification through crystallization is difficult. Furthermore, as the compounds comprise polymer groups, the compounds are non-volatile or essentially non-volatile and purification through distillation and/or sublimation is impossible or essentially impossible.

The compounds also comprise reactive silyl groups, making most commonly employed chromatographic purification techniques unsuitable. The reactive silyl groups bind and form strong bonds to surfaces containing silanol functionalities. Chromatography using silica and similar materials as stationary phases, which is generally referred to as normal phase chromatography, is thus difficult as a significant portion of the material to be purified irreversibly binds to the stationary phase and thus cannot be eluted.

The reactive silyl groups are also sensitive to hydrolysis and selfcondensation reactions in the presence of water. Chromatography using hydrophobic materials as stationary phases and aqueous mobile phases, which is generally referred to as reverse phase chromatography, is thus difficult as a significant portion of the material to be purified will hydrolyze and/or oligomerize during the chromatographic procedure.

The method of the current disclosure has the advantage that it generates coating precursors of sufficient purity for the use in the production of high-quality coated nanostructures without the need for other purification of the coating precursor than the removal of catalyst residues and the evaporation of volatile solvents and reagents.

By the method, the introduction of the silyl groups into the coating precursor molecules is efficiently achieved through hydrosilylation of alkene moieties with reagents of the structure $H\text{-}SiR^1R^2R^3$, where $R^1$, $R^2$, and $R^3$ are defined as for Formula (I) above, i.e. $R^1$, $R^2$, and $R^3$ are independently chosen from the group consisting of chloride, bromide, iodide, lower alkoxy, aryloxy, carboxy, amino, and -NH-acyl. It is clear to one skilled in the art that to result in products of the structure of Formula (I), the hydrosilylation reaction has to be performed on a substrate comprising two alkene moieties, i.e. on a diene.

Further, it has been found that the introduction of the polymer groups is most efficiently accomplished prior to the introduction of the silyl groups. The introduction of the silyl groups by hydrosilylation is therefore accomplished by the hydrosilylation of a diene that is a polymer dialkene conjugate.

Hydrosilylation is commonly achieved by the use of a catalyst. The catalyst may be a platinum catalyst, such as a platinum(0) compound, such as Karstedt's catalyst. It is known in the art that when such catalysts are used for

hydrosilylation, several side reactions are possible. Such side reactions include reduction of the alkene double bond and migration of the alkene double bond. It is known that hydrosilylation reactions on internal double bonds are significantly slower than hydrosilylation on terminal double bonds.

Specifically, for the synthesis of coating precursors according to Formula (I) wherein $Poly^1$ and $Poly^2$ are polyethylene glycol, and each polyethylene glycol chain is terminated by a lower alkyl group; and wherein for $Y^1$ A is a covalent bond and B is -O-, and n is between 1 to 3; and for $Y^2$ A is a covalent bond and B is -O-, and n is between 1 to 3; and wherein for $Y^3$ E is a covalent bond and m is between 2 and 5; and for $Y^4$ E is a covalent bond and m is between 2 and 5; and wherein for $Z^1$ $R^1$, $R^2$, and $R^3$ are lower alkoxy; and for $Z^1$ $R^1$, $R^2$, and $R^3$ are lower alkoxy, it is suitable to use hydrosilylation of dienes according to Formula (II):

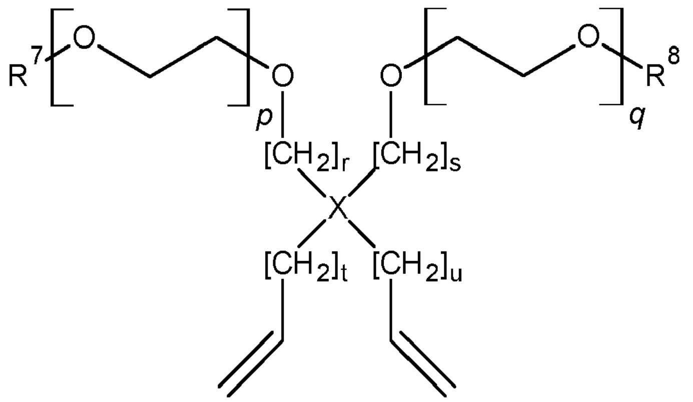

(II)

wherein $R^7$ and $R^8$ are independently chosen to be lower alkyl; p is an integer between 20 and 150; q is an integer between 20 and 150, preferably 30 to 60 or 40-50; r is an integer between 1 and 3; s is an integer between 1 and 3; t is an integer between 0 and 3; u is an integer between 0 and 3; and X is chosen from the group consisting of $X^1$, $X^2$, $X^3$, $X^4$, and $X^5$:

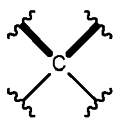
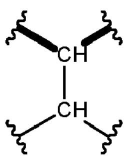
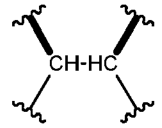
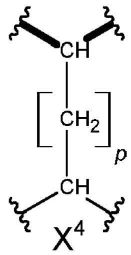
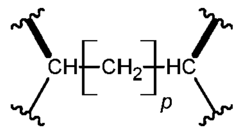

$X^1$ $X^2$ $X^3$ $X^4$ $X^5$

wherein bold bonds represent bonds to $[CH_2]_r$ and $[CH_2]_q$; non-bold bonds represent bonds to $[CH_2]_t$ and $[CH_2]_u$; and p is an integer between 1 and 5.

Thus, the present disclosure also relates to such dienes (described in detail below).

Preferably, p is an integer between 30 to 60, even more preferred between 40 to 50.

Preferably, q is an integer between 30 to 60, even more preferred between 40 to 50.

If, when considering the structure above, two or more covalent bonds are connected in series, they should together be interpreted as one bond.

Notably, when dienes according to Formula (II) are hydrosilylated with hydrosilylation reagents of the structure $HSiR^1R^2R^3$, where $R^1$, $R^2$, and $R^3$ are lower alkoxy, under standard hydrosilylation conditions, significant amounts of impurities are produced. In Example 16 is shown the results of an unsuccessful synthesis using standard hydrosilylation conditions.

Examples of such impurities include the products of hydrogenation of one or both of the double bonds in the structures according to Formula (II), and the products of double bond migration reactions. An example of an attempt to synthesize compound **10** using standard hydrosilylation conditions is given in Example 19, and a chromatogram showing the product composition of example 19 is depicted in Figure 10.

It has been found that when coating precursors containing large amounts byproducts formed from the diene, but comprising only one silyl group, are used to coat nanostructures, the resulting coated nanostructures have inferior properties. Furthermore, the coating precursors can only be separated from the byproducts comprising only one silyl

group by chromatography under chromatographic conditions that are not suitable for large scale production of the coating precursors.

However, surprisingly, when dienes according to Formula (II) are hydrosilylated with hydrosilylation reagents of the structure $HSiR^1R^2R^3$, where $R^1$, $R^2$, and $R^3$ are lower alkoxy, and the hydrosilylation reagents is present in an excess of more than 30 equivalents relative to the diene, the formation of impurities comprising less than 2 silyl groups is significantly reduced.

Thus, the present disclosure also relates to a method for producing a compound according to the present disclosure wherein both $Poly^1$ and $Poly^2$ comprise a PEG chain and wherein each PEG chain is terminated with a lower alkyl, and wherein the hydrophilic polymer group comprises a PEG chain, optionally terminated with a lower alkyl, optionally wherein each hydrophilic polymer group comprises 20 to 150 ethylene glycol residues; or wherein $Poly^1$ and $Poly^2$ are $\omega$-methyl-(ethyleneoxy)$_w$ and w is 20 to 150, preferably 30 to 60; in $Y^1$ and $Y^2$, A is a covalent bond, B is -O-, and n is 1; $Y^3$ and $Y^4$ are both $-CH_2-CH_2-CH_2-$; X is $X^1$; and $Z^1$ and $Z^2$ are independently chosen from the group consisting of triethoxysilyl and trimethoxysilyl.

Specifically, the method comprises the steps of:

i) providing a diene according to Formula (II) as described above, optionally purified as described below;
ii) contacting the diene with at least 30 equivalents of a hydrosilylation reagent having the structure $HSiR^1R^2R^3$, wherein $R^1$, $R^2$, and $R^3$ are independently chosen from the group consisting of lower alkoxy-groups, in the presence of a platinum catalyst and an aromatic hydrocarbon solvent at a temperature of between 10 and 35 °C;
iii) removing the excess of the hydrosilation reagent; and
iv) removing platinum from the product.

When nanostructures are coated with coating precursor prepared in this way, the resulting coated nanostructure will be of a high quality.

The diene provided in step i) may have of a purity of at least 95 %.

Typically, when the diene provided in step i) of the method is of a purity of at least 95 %, the resulting coating precursor is of purity of at least 90%.

Preferably, the alkoxy-groups in step ii) are methoxy and/or ethoxy.

Especially, when the diene used in the hydrosilylation is of a high purity, such as above 90%, such as above 95 %, or such as above 97%, and the hydrosilylation reagent is present in more than 30 equivalents, such as 40 equivalents, the resulting coating precursors are of a high purity, such as above 90%, such as above 95 %. Coating precursors prepared under such conditions contain only limited amounts of byproducts comprising less than 2 silyl groups, such as less than 10 %, or less than 5 %, or less than 3 %, or less than 1 %, of the amount of the coating precursor. When nanostructures are coated with coating precursor prepared in this way, the resulting coated nanostructure will be of an especially high quality.

In Examples 2, 3, 4, 5, 7, 8, and 9 are shown examples of the application of the method to generate coating precursors of high purity.

Alternatively, if the impurities present in the diene are of such a nature that they do not interfere with the use of the coating precursor to coat nanostructures, and of such a nature that they do not interfere with the hydrosilylation, the diene used in the hydrosilylation may be of a lower purity. Impurities that are known to not interfere with neither the use of the coating precursor to coat nanostructures nor the hydrosilylation, include polymers of the structure $mPEG_2O$ and mPEG.

In the diene according to Formula (II), $R^7$ and $R^8$ may be methyl, ethyl, iso-propyl, n-propyl or t-butyl. Preferably, $R^7$ and $R^8$ are methyl,

In the diene according to Formula (II), r and s may be 1.

In the diene according to Formula (II), t and u may be 1.

The solvent used in step ii) may be toluene or xylene, or a mixture comprising toluene or xylene.

Preferably, the solvent in step ii) is toluene or xylene.

Further, it is often advantageous to dry the diene prior to contacting the diene with the hydrosilylation reagent. This is conveniently realized through azeotropic distillation of a solution of the diene in the reaction solvent. The azeotropic distillation can be realized in a vessel that can subsequently be used as the reaction vessel for the hydrosilylation.

The catalyst in step ii) may be a platinum(0) compound, such as Karstedt's catalyst. Alternatively, other platinum catalysts such as $K_2PtCl_6$ can be used.

It is advantageous for the concentration of the diene in step ii) to be high, such as more than 10% (w/v).

A catalyst loading between 0.5 and 3 mol%, such as between 1 and 2 mol%, relative to the diene, is suitable in step ii).

It is often convenient to first contact the diene and the hydrosilylation reagent in the presence of the reaction solvent prior to introduction of the catalyst. This is conveniently realized through the mixing of an azeotropically dried

solution of the diene with the hydrosilylation reagent.

Often, an exotherm is observed on introduction of the catalyst. In such cases, it is convenient to introduce the catalyst slowly, such as by addition in portions or as a slow infusion of the catalyst solution into the solution of the diene and the hydrosilylation reagent.

The hydrosilylation reagent used in step ii) may be trimethoxysilane. triethoxysilane or triisopropoxysilane.

In a specific embodiment, the hydrosilylation reagent used in step ii) is trimethoxysilane.

In another specific embodiment, the hydrosilylation reagent used in step ii) is triethoxysilane.

In a further specific embodiment, the hydrosilylation reagent used in step ii) is triisopropoxysilane.The hydrosilylation reagent used in step ii) may be present in more than 30 equivalents, such as present in 40 equivalents, or such as present in more than 40 equivalents.

The catalyst used in step ii) may be a platinum(0) compound. Preferably, the catalyst used in step ii) is a platinum(0) compound, free of chlorides or essentially free of chlorides.

In one embodiment, the catalyst used in step ii) is Karstedt's' catalyst.

When step ii) is performed at a temperature between 20 to 25 °C, the reaction is often complete or essentially complete within an hour after the addition of the catalyst.

The presence of unreacted hydrosilylation reagent is detrimental to the scavenging of platinum by thiol-derivatized polystyrene resins. Thus, the excess if the hydrosilylation reagent is removed in step iii).

The complete or essentially complete removal of unreacted silane reagent from the coating precursors can be achieved through distillation of the hydrosilylation reagent from the reaction mixture, especially through repeated co-distillation with a solvent. Preferably, the reaction mixture is first concentrated, such as by using a rotary evaporator or such as by vacuum distillation from the reaction vessel, resulting in a residue comprising significant amounts of unreacted silane reagent. The residue is then repeatedly redissolved in an aromatic hydrocarbon solvent, such as toluene or xylene, and the concentration procedure is repeated, each repetition of the dissolution-concentration cycle resulting in a residue with a lower concentration of unreacted hydrosilylation reagent. Often between 3 and 8 cycles of dissolution-concentration are enough to result in a residue that is free of or essentially free of the unreacted hydrosilylation reagent. It is advantageous to perform the repeated dissolution-concentration procedure under dry conditions.

As the coating precursors are non-volatile or essentially non-volatile, separating the coating precursors from the catalyst and residues derived from the catalyst by distillation of the silylated product, as is the standard procedure for many silylated materials, is not feasible. However, there are several ways of performing step iv), some of which are presented below.

Scavenging of platinum from the coating precursors by some material with affinity for platinum and separation of the coating precursors and the scavenging material by some physical method, such as filtration, is a satisfactory method for the removal of platinum from the coating precursors. Especially thiol-derivatized polystyrene resins have been found to be useful for the scavenging of platinum from the coating precursors. Activated charcoal has also been found to be useful. Examples of other scavenging materials are materials such as materials comprising the thiourea group, or materials comprising phosphine groups. Other possible scavenging materials are known to one skilled in the art.

Thus, contacting a toluene solution of the coating precursor with a thiol-derivatized polystyrene resin at between 45 and 75 °C often lowers the concentration of platinum to a very low level, such as to below 10 ppm as measured by ICP-OES, within a few days. If a satisfactory removal of platinum cannot be obtained within a few days, separation of the solution from the thiol-derivatized polystyrene resin and treatment with either fresh thiol-derivatized polystyrene resin or with activated charcoal often results in satisfactory low levels of platinum. If needed, multiple treatments with thiol-derivatized polystyrene resin and/or charcoal can be used.

In one embodiment, the platinum present at the end of the hydrosilylation reaction is removed through scavenging with a thiolated polystyrene resin.

In another embodiment, the platinum present at the end of the hydrosilylation reaction is removed through scavenging with a scavenging material that comprises charcoal, such as activated charcoal.

In a further embodiment, the platinum present at the end of the hydrosilylation reaction is removed through a combination of scavenging with a thiolated polystyrene resin and scavenging with a scavenging material that comprises charcoal.

In examples 2c, 3b, 4b, 5b, 6b, 7d, and 9d are shown several examples of the application of the current method of synthesis of coating precursors.

*Specific embodiments of the method for producing a coating precursor*

Some embodiments of the method use a diene according to Formula (II) where $R^7$ and $R^8$ are methyl, and p and q are between 30 and 60.

Some embodiments of the method use a diene according to Formula (II) where $R^7$ and $R^8$ are methyl, and p and q are between 50 and 70.

Some embodiments of the method use a diene according to Formula (II) where $R^7$ and $R^8$ are methyl, p and q are between 30 and 60, and r and s are 1.

Some embodiments of the method use a diene according to Formula (II) where $R^7$ and $R^8$ are methyl, p and q are between 30 and 60, r and s are 1, and t and u are 1.

Some embodiments of the method use a diene according to Formula (II) where $R^7$ and $R^8$ are methyl, p and q are between 30 and 60, r and s are 1, t and u are 1, and X is $X^1$.

In one embodiments, the hydrosilylation reagent used in step ii) is triethoxysilane and present in 40 equivalents.

In another embodiment, the hydrosilylation reagent used in step ii) is triethoxysilane, and for the diene t and u are 1.

In a further embodiment, the hydrosilylation reagent used in step ii) is triethoxysilane, and for the diene $R^7$ and $R^8$ are methyl, p and q are between 30 and 60, r and s are 1, and t and u are 1.

In yet another embodiment, the hydrosilylation reagent used in step ii) is triethoxysilane, and for the diene $R^7$ and $R^8$ are methyl, p and q are between 30 and 60, r and s are 1, t and u are 1, and X is $X^1$.

In one embodiment, the catalyst used in step ii) is a platinum compound comprising 1,1,3,3-tetramethyl-1,3-divinyldisiloxane and further comprising other ligands coordinated to the platinum.

In a specific embodiment, the hydrosilylation reagent used in step ii) is triethoxysilane, and the triethoxysilane is present at between 35 and 45 equivalents, and for the diene $R^7$ and $R^8$ are methyl, p and q are between 30 and 60, r and s are 1, t and u are 1, and X is $X^1$, and the solvent is toluene.

In another specific embodiment, the hydrosilylation reagent used in step ii) is triethoxysilane, and the triethoxysilane is present at between 35 and 45 equivalents, and for the diene $R^7$ and $R^8$ are methyl, p and q are between 30 and 60, r and s are 1, t and u are 1, and X is $X^1$, and the solvent is toluene, the concentration of diene is between 10% and 20 %, w/v, and the catalyst is Karstedt's catalyst, the catalyst loading is between 1 mol% and 2 mol%, and the reaction temperature is between 18 °C and 28 °C.

In a further specific embodiment, the hydrosilylation reagent used in step ii) is triethoxysilane, and the triethoxysilane is present at between 35 and 45 equivalents, the solvent is toluene, and the excess of triethoxysilane is removed through repeated co-distillation with toluene.

In yet another specific embodiment, the hydrosilylation reagent used in step ii) is triethoxysilane, and the triethoxysilane is present at between 35 and 45 equivalents, the solvent is toluene, and the excess of triethoxysilane is removed through repeated co-distillation with xylene.

In another pecific embodiment, the hydrosilylation reagent used in step ii) is triethoxysilane, and the triethoxysilane is present at between 35 and 45 equivalents, and for the diene $R^7$ and $R^8$ are methyl, p and q are between 30 and 60, r and s are 1, t and u are 1, and X is $X^1$, and the solvent is toluene, the concentration of diene is between 10% and 20 %, w/v, and the catalyst is Karstedt's catalyst, the catalyst loading is between 1 mol% and 2 mol%, the reaction temperature is between 18 °C and 28 °C, the excess of triethoxysilane is removed through repeated co-distillation with toluene or xylene, and the platinum is removed through scavenging with a thiolated polystyrene resin.

*Di-PEGylated dienes*

As stated above, the present disclosure also relates to a compound according to Formula (II):

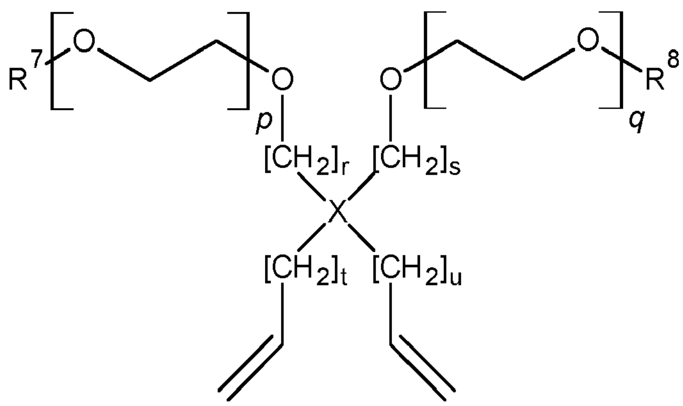

(II)

wherein $R^7$ and $R^8$ are independently chosen to be lower alkyl; p is an integer between 20 and 150; q is an integer between 20 and 150; r is an integer between 1 and 3; s is an integer between 1 and 3; t is an integer between 0 and 3; u is an integer between 0 and 3; and X is chosen from the group consisting of $X^1$, $X^2$, $X^3$, $X^4$, and $X^5$:

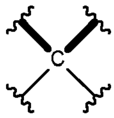

$X^1$

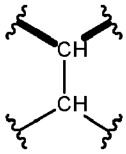

$X^2$

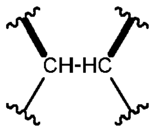

$X^3$

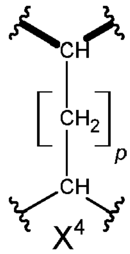

$X^4$

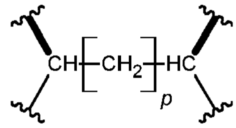

$X^5$

wherein bold bonds represent bonds to $[CH_2]_r$ and $[CH_2]_q$; non-bold bonds represent bonds to $[CH_2]_t$ and $[CH_2]_u$; and p is an integer between 1 and 5.

If, when considering the structure above, two or more covalent bonds are connected in series, they should together be interpreted as one bond.

$R^7$ and $R^8$ may be methyl, ethyl, iso-propyl, n-propyl and/or t-butyl.

Preferably, $R^7$ and $R^8$ are methyl.

In one embodiment, $R^7$ and $R^8$ are methyl, and p and q are between 30 and 60.

In another embodiment, $R^7$ and $R^8$ are methyl, and p and q are between 50 and 70.

In yet another embodiment, r and s are 1.

In a further embodiment, t and u are 1.

In a specific embodiment, $R^7$ and $R^8$ are methyl, p and q are between 30 and 60, and r and s are 1.

In another specific embodiment, $R^7$ and $R^8$ are methyl, p and q are between 30 and 60, r and s are 1, and t and u are 1.

In a further embodiment, $R^7$ and $R^8$ are methyl, p and q are between 30 and 60, r and s are 1, t and u are 1, and X is $X^1$.

*Synthesis of di-PEGvlated dienes*

It has been found that the alkylation of di-alkene-diols with electrophilic PEGylation reagents is a suitable method of producing di-PEGylated dienes. To ensure complete conversion of the di-alkene-diols into the di-PEGylated diene and minimize the formation of the intermediate mono-PEGylated di-alkene-diol, an excess of the PEGylation reagent is necessary. This generates reaction mixtures comprising multiple components, requiring purification prior to hydrosilylation in order for the di-PEGylated diene to be useful in the production of coating precursors.

Specifically, it has been found that when dienes according to Formula (III):

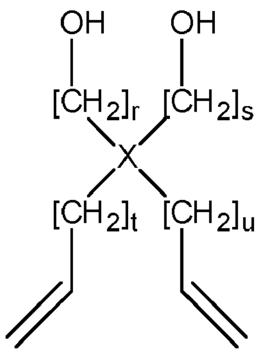

(III)

wherein r is an integer between 1 and 3; s is an integer between 1 and 3; t is an integer between 0 and 3; u is an integer between 0 and 3; and X is chosen from the group consisting of $X^1$, $X^2$, $X^3$, $X^4$, and $X^5$:

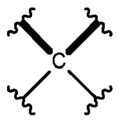
$X^1$

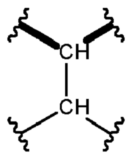
$X^2$

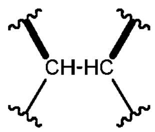
$X^3$

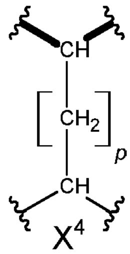
$X^4$

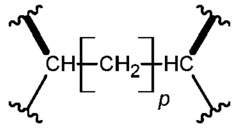
$X^5$

wherein bold bonds represent bonds to $[CH_2]_r$ and $[CH_2]_q$; non-bold bonds represent bonds to $[CH_2]_t$ and $[CH_2]_u$; and p is an integer between 1 and 5; are reacted with PEGylation reagents according to Formula (IV):

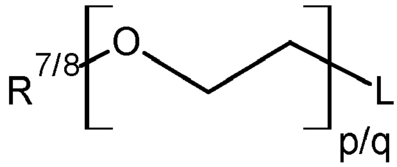
(IV)

wherein $R^{7/8}$, i.e. $R^7$ and $R^8$, are independently chosen from the group consisting of lower alkyls; p/q, i.e. p and q, are independently an integer between 20 and 150; and L is a leaving group chosen from the group consisting of chloride, bromide, iodide, mesylate, tosylate, and triflate;
are reacted to synthesize di-PEGylated dienes according to Formula (II) above, the reaction mixtures are suitable for purification by the present method.

In a specific diene according to Formula (III), r and s are 1, t and u are 0, and X is $X^1$.

In a specific diene according to Formula (III), r and s are 1, t and u are 1, and X is $X^1$.

In a specific diene according to Formula (III), r and s are 1, t and u are 2, and X is $X^1$.

In a specific diene according to Formula (III), r and s are 1, t and u are 3, and X is $X^1$.

In a specific diene according to Formula (III), r and s are 1, t and u are 1, and X is $X^4$ and p is 1.

In a specific diene according to Formula (III), r and s are 1, t and u are 2, and X is $X^4$ and p is 1.

In a specific diene according to Formula (III), r and s are 1, t and u are 0, and X is $X^4$ and p is 1.

In the PEGylation reagent according to Formula (IV), $R^{7/8}$ may independently be methyl or ethyl.

In the PEGylation reagent according to Formula (IV), L may be tosylate or mesylate.

In the PEGylation reagent according to Formula (IV), p and q may independently be an integer between 30 and 60.

In a PEGylation reagent according to Formula (IV), p and q are independently an integer between 30 and 60, L is tosylate and $R^{7/8}$ are methyl.

The reaction mixture from the alkylation of di-alkene-diols according to Formula (III) with more than two equivalents of PEGylation reagents according to Formula (IV) typically comprises a di-PEGylated diene according to Formula (II) as a mixture with impurities. Examples of such impurities are the mono-PEGylated di-alkene-diol reaction intermediate; unreacted PEGylation reagent, $R^{7/8}$-PEG-L; the product of dimerization of the PEGylation reagent, $(R^{7/8}\text{-PEG})_2O$; the product of hydrolysis of the PEGylation reagent, $R^{7/8}$-PEG-OH; as well as HL and/or metal salts of $L^-$.

The reaction mixture may also comprise hydrophobic impurities, such as solvent stabilizers and mineral oil used to protect reagents such as hydride bases used in the synthesis.

An example of the components of the reaction mixture from the alkylation of 2,2-diallyl-propane-1,3-diol with mPEG-OTs from the synthesis of compound **9** (Example 3a), the di-PEGylated diene used in the synthesis of compound **10** (Example 3b), is shown in Fig. 9. The n-decane in Fig. 9 represents mineral oil from the sodium hydride suspension used in the synthesis.

When analyzed by reverse phase HPLC, the PEGylated species elute in the order $R^{7/8}$-PEG-OH < $(R^{7/8}\text{-PEG})_2O$ < $R^{7/8}$-PEG-L $\lesseqgtr$ mono-PEGylated intermediate < di-PEGylated diene. In Fig. 10A is shown a reverse phase HPLC-ELSD chromatogram of a reaction mixture from the synthesis of compound **9** (Example 3a), showing mPEG (Tr (retention time) = 0.86 min), $mPEG_2O$ (Tr = 1.38 min), mPEG-OTs (Tr = 2.38 min), mono-PEGylated intermediate (Tr =2.63 min) and di-PEGylated diene (Tr = 3.68 min). The purity of the reaction mixture in Fig. 10A, as measured by HPLC-ELSD, is approximately 67 %, area/area (a/a).

*Purification of di-PEGvlated dienes*

The present disclosure also relates to a method of purification of di-PEGylated dienes, suitable for use in the production of coating precursors by hydrosilylation as described above. The method has the advantage that it produces di-PEGylated dienes of high purity without the use of costly chromatography. The method also has the advantage that it is useful over a variety of scales, such as from small scale laboratory scale to kilogram production scale and larger, offering a clear advantage over alternative methods suitable only at some limited scale.

The method comprises a stepwise extraction of an aqueous solution of the impure di-PEGylated diene with an organic solvent at elevated temperature. During the extraction the polarity of the aqueous phase is controlled by the addition of salt.

Surprisingly, impurities eluting in the middle of a reverse phase HPLC chromatogram can selectively be extracted from the aqueous phase before the di-PEGylated diene, allowing the separation of the di-PEGylated diene from the impurities.

The method generates di-PEGylated dienes of high purity, such as above 90%, or above 95%, or above 97%. When di-PEGylated dienes of such high purity are used for the synthesis of coating precursors, the resulting coating precursors can be of high purity.

Surprisingly, it has been found that under certain extraction conditions, the desired di-PEGylated diene can be selectively extracted from such a reaction mixture by the stepwise extractive purification process disclosed herein. Specifically, impurities that elute close to, but earlier than the di-PEGylated diene, in a reverse phase chromatogram can under these certain extraction conditions selectively be extracted into an organic phase from an aqueous solution containing the reaction products, leaving the di-PEGylated diene in the aqueous phase. Notably, this behavior is the opposite from what is expected from the elution order. The di-PEGylated diene can subsequently be extracted from the aqueous phase, leaving early eluting impurities in the aqueous phase.

In short, the stepwise extraction comprises two main parts - an intermediate extraction step (steps a) and b)) and a product extraction step (step c)).

In the intermediate extraction step, an aqueous solution of the impure di-PEGylated diene is treated with a salt to adjust the polarity of the solution (step a)), and the aqueous solution is repeatedly extracted with a primary organic extraction solvent at elevated temperature (step b)). In this intermediate extraction, unreacted $R^{7/8}$-PEG-L and any mono-PEGylated di-alkene-diol reaction intermediate(s) present are extracted into the organic phases, leaving the aqueous phase depleted in these impurities.

For the subsequent product extraction step (step c)), the amount of salt in the aqueous solution is increased, and the solution is again repeatedly extracted with the primary extraction solvent at an elevated temperature. In the product extraction, the di-PEGylated diene is extracted into the organic phases along with only minor amounts of impurities.

The organic phases from the product extraction are concentrated (step d)), thereby obtaining a residue (step e)). The residue obtained in step e) is dissolved in an aqueous buffer (step f)) and subjected to polishing extraction (step g)) followed by concentration of the organic phases from the polishing extraction (steps h) and i)) prior to being used for the synthesis of coating precursors.

Specifically, the method comprises the steps of:

a) providing an aqueous solution of an impure compound according to Formula (II) above, wherein the aqueous solution comprises:

  water in an amount of 7.5 to 16.5 times the total mass of the impure compound according to Formula (II) above; and
  NaCl in an amount of 6% to 9% (w/v) of the amount of water;

b) subjecting the aqueous solution of step a) to between 2 and 5 intermediate extractions, performed at a temperature between 40 °C and 70 °C, wherein each intermediate extraction comprises the steps of:

  b1) optionally; adding a further portion of NaCl so that the total amount of NaCl added corresponds to an amount of NaCl less than 9% (w/v) of the amount of water in the aqueous solution of step a);
  b2) extracting the aqueous solution with a carboxylate ester solvent; and
  b3) removing the organic phase, thereby providing an aqueous phase;

c) adding NaCl to the aqueous phase from step b3) in an amount of at least 1 % of the amount of water in step a), so that the total amount of NaCl corresponds to an amount of NaCl between 8% and 12% and subjecting the aqueous phase to between 2 and 5 product extractions, performed at a temperature between 40 °C and 70 °C, wherein each

product extraction comprises the steps of:

c1) extracting the aqueous phase with a carboxylate ester solvent; and
c2) removing the organic phase;

d) pooling the organic phases from each step c2);
e) concentrating the pooled organic phases from step d), thereby obtaining a residue;
f) dissolving the residue from step e) in an aqueous buffer having a pH of between 6 and 9 to provide an aqueous phase;
g) subjecting the aqueous phase from step f) to 2 to 4 polishing extractions, wherein each polishing extraction comprises the steps of:

g1) extracting the aqueous phase from step f) with a chlorinated solvent; and
g2) removing the organic phase;

h) pooling the organic phases from each step g2); and
i) concentrating the pooled organic phases from step h), thereby obtaining a residue, comprising a diPEGylated diene according to Formula (II) above, comprising less than 10 % (w/w) impurities.

The aqueous solution in the intermediate extraction contains between 7.5 and 16.5 parts water for each part PEG-comprising material (step a)). The total amount of PEG-comprising material can be estimated as the sum of masses of the PEG-comprising compounds introduced into the reaction mixture, if the aqueous solution is derived from a reaction mixture stemming from the PEGylation of a diene. Often this is simply the mass of the $R^{7/8}$-PEG-L used in the reaction. Alternatively, the amount of PEG-comprising material can be estimated from the mass fraction of PEG in the material dissolved in the aqueous solution. The mass fraction of PEG can be estimated by a number of methods known to one skilled in the art, such as by quantitative $^1$H-NMR.

The salt used to adjust the polarity in the aqueous phase is NaCl, and concentrations given in this description are for NaCl. However, the salt can be essentially any inorganic salt. The use of other salts, such as KBr, LiCl, $Na_2SO_4$, or $MgCl_2$, can be contemplated. If salts other than NaCl are used to adjust the polarity of the aqueous solutions, the relevant concentrations need to be recalibrated. In Example 20 is shown an example of how to recalibrate the salt concentrations.

The amount of NaCl in the aqueous solution at the start of the intermediate extraction step is between 6% and 9% (w/v) of the amount of water (step a)).

The aqueous phase in the intermediate extraction is extracted with between 2 and 5 portions of the primary extraction solvent at the initial concentration of salt, performed at a temperature between 40 °C and 70 °C (step b)).

In one embodiment, the aqueous phase is first extracted with between 2 and 5 portions of the primary extraction solvent, the primary extraction solvent being a carboxylate ester solvent, (steps b2) and b3)), before the amount of salt in the aqueous phase is increased (step b1)) to a concentration higher than the starting concentration, but still below 9% (w/v) of the amount of water in the aqueous solution of step a), and thereafter the aqueous phase extracted with between 2 and 5 additional portions of the primary extraction solvent (step b2).

The primary extraction solvent is a carboxylate ester (i.e. an alkyl ester of a carboxylic acid), such as ethyl acetate or isopropyl acetate.

Primary extraction solvents other than carboxylate esters can be contemplated, such as ketones, such as methyl ethyl ketone, methyl propyl ketone or diethyl ketone. Solvent mixtures with solvent properties similar to carboxylate esters can also be contemplated. Such solvent mixtures may comprise mixtures of aliphatic or aromatic hydrocarbons with chlorinated solvents.

The elevated temperature of the extractions of step b) is necessary both to realize the desired selectivity in the extractions and to facilitate phase separation. A room temperature separation of the phases is unpractically slow. At temperatures below 40 °C, the selectivity in the extraction is unsatisfactory. It has been found temperatures between 50 °C and 70 °C, such as 60 °C, to be especially suitable.

The amount of NaCl in the aqueous solution at the start of the product extraction step (step c)) is between 8% and 12% (w/v) of the amount of water.

The aqueous phase is extracted between 2 and 5 times in the product extraction (step c)).

The organic phases from the product extraction are pooled (step d)) and concentrated (step e)), or concentrated prior to pooling. The organic phases may be subjected to a step of drying prior to concentration (step e). This can be realized by a number of methods known to one skilled in the art, such as by drying over a drying agent such as magnesium sulphate or molecular sieves.

The residue obtained from the product extraction may comprise some amount of salts of $L^-$ or HL. It may also

comprise some amount of the NaCl used to adjust the polarity of the aqueous phases.

In step g), the residue from step e) is dissolved in an aqueous buffer (step f) and thereafter subjected to a polishing extraction step (step g)). In step f), the residue from the product extraction step is dissolved in a neutral or slightly basic aqueous buffer, having a pH between 6 and 9. The aqueous solution is then extracted 2 to 4 times with a secondary extraction solvent, the secondary extraction solvent being a chlorinated solvent (step g)).

In the polishing extraction step (step g)), the di-PEGylated diene is extracted into the organic phases along with only minor amounts of salts of $L^-$ or HL and only minor amounts of NaCl.

The secondary extraction solvent is a chlorinated organic solvent, such as dichloromethane, chloroform, tetrachloroethane, or a chlorinated aromatic solvent. Dichloromethane is especially useful as the secondary extraction solvent.

A suitable buffer for use in the polishing extraction step (step f)) is aqueous sodium bicarbonate.

The organic phases from the polishing extraction are pooled (step h)) and concentrated (step i)), obtaining a residue. The organic phases may be subjected to an additional step of drying over a drying agent, such as magnesium sulphate or molecular sieves, prior to removal of the solvent.

The residue obtained in step i) comprises the di-PEGylated diene in a high purity, comprising less than 10%, w/w, of impurities. The amount of impurities may be measured by HPLC-ELSD.

Typically, the purity of the di-PEGylated diene is above 90%, such as above 95%, such as above 97%, such as above 99 %. In Fig. 10B is shown a reverse phase HPLC-ELSD chromatogram of the of the di-PEGylated diene **9,** purified according to the present disclosure, indicating a purity of > 99% (area/area).

The residue obtained after the polishing extraction may be subjected to hydrosilylation to generate coating precursors according to the present disclosure. Conditions for hydrosilylation is known to one skilled in the art.

The aqueous solution provided in step a) can be realized through evaporation of the reaction solvent from a reaction mixture from the reaction between a di-alkene-diol according to Formula (III) and a PEGylation reagent according to Formula (IV) and dissolving the resulting residue in water.

It is often more convenient to generate the aqueous solution used in the intermediate extraction step (step b) through a preliminary extraction of the reaction mixture, given that the reaction solvent used to synthesize the di-PEGylated diene is immiscible with water. In such a preliminary extraction, the reaction solvent and hydrophobic impurities are removed from the reaction mixture. In the preliminary extraction, the reaction mixture is treated with water and salt, forming a two-phase system, and the organic phase is removed and the aqueous phase is used, possibly after further addition of salt, as the aqueous solution provided in step a) and used for the intermediate extraction.

In one embodiments of the method, the purification of the di-PEGylated diene starts from a quenched reaction mixture, stemming from reacting a di-alkene-diol according to Formula (III) and a PEGylation reagent according to Formula (IV) under basic conditions in a water immiscible organic solvent, and comprises four extraction steps - a preliminary extraction 100, an intermediate extraction 200, extraction of the product 300, and product polishing 400. The overall process of such a process comprising all four extraction steps is outlined in Fig. 3. The details of the extraction steps are elaborated below.

The preliminary extraction 100, the intermediate extraction 200 and the product extraction steps 300 are performed at a temperature between 50 °C and 70 °C, such as between 55 °C and 65 °C.

The process of the preliminary extraction 100 is outlined in Fig. 4. The reaction mixture (101 in Fig. 4) is treated with water and a salt (102 in Fig. 4), forming a two-phase system (103 in Fig. 4).

The phases are mixed by vigorous agitation and allowed to separate (104 in Fig. 4), again forming a two-phase system (105 in Fig. 4). The organic phase contains most of the reaction solvent and most hydrophobic components of the reaction mixture. The hydrophobic components are generally limited to mineral oil from the use of hydride bases as dispersions in mineral oil, but can include other unwanted nonpolar compounds of any origin. The organic phase is removed (106 in Fig. 4) and the aqueous phase (107 in Fig. 4) is kept for further processing in the intermediate extraction (108 in Fig. 4).

The process of the intermediate extraction (steps a) and b)) may be performed as outlined in Fig. 5. The aqueous phase, e.g. from the preliminary extraction (201 in Fig. 5) is treated with the primary extraction solvent (a carboxylate ester solvent), resulting in a two-phase system that is mixed by vigorous agitation and allowed to separate (202 in Fig. 5), forming a two-phase system (203 in Fig. 5). The organic phase is removed (204 in Fig. 5). The extraction procedure is repeated (206 in Fig. 5) a number of times so that step 204 is performed in total between 2 and 5 times.

Typically, the aqueous phase (205) is essentially free of $R^{7/8}$-PEG-L and mono-PEGylated di-alkene-diol reaction intermediate after 3 to 4 extractions, and the aqueous phase can be passed on to the product extraction (214 in Fig. 5).

Alternatively, if unsatisfactory amounts of $R^{7/8}$-PEG-L and mono-PEGylated di-alkene-diol reaction intermediate(s) remain in the aqueous phase, more NaCl can be added (step b1) and 207 in Fig. 5) and the aqueous phase be subjected to repeated extraction (208 to 213 in Fig. 5). The amount of NaCl added in 207 is such that that the total amount of salt added in 102 and 207 is less than 9%, w/v, of the water added in 102. The extraction procedure is repeated

(213 in Fig. 5) a number of times so that step 211 is performed in total between 2 and 5 times.

The aqueous phase at the end of the intermediate extraction (213 in Fig. 5) is essentially free of $R^{7/8}$-PEG-L and mono-PEGylated di-alkene-diol reaction intermediate(s). The aqueous phase at the end of the intermediate extraction (213) comprises most of the di-PEGylated diene product and most of the product of dimerization of the PEGylation reagent, $(R^{7/8}\text{-PEG})_2O$; and the product of hydrolysis of the PEGylation reagent, $R^{7/8}$-PEG-OH, present in the reaction mixture prior to the preliminary extraction.

The process of the product extraction (step c) may be performed as outlined in Fig. 6. The aqueous phase from the intermediate extraction (301 in Fig. 6) is treated with an additional portion of NaCl (302 in Fig. 6). The amount of NaCl added in 302 is such that that the total amount of salt added in 102, 207 and 302 is between 8 and 12% (w/v), of the water added in 102. The amount of NaCl added in 302 is at least 1 % (w/v) of the water added in 102.

The aqueous phase (303 in Fig. 6) is then treated with a portion of the primary extraction solvent (a carboxylate ester solvent), the phases mixed by vigorous agitation and allowed to separate (304 in Fig. 6), resulting in a two-phase system (305 in Fig. 6). The organic layer is withdrawn (306 in Fig. 6). The organic phase in the product extraction (309 in Fig. 6) comprises the di-PEGylated diene product along with some of salts of $L^-$ or HL, and essentially no other PEGylated species.

The aqueous phase (307 in Fig. 6) is subjected to further extractions (308 in Fig. 6) so that step 306 is performed in total between 2 and 5 times.

The organic phases comprising the di-PEGylated diene product are concentrated (310 in Fig. 6), resulting in a residue (311 in in Fig. 6). This is most conveniently realized through distillation of the solvent under reduced pressure. Often it is advantageous to dry the organic phases prior to concentration. This can be realized by a number of methods known to one skilled in the art, such as by drying over a drying agent such as magnesium sulphate or molecular sieves.

The product polishing may be performed as outlined in Fig. 7. The residue from the product extraction (401 in Fig. 7) is first dissolved in an aqueous buffer buffered to a pH between 6 and 9 (402 in Fig. 7).

The buffered aqueous solution (403 in Fig. 7) is extracted with a secondary extraction solvent (a chlorinated solvent) (404 in Fig. 7) resulting in a two-phase system (405 in Fig. 7).

The organic phase is withdrawn (406 in Fig. 7). The organic phase in the product polishing (409 in Fig. 7) comprises the di-PEGylated diene product essentially free of low molecular weight impurities, and essentially free of PEGylated impurities.

The aqueous phase (407 in Fig. 7) is subjected to further extractions (408 in Fig. 7) so that step 406 is performed in total between 2 and 4 times.

The organic phases from the polishing step (409 in Fig. 7) are pooled and concentrated (410 in Fig. 7). It is advantageous to dry the organic phases over a drying agent, such as magnesium sulphate or molecular sieves, prior to removal of the solvent.

The residue (411 in Fig. 7) obtained from removal of the solvent from the organic phases (i.e. after step i) comprises di-PEGylated diene of sufficient purity such that when the di-PEGylated diene is used to produce coating precursors according to the present disclosure by hydrosilylation the coating precursors require only limited purification.

Typically, the purity of the di-PEGylated diene is above 90%, such as above 95%, such as above 97%, such as above 99 %. In Fig. 10B is shown a reverse phase HPLC-ELSD chromatogram of the of the di-PEGylated diene 9, purified according to the present disclosure, indicating a purity of > 99% (area/area).

In one embodiment; in the intermediate extraction step (step b)) the concentration of NaCl is between 7% and 8% (w/v); in the product extraction step (step c)) the concentration of NaCl is between 9% and 11% (w/v); the primary extraction solvent is ethyl acetate; the intermediate and product extractions are performed at between 55 °C and 65 °C; and the secondary extraction solvent is dichloromethane.

***Use of the coating precursors to coat surfaces, specifically the surface of nanostructures***

The present disclosure also relates to the use of coating precursors according to the present disclosure, or the product of the method described above, to coat surfaces.

Specifically, the coating precursors described herein can be used as an intermediate in the production of coated nanostructures.

The surfaces may be surfaces of macroscopic to microscopic to nanosized objects. Typically, the surface is the surface of a nanostructure, and the product of the coating precursor coating the surface is a coated nanostructure. Such coated nanostructures and the use thereof are described below.

The surface to be coated preferably comprises silanol functionalities.

If the silanol functionalities in the coating precursor comprise alkoxy groups, the presence of some water is beneficial for the coating of the surface.

If the surface to be coated is the surface of a nanostructure, dissolving the nanostructures in water or an aqueous mixture of solvents prior to contacting the surface with the coating precursor is a suitable method of coating the surface.

It is suitable to bring the coating precursor into contact with the surface to be coated by dissolving the coating precursor in a solvent and bring the solution of the coating precursor in contact with a solution of the nanostructure to be coated.

Suitable solvents for dissolving the coating precursor are water-miscible non-protic polar organic solvents, such as dioxane, THF, DMSO or DMF.

If $R^1$, $R^2$, and $R^3$ in $Z^1$ and $Z^2$ are the same alkoxy group, the alcohol $R^1H$ is a suitable solvent for dissolving the coating precursor.

The use of anhydrous solvents for dissolving the coating precursor is advantageous.

The use of coating precursors to coat surfaces is advantageously performed at elevated temperatures, such as above 60 °C, such as above 80 °C, such as above 100 °C, such as above 120 °C, such as above 140 °C. Thus, the coating is preferably carried out at a temperature between 80 °C and 140 °C, preferably between 100 °C and 120 °C.

It is advantageous to add the solution of coating precursor to the surface to be coated slowly, such as by addition of multiple portions of the solution of coating precursor. Alternatively, the solution of coating precursor can be added to the surface to be coated by slow infusion of the solution of coating precursor.

The coating precursor can be added to the surface to be coated as a melt.

Alternatively, the coating precursor may be added to the solution of nanostructures to be coated as a solid.

Specifically, a solution of the coating precursor may be slowly infused into a solution of the nanostructures to be coated.

In a preferred embodiment, the solution of the coating precursor in anhydrous THF or anhydrous dioxane is slowly infused into a solution of the nanostructures to be coated.

In a specific embodiment, $R^1$, $R^2$, and $R^3$ in $Z^1$ and $Z^2$ in the coating precursor are ethoxy, and a solution of the coating precursor in anhydrous ethanol is slowly infused into a solution of the nanostructures to be coated.

In a preferred embodiment, $R^1$, $R^2$, and $R^3$ in $Z^1$ and $Z^2$ in the coating precursor are ethoxy, and a solution of the coating precursor in anhydrous ethanol is slowly infused into a solution of the nanostructures to be coated having a temperature of between 100 °C and 120 °C.

In a particularly preferred embodiment, the coating precursor is 1,7-bis(triethoxysilyl)-4,4-bis(ω-methyl-(ethyleneoxy)45-methyl)-heptane, the nanostructure to be coated comprises monomer residues derived from 1,7-bis-(triethoxysilyl)-4,4-bis-(dimethylphosphonato)-heptane, the coating precursor is dissolved in anhydrous ethanol or dioxane, the nanostructure to be coated is dissolved in a solvent mixture comprising ethylene glycol and water, the solution of the nanostructures to be coated is heated to between 100 °C and 120 °C, and the coating precursor is added to the solution of the nanostructure to be coated by slow infusion over a period of between 10 and 50 hours, such as 20 hours.

Example 14 shows the use of coating precursors according to the present disclosure to coat nanostructures.

Examples 16a and 16b demonstrate that nanostructures coated with precursors according to the present disclosure have longer plasma half-life and better storage stability than nanostructures coated with reference precursors.

### *Nanostructures comprising a coating derived from the coating precursors*

As stated above, the coating precursors according to the present disclosure can be used to produce a coating on nanostructures.

Thus, the present disclosure also relates to a globular nanostructure comprising a coating derived from coating precursors according to the structure of Formula (I) as described above or derived from the product of the method according to the present disclosure.

As stated above, the coating precursors according to the present disclosure comprise reactive silane functionalities. This renders the coating precursors capable of forming bonds to surfaces comprising functional groups capable of forming bonds of the structure G-O-Si, where G represents an atom in the surface, such as a silicon atom, and Si represents a silicon atom from a coating precursor molecule. Especially nanostructures where the surface comprises silanol functionalities are suitable intermediates in the production of coated nanostructures according to the current disclosure.

Nanostructures to be coated are also referred to as "precursor nanostructures".

Nanostructures to be coated with the coating precursors according to present disclosure may be referred to as "central part". Such a central part typically comprises a polymer which, on the surface, has reactive groups suitable to form bonds to the coating precursors. The central part preferably comprises a cross-linked polymer network, comprising siloxane linkages. The reactive groups on the surface may comprise silanol groups.

The precursor nanostructures can have a simple structure containing a single kind of polymer, or a more complicated structure comprising composites of several different materials, such as multiple layers or zones of different materials, or be made up of several substructures joined together, optionally arranged in layers.

The coated nanostructures comprise an outer layer, i.e. the coating layer, comprising residues derived from the coating precursor.

Figure 2 shows one example of the structure of a coated nanostructure according to the present disclosure. The coated nanostructure comprises three parts: 1) a central part having a hydrodynamic diameter $D_A$, 2) an intermediate layer of thickness [($D_B$-$D_A$)/2], where $D_B$ is the hydrodynamic diameter of the central and intermediate layer, and 3) a coating layer of thickness [($D_C$-$D_B$)/2], where $D_C$ is the hydrodynamic diameter of the coated nanostructure.

The coated nanostructures may further comprise a radionuclide.

The coating precursor molecules attach through silane groups in the coating precursors to surface groups of precursor nanostructures via siloxane bonds. The coating layer serves to protect the central part of the nanostructures from aggregation and from interactions with proteins in living organisms, thus enhancing the biocompatibility and stability of the coated nanostructure under physiological conditions.

Coated nanostructures where the coating is derived from coating precursors according to the current disclosure have superior properties as compared to coated nanostructures where the coating is derived from certain other coating precursors. For example, coated nanostructures where the coating is derived from coating precursors according to the current disclosure exhibit higher storage stability than coated nanostructures where the coating is derived from coating precursors of the structure mPEG-$CH_2$-$CH_2$-Si$R^1R^2R^3$, where $R^1$, $R^2$, and $R^3$ are chloride, bromide, iodide, lower alkoxy, aryloxy, carboxy, amino, or -NH-acyl (see Example 16).

Coated nanostructures where the coating is derived from coating precursors according to the current disclosure exhibit superior *in vivo* properties as compared to coated nanostructures where the coating is derived from certain other coating precursors. In Example 16a is shown that coated nanostructures where the coating is derived from 1,7-bis(triethoxysily)-4,4-bis(ω-methyl-(ethyleneoxy)$_{45}$-methyl)-heptane, compound **10**, exhibit longer circulation times *in vivo* as compared to similar nanostructures where the coating is derived from 1-(ω-methyl-(ethyleneoxy)$_{45}$-methyl)-3,5-bis(3-(triethoxysilyl)propyloxy)-benzene, compound **51**, a compound outside the scope of the present disclosure.

Coated nanostructures where the coating is derived from coating precursors according to the current disclosure exhibit high stability with regard to aggregation (see Example 16b).

Further, coated nanostructures where the coating is derived from coating precursors according to the current disclosure exhibit high stability with regard to the loss of coating residues from the nanostructures. In Example 16b is shown that nanostructures where the coating is derived from coating precursors according to the current disclosure have a better storage stability than nanostructures coated with precursors outside the scope of the present disclosure.

Coated nanostructures where the coating is derived from coating precursors according to the current disclosure have good solubility in water. In Example 21 is shown that the solubility of nanostructures coated with coating according to the present disclosure is at least 25 % (w/v).

*Specific embodiments of coated nanostructures*

The coated nanostructures preferably have a hydrodynamic diameter between 10 nm and 100 nm, such as between 12 nm and 80 nm, such as between 14 nm and 60 nm, such as between 16 nm 50 nm, such as between 18 nm and 45 nm, such as between 20 nm and 40 nm, such as between 25 nm and 35 nm.

In one embodiment, the coated nanostructures comprise chelating groups. Preferably, the chelating groups are bisphosphonate groups, more preferably germinal bisphosphonates.

The coated nanostructures may further comprise a radionuclide.

In a specific embodiment, the coated nanostructures comprise a central part derived from 1,7-bis-(triethoxysilyl)-4,4-bis-(dimethyl-phosphonato)-heptane.

In another specific embodiment, the coated nanostructures comprise a central part derived from 1,7-bis-(triethoxysilyl)-4,4-bis-(dimethylphosphonato)-heptane, and have a hydrodynamic diameter between 10 nm and 100 nm, such as between 12 nm and 80 nm, such as between 14 nm and 60 nm, such as between 16 nm 50 nm, such as between 18 nm and 45 nm, such as between 20 nm and 40 nm, such as between 25 nm and 35 nm. Preferably, the hydrodynamic diameter is between 25 nm and 35 nm.

In a further specific embodiment, the coated nanostructures have a hydrodynamic diameter between 25 nm and 35 nm, comprise a central part derived from 1,7-bis-(triethoxysilyl)-4,4-bis-(dimethylphosphonato)-heptane, and have a coating layer derived from bis(triethoxysilyl)methane.

In a preferred embodiment, the coated nanostructures have a hydrodynamic diameter between 25 nm and 35 nm, comprise a central part derived from 1,7-bis-(triethoxysilyl)-4,4-bis-(dimethylphosphonato)-heptane surrounded by an intermediate layer of a polymer derived from bis(triethoxysilyl)methane, and an outer coating layer derived from 1,7-bis(triethoxysily)-4,4-bis(ω-methyl-(ethyleneoxy)$_{45}$-methyl)-heptane.

Example 14 shows some examples of coated nanostructures with coatings derived from coating precursors according to the present disclosure.

Examples 16a and 16b demonstrate that nanostructures coated with precursors according to the present disclosure have longer plasma half-life and better storage stability than nanostructures coated with reference precursors.

***Pharmaceutical composition comprising coated nanostructures***

The present disclosure also relates to a pharmaceutical composition comprising a plurality of globular nanostructures according to the present disclosure.

The present disclosure further relates to a pharmaceutical composition for use in the treatment of cancer or in imaging, wherein the pharmaceutical composition comprises a plurality of globular nanostructures according to the present disclosure and wherein the nanostructures comprise a radionuclide

Such pharmaceutical compositions have the advantage that the coating derived from coating precursors according the present disclosure renders the components of the pharmaceutical composition highly biocompatible and/or bioinert.

The pharmaceutical composition may be used for medical imaging, such as medical imaging for the diagnosis of cancer.

The medical imaging may be e.g. SPECT or PET imaging.

When the pharmaceutical composition, is used for medical imaging, the pharmaceutical composition further comprises a radioactive isotope (a radionuclide).

A pharmaceutical composition according to the present disclosure used for SPECT imaging typically comprises gallium-67, indium-111, technetium-99m, lutetium-177 and/or thallium-201.

A pharmaceutical composition according to the present disclosure used for PET imaging typically comprises copper-62, gallium-68, rubidium-82, yttrium-86 and/or zirconium-89.

The pharmaceutical composition may be used for the treatment of cancer, such as the treatment of cancer by radionuclide therapy. In such cases, the coated nanostructures comprise radionuclides, such as actinium-225, copper-64, copper-67, holmium-166, lead-212, lutetium-177, radium-223, rhenium-186, rhenium-188, samarium-153, strontium-89, thorium-227 and/or yttium-90.

In one embodiment, the rardionuclide is. $^{177}Lu$, $^{153}Sm$ and/or $^{90}Y$.

A pharmaceutical composition according to the present disclosure shows desirable pharmaceutical properties, such as pharmacokinetic profiles suitable for the accumulation of the coated nanostructures in tumor tissue.

In Examples 17 and 18 are shown the use of a pharmaceutical composition comprising coated nanostructures with a coating derived from coating precursors according the present disclosure.

Mice having a tumor treated with a pharmaceutical composition comprising nanostructures according to the present disclosure showed significant increased mean and median survival time as compared to a control group (see Example 17). Further, the tumor growth was significantly slowed down for the treatment group (see Example 17 and Fig. 12.

***Nanostructures* as *carriers of radionuclides***

The present disclosure also relates to the use of a globular nanostructure according to the present disclosure as a carrier for radioactive isotopes (radionuclides).

In such cases, the nanostructures further comprise chelating groups. Preferably, the chelating groups comprise phosphonates, such as geminal bisphosphonates.

Such carriers for radioactive isotopes may be used for diagnosis (such as medical imaging) and treatment of cancer as described above (see Examples 17 and 18).

In one preferred embodiment, , the coated nanostructures comprise a central part comprising monomer residues derived from 1,7-bis(triethoxysily)-4,4-bis($\omega$-methyl-(ethyleneoxy)$_{45}$-methyl)-heptane.

As stated above, the carriers for radioactive isotopes may carry a radioisotope suitable for medicinal imaging, such as a radioisotope suitable for PET or a radioisotope suitable for SPECT, and/or a radioisotope suitable for radionuclide therapy.

The radioactive isotopemay be e.g. $^{177}Lu$, $^{153}Sm$ and/or $^{90}Y$.

**EXAMPLES**

Examples of different embodiments of the present disclosure will be described below.

***General experimental conditions***

Materials, reagents and solvents were obtained from commercial sources and were used without further purification unless otherwise noted. Solvents were of reagent grade or similar if not otherwise noted. Reactions were carried out under $N_2$ unless otherwise noted.

MilliQ water refers to ultrapure water, e.g. water purified using a Millipore Milli-Q lab water system.

SIR-200, a thiolated polystyrene resin was purchased from ResinTech, USA (resintech.com) and activated by aqueous $NaHCO_3$, carefully washed in EtOH and toluene, and dried azeotropically before use.

HPLC was performed on a Hewlett Packard Series 1100 equipped with an Agilent Poroshell 120 EC-C18 4.6 × 50 mm column, a DAD detector recording at 220 nm and an ELSD detector (ELSD settings: 40 °C, 1.4 l $N_2$/min, gain=4, impactor on), using a nonlinear gradient starting at 43% acetonitrile in water, at 1 ml/min with an oven temperature of 40 °C.

DLS was measured using a Malvern Instruments Zetasizer Nano ZEN3600 and processed using the general process setting in the Zetasizer software.

SEC was performed on a Younglin Instrument YL9100 equipped with an Agilent Bio SEC-5 1000 Å column eluting at 1.2 ml/min at ambient temperature a DAD detector recording at 220 nm, 280 nm and 560 nm, and an ELSD detector (ELSD settings: 60 °C, 1.2 l N2/min, gain=4).

NMR spectra were recorded on a Varian Unity Inova 400 MHz (1H at 399.95 MHz, 13C at 125.68 MHz) spectrometer using the residual solvent peak as internal standard for $^{1}H$ NMR and $^{13}C$ NMR.

Compounds **31** (Tanikaga,R. et al. Synthesis, 1977, p. 299 - 301), **50** (EP2572736), **51** (WO2018130713), and **52** (US2005255514) were synthesized according to literature procedures.

*Acronyms*

Tetrahydrofuran (THF), dichloromethane (DCM), polytetrafluoroethylene (PTFE), ethyl acetate (EtOAc), methanol (MeOH), ethanol (EtOH), potassium acetate (KOAc), acetonitrile (MeCN), lithium aluminum hydride (LAH), methanesulfonyl chloride (MsCl), triethylamine (TEA).

***Example 1: Synthesis of Compound 5, 4-[ω-methyl-(ethyleneoxy)$_{45}$-methyl]-1,7-Bis(triethoxysilyl)heptane.***

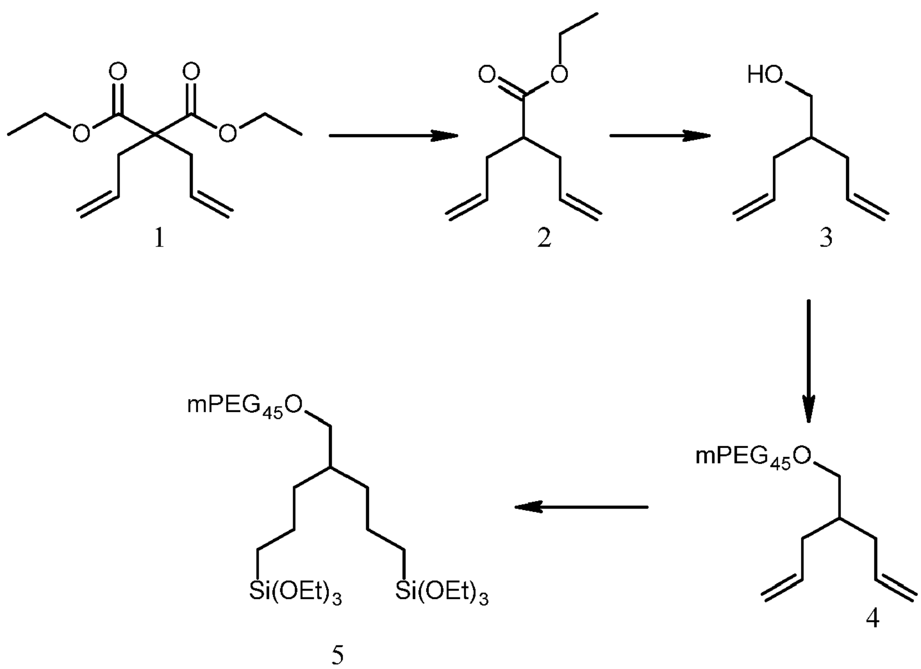

*Example 1a: Compound **2**, 4-ethoxycarbonyl-1,6-heptadiene*

Diethyl diallylmalonate 1 was dissolved in DMSO (57 ml) followed by the addition of milliQ water (650 μl) and LiCl (2.98 g, 70.5 mmol). The reaction mixture was stirred under nitrogen at 190 °C for 3 hours. The reaction was monitored by TLC until complete conversion of the staring material. The solution was diluted with 100 ml brine and extracted three times with 100 ml diethyl ether. The combined organic phases were dried over anhydrous $MgSO_4$, filtered and evaporated under vacuum to obtain compound **2** as a colorless oil (3.90 g, 72 % yield).
$^{1}H$ NMR (400 MHz, $CDCl_3$) δ: 5.74 (m, *J* = 17.1, Hz, 2H), 5.04 (m, 4H), 4.13 (q, *J* = 7.1 Hz, 2H), 2.50 (m, *J* = 8.0, 1H), 2.31 (m, 4H), 1.24 (t, *J* = 7.1 Hz, 3H).

*Example 1b: Compound **3**, 4-hydroxymethyl-1,6-heptadiene*

$LiAlH_4$ was added to anhydrous THF (26.74 ml), under nitrogen at 0 °C, in a 50 ml reactor. The mixture was cooled down to -78 °C and 4-ethoxycarbonyl-1,6-heptadiene **2** (2.34 g, 139 mmol) was added. After one hour the mixture was brought to room temperature. The reaction was monitored by TLC until complete conversion of the staring material. The reaction mixture was cooled down to 0 °C and 225 μl milliQ water was added in portions, followed by the addition of 225 μl 15% NaOH and 670 μl milliQ water. Filtration and evaporation of the volatiles yielded the product **3** as an oil that was purified by chromatography on silica. (1.272 g, 72 % yield).
$^1H$ NMR (400 MHz, $CDCl_3$) δ: 5.82 (m, *J* = 17.2 Hz, 2H), 5.05 (t, 4H), 3.57 (d, *J* = 5.6 Hz, 2H), 2.12 (t, *J* = 6.1 Hz, 4H), 1.72 (hept, *J* = 6.4 Hz, 1H).

*Example 1c: Compound **4**, 4-[(ω-methyl-(ethyleneoxy)$_{45}$-methyl]-1,6-heptadiene*

In a three-necked 100 ml round bottom flask the diallyl alcohol **3** of example 1b (262 mg, 1.23 mmol) was dissolved in anhydrous toluene (20 ml). The temperature of the reaction medium was set to 0 °C and NaH (179.5 mg, 4.5 mmol, 60% dispersion in mineral oil) was added and the mixture was stirred for one hour. The azeotropically dried solution of m-$PEG_{45}$-OTs (5.84 g, 2.7 mmol) in toluene (25 ml) was added. The mixture was brought to room temperature over the course of 1 hour before being heated to a reflux. The reaction was monitored by TLC (Heptane : Ethyl acetate 5:1) and HPLC until complete conversion and quenched by the addition of milliQ water (330 μl) in portions at 60 °C. Toluene was evaporated under vacuum and the remaining solution was diluted in milliQ water (78 ml) and NaCl (4.1 g) and EtOAc (25 ml) were added. After vigorous stirring, the two layers were separated and the organic phase removed. The remaining aqueous phase was extracted twice with EtOAc (50 ml). The combined organic phases were dried over anhydrous $MgSO_4$, filtered and evaporated under vacuum to obtain a white solid compound **4** (22% yield).
$^1H$ NMR (400 MHz, $CDCl_3$) δ: 5.76 (m, 2H), 5.01 (m, 4H), 3.64 (s, 225H), 3.37 (s, 3H), 3.33 (d, *J* = 6.1 Hz, 2H), 2.08 (m, 4H), 1.78 (m, 1 H).

*Example 1d: Compound **5**, 4-[ω-methyl-(ethyleneoxy)$_{45}$-methyl]-1,7-bis(triethoxysilyl)heptane*

To the azeotropically dried solution of compound **4** (595 mg, 0.27 mmol) from example 1c in dry toluene (7 ml) triethoxysilane (205 μl, 0.182 g, 1.11 mmol, 4.0 eq) was added. The bath temperature was set to 30 °C and Karstedt's catalyst (20.4 μl, 2% in xylenes) was added. The reaction mixture was stirred at room temperature overnight. The reaction was monitored with $^1H$-NMR for the disappearance of the olefinic protons. The solvent was then evaporated and excess silane was removed by co-evaporating with anhydrous toluene (4 ml). The product was dissolved in toluene (5 ml) degassed with 3 cycles of vacuum/nitrogen and stirred with activated SIR-200 resin (1.56 g) for 4 days at 60 °C. Filtration and evaporation of the solvent gave 217.9 mg (36.2 % yield) of compound **5** as a colorless solid.
$^1H$ NMR (400 MHz, $CDCl3_3$) δ: 3.72 (q, *J* = 7.0 Hz, 2H), 3.64 (s, 191H), 3.38 (s, 2H), 1.24 (m, 3H).

***Example 2: Synthesis of compound 8, 1,7-bis(triethoxysilyl)-4,4-bis(ω-methyl-(ethyleneoxy)17-methyl)-heptane:***

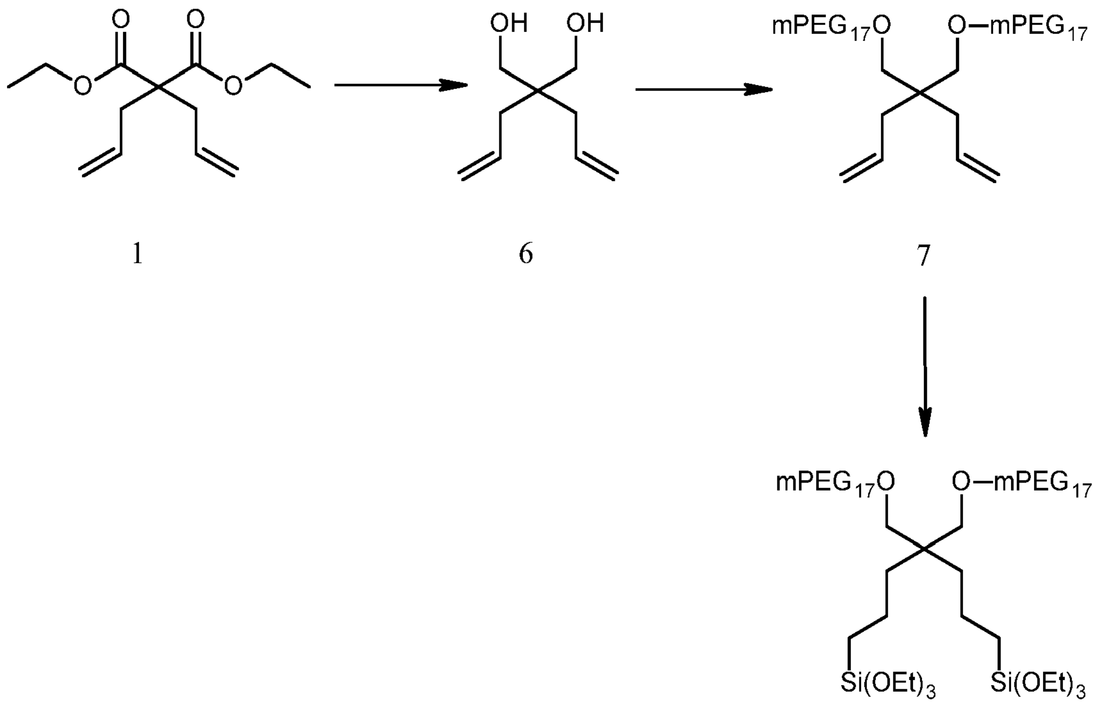

1 6 7

8

*Example 2a: Compound **6**, 2,2-diallyl-1,3-propanediol.*

Diethyl diallylmalonate 1 (245 g, 1 mmol) was added dropwise to a suspension of $LiAlH_4$ (94.93 g, 2.37 mmol, 2.4 eq) in anhydrous THF (1000 ml) at 0 °C. The rate of addition was adjusted so that the inner temperature was maintained below 10 °C. The reaction was stirred at room temperature overnight. Completion of the reaction was confirmed by TLC (Heptane:EtOAc 7:3, Rf(SM) 0.6, Rf(prod) 0.2, developed with $KMnO_4$). The reaction mixture was cooled to -5 °C and quenched by slow addition of $H_2O$ (100 ml) in portions over 4.5 hours, followed by a slow addition of a 15 % aqueous solution of NaOH (100 ml) in portions, and 282 ml of $H_2O$ and stirred at 20°C overnight The suspension was filtered through two GF/A filter papers and the filter cake washed three times with 300 ml THF. The volatiles were evaporated under reduced pressure to obtain the desired product **6** as a yellow oil (158.7 g, ≥ 99 % yield).
$^1$H NMR (400 MHz, $CDCl_3$) δ: 5.83 (m, 2H), 5.11 (m, 4H), 3.56 (s, 4H), 2.07 (d, 4H).

*Example 2b: Compound **7**, 4,4-bis(ω-methyl-(ethyleneoxy)$_{17}$-methyl)-hepta-1.6-diene.*

Compound **6** (178 mg, 1.13 mmol) from example 2a was dissolved in anhydrous toluene (15 ml) and cooled in an ice bath. When the inner temperature reached below 10 °C, NaH (199 mg, 7.9 mmol, 95 % in mineral oil, 7 eq) was added in portions. The mixture was then stirred at room temperature for 30 minutes and an azeotropically dried solution of m-PEG$_{17}$-OTs (4.44 g,4.74 mmol, 4.2 eq) in toluene (25 ml) was added under $N_2$ at 0 °C. The reaction mixture was heated to reflux overnight. The reaction was monitored by HPLC and upon completion the temperature was lowered to 15 °C and the reaction was quenched by a slow addition of $H_2O$ (1 ml). The pH of the crude product was adjusted to a pH of 5-7 with 1.0 M HCl and the mixture was diluted with $H_2O$ (60 ml). NaCl (4.5 g) was added at 60 °C and the reaction mixture was extracted three times with EtOAc (20 ml). To the remaining aqueous phase NaCl (1.5 g) was added and the mixture was additionally extracted with EtOAc (20 ml) at 60 °C. The extracted fractions with acceptable product purity were combined and the solvent evaporated. The resulting residue was purified by flash chromatography (C18 column, $H_2O$ : acetonitrile 60 : 40) and dissolved in DCM. The mixture was then dried over $MgSO_4$, filtered and evaporated to obtain the desired product **7** (892 mg, 46 % yield).
$^1$H NMR (400 MHz, $CDCl_3$) δ 5.79 (m, 2H), 5.04 (m, 4H), 3.65 (s, 350 H), 3.38 (s, 6H), 3.23 (s, 2H), 2.05 (d, 2H)

*Example 2c: Compound **8**, 1,7-bis(triethoxysilyl)-4,4-bis(ω-methyl-(ethyleneoxy)$_{17}$-methyl)-heptane*

To the azeotropically dried solution of compound **7** from example 2b (0.88 g, 0.528 mmol) in toluene (7 ml), triethoxysilane (3.97 g, 21 mmol, 40 eq) was added. The bath temperature was set to 30 °C and Karstedt's catalyst (77 μl, 2% in xylenes) was added. The reaction mixture was left stirring at room temperature overnight. The reaction was

monitored with $^1$H-NMR for the disappearance of the olefinic protons. The solvent was then evaporated and excess silane was removed by co-evaporating with anhydrous toluene (10 ml). The product was dissolved in toluene (17 ml), degassed with 3 cycles of vacuum/nitrogen, and stirred with activated SIR-200 (710 mg) resin for 4 days at 60 °C. The solution was filtered from the resin, the resin washed with toluene (3 × 12 ml), and the solvent was evaporated *in vacuo,* yielding compound **8** (876 mg, 82.4% yield) as a colorless solid.

$^1$H NMR (400 MHz, $CDCl_3$) δ: 3.71-3.60 (d, 123H), 3.56 (m, 4H), 3.39 (s, 6H), 1.26 (d, *J* = 2.8 Hz, 5H).

***Example 3: Synthesis of Compound 10, 1,7-bis(triethoxysilyl)-4,4-bis(ω-methyl-(ethyleneoxy)$_{45}$-methyl)-heptane***

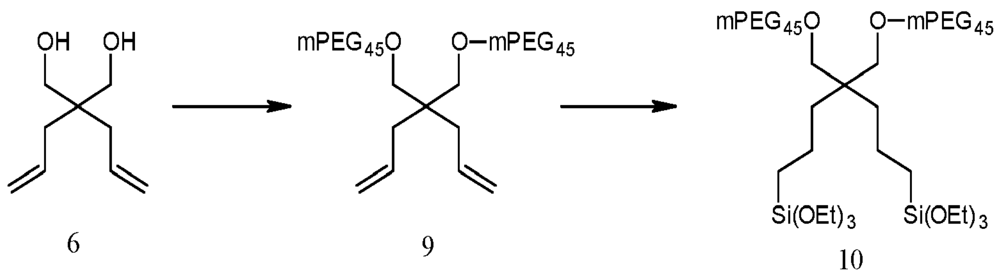

6 9 10

*Example 3a*: *Compound **9,** 4,4-bis(ω-methyl-(ethyleneoxy)$_{45}$-methyl)-hepta-1.6-diene*

Diallyl propanediol **6** (22 g, 0.1411 mol) was dissolved in anhydrous toluene (2.61 l) and cooled in an ice bath. When the inner temperature reached below 10 °C, NaH (23.7 g, 0.593 mol, 60 % in mineral oil, 4.2 eq) was added in three portions maintaining the temperature below 10 °C. The slurry was then stirred at room temperature for 60 minutes and then added to an azeotropically dried solution of m-PEG$_{45}$-OTs (1.071 kg, 0.9877 mmol, 3.5 eq) in anhydrous toluene (2.61 l) under $N_2$ at 0 °C. The reaction mixture was heated to reflux overnight and stirred under $N_2$. The reaction was monitored by HPLC and upon completion, the temperature was lowered to 15 °C and the reaction was quenched by a dropwise addition of $H_2O$ (70 ml). The pH of the crude reaction mixture was adjusted to between 5 and 7 with 1.0 M HCl (100 ml). The crude reaction mixture was split in two equal portions for practical reasons and the two portions extracted separately. Each half of the crude mixture was diluted with $H_2O$ (7.14 l). The temperature was increased to 60 °C and NaCl (540 g) was added. The mixture was then stirred for 45 minutes and extracted three times with EtOAc (2.1 l). To the remaining aqueous phase NaCl (200 g) was added and the mixture was again extracted three times with EtOAc (2.1 l). The last three extracted fractions had an acceptable product purity (HPLC) and were dried over $MgSO_4$, filtered through double GF/A filters and the solvent evaporated. The resulting residues were pooled and dissolved in $H_2O$ (2.0 l) and the pH was adjusted to pH 8 with an 0.8 M aqueous solution of $NaHCO_3$ (100 ml). The aqueous phase was extracted three times with DCM (500 mL). The organic phases were dried over $MgSO_4$, filtered and evaporated to obtain a white residue.

Consequently, the extraction process repeated for the other half of the crude mixture and the final products of both extractions were pooled to obtain the desired compound **9** (758 g, 66.36 % yield, 96.8 % purity (HPLC-ELSD)).

$^1$H NMR (400 MHz, $CDCl_3$) δ 5.80 (m, 2H), 5.03 (m, 4H), 3.81 (m, 4H), 3.70-3.60 (s, 540H), 3.37 (s, 6H), 3.22 (s, 4H), 2.04 (d, 4H).

*Example 3b: Compound **10,** 1,7-bis(triethoxysilyl)-4,4-bis(ω-methyl-(ethyleneoxy)$_{45}$-methyl)-heptane:*

To the azeotropically dried solution of compound **9** from example 3a (714 g, 0.172 mol) in toluene (5.5 l), triethoxysilane (1117 g, 6.88 mol, 40 eq) was added at 22 °C under nitrogen. Karstedt's catalyst (25.34 ml, 2 % in xylenes, 1.14 mmol, 0.0066 eq) was added in 1 mL portions using a syringe over 30 minutes which resulted in an exotherm of ≤ 2 °C. The reaction mixture was left stirring at 22 °C under nitrogen overnight.

The reaction was monitored by $^1$H-NMR for the disappearance of the olefinic protons. The solvent was then evaporated and excess silane was removed by co-evaporating with anhydrous toluene (2.5 l) for a total of four times. The residue was then redissolved in toluene (4.2 l) degassed with 3 cycles of vacuum/nitrogen and stirred with activated SIR-200 resin (175 g) for 3 days at 60 °C. The solution was filtered from the resin, the resin washed with toluene (3 x 2.8 l), the collected fractions were filtered through double GF/A filters, pooled and the solvent evaporated to obtain compound **10** as a white solid in quantitative yield (783.2 g, ≥99 %, 94.4 % purity (HPLC-ELSD)).

$^1$H NMR (400 MHz, $C_6D_6$) δ 3.88 (q, 12H), 3.70-3.40 (s, 400H), 3.14 (s, 6H), 1.68 (m, 4H), 1.59 (m, 4H), 1.23 (t, 18H), 0.79 (t, 4H).

***Example 4: Synthesis of Compound 12, 1,7-bis(triethoxysilyl)-4,4-bis(ω-methyl-(ethyleneoxy)$_{67}$-methyl)-heptane***

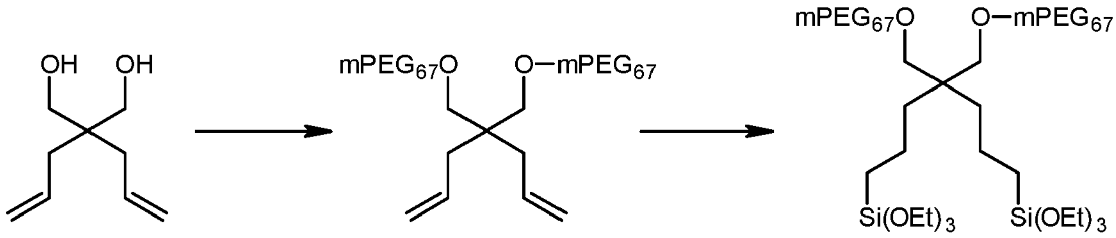

6 11 12

*Example 4a: Compound **11**, 4,4-bis(ω-methyl-(ethyleneoxy)$_{67}$-methyl)-hepta-1,6-diene*

Diallyl propanediol **6** (1.14 g, 7.14 mmol) was dissolved in anhydrous toluene (195 ml) and cooled in an ice bath. When the inner temperature reached below 10 °C, NaH (717 mg, 17.9 mmol, 60 % in mineral oil, 2.5 eq) was added in three portions maintaining the temperature below 10 °C. The slurry was then stirred at room temperature for 60 minutes and then added to an azeotropically dried solution of m-PEG$_{67}$-OTs (80 g, 25 mmol, 3.5 eq) in anhydrous toluene (195 ml) under $N_2$ at 0 °C. The reaction mixture was heated to reflux overnight and stirred under $N_2$. The reaction was monitored by HPLC and upon completion the temperature was lowered to 15 °C and the reaction was quenched by a dropwise addition of $H_2O$ (3 ml). The pH of the crude product was adjusted to a pH of 5-7 with 1.0 M HCl (6.5 ml). The reaction mixture was diluted with $H_2O$ (533 ml), the temperature was increased to 60 °C and NaCl (80 mg) was added. The mixture was then stirred for 45 minutes extracted three times with EtOAc (300 ml). To the remaining aqueous phase NaCl (16 g) was added and the mixture was additionally extracted three times with EtOAc (300 ml). The fourth and fifth extracted fractions had an acceptable product purity (HPLC) and were dried over $MgSO_4$, filtered through double GF/A filters and the solvent evaporated. The resulting residues were dissolved in $H_2O$ (500 ml) and the pH was adjusted to pH 8 with 0.8 M aqueous solution of $NaHCO_3$ (30 ml). The aqueous phase was extracted three times with DCM (100 mL). The organic phase was then dried over $MgSO_4$, filtered and evaporated to obtain the desired compound **11** (11.1 g, 49.0 % yield, 98.3 % purity (HPLC-ELSD)).
$^1$H NMR (400 MHz, $CDCl_3$) δ 5.77 (m, 2H), 5.02 (m, 4H), 3.81 (m, 4H), 3.70-3.60 (s, 875H), 3.37 (s, 6H), 3.22 (s, 4H), 2.04 (d, 4H).

*Example 4b: Compound **12**, 1,7-bis(triethoxvsilyl)-4,4-bis(ω-methyl-(ethyleneoxy)$_{67}$-methyl)-hepotane:*

To the azeotropically dried solution of compound **11** from example 4a (10 g, 1.6 mmol) in toluene (77.5 ml), triethoxysilane (10.4 g, 64 mmol, 40 eq) was added at 22 °C under nitrogen. Karstedt's catalyst (235.6 μl, 2 % in xylenes, 0.023 mmol, 0.0066 eq) was then added using a syringe over 30 minutes which resulted in an exotherm of ≤ 2 °C. The reaction mixture was left stirring at 22 °C under nitrogen overnight.

The reaction was monitored with $^1$H-NMR for the disappearance of the olefinic protons. The solvent was then evaporated and excess silane was removed by co-evaporating with anhydrous toluene (39 ml) for a total of four times. The residue was then redissolved in toluene (59 ml) degassed with 3 cycles of vacuum/nitrogen and stirred with activated SIR-200 resin (1.65 g) for 3 days at 60 °C. The mixture was then filtered through two GF/A filters and the remaining SIR-200 resin was additionally stirred in anhydrous toluene (39 ml) to recover the product. This step was repeated for a total of three times. The collected fractions were filtered through two GF/A filters, pooled and the solvent evaporated to obtain the desired product **12** in quantitative yield (11.22 g, ≥99 % yield, 78.3 % purity (HPLC-ELSD)).
$^1$H NMR (400 MHz, $C_6D_6$) δ 3.88 (q, 12H), 3.60-3.40 (s, 583H), 3.14 (s, 6H), 1.67 (m, 4H), 1.57 (m, 4H), 1.23 (m, 18H), 0.79 (t, 4H).

***Example 5: Synthesis of Compound 14, 1,7-bis(triethoxysilyl)-4,4-bis(ω-methyl-(ethyleneoxy)$_{90}$-methyl)-heptane***

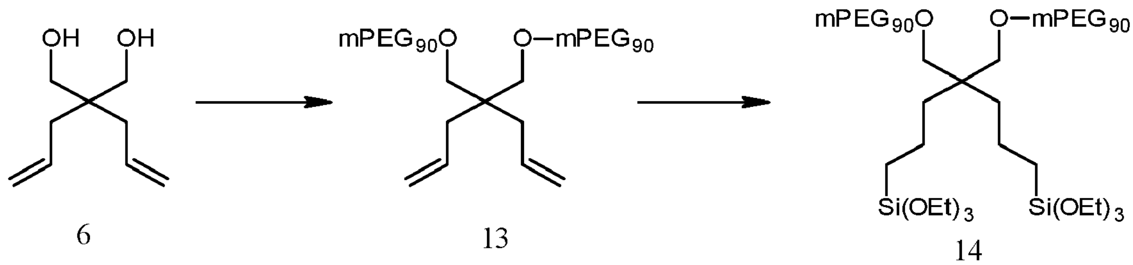

6 13 14

*Example 5a: Compound **13,** 4,4-bis(ω-methyl-(ethyleneoxy)$_{90}$-methyl)-hepta-1,6-diene*

Diallyl propanediol **6** (176.2 mg, 1.102 mmol) was dissolved in anhydrous toluene (42 ml) and cooled in an ice bath. When the inner temperature reached below 10 °C, NaH (185.2 mg, 4.628 mmol, 60 % in mineral oil, 4.2 eq) was added in three portions maintaining the temperature below 10 °C. The slurry was then stirred at room temperature for 60 minutes and then added to an azeotropically dried solution of m-PEG$_{90}$-OTs (17 g, 3.86 mmol, 3.5 eq) in anhydrous toluene (42 ml) under $N_2$ at 0 °C. The reaction mixture was heated to reflux overnight and stirred under $N_2$. The following day additional NaH (22 mg, 0.917 mmol, 0.5 eq) was added and the mixture was stirred at reflux for 4 hours. Crushed NaOH (50 mg) was added to the mixture and stirring was resumed at reflux under $N_2$ overnight. The reaction was monitored by HPLC and upon completion the temperature was lowered to 15 °C and the reaction was quenched by a dropwise addition of $H_2O$ (1 ml). The pH of the crude product was adjusted to a pH of 5-7 with 1.0 M HCl (2.7 ml). The reaction mixture was diluted with $H_2O$ (113 ml), the temperature was increased to 60 °C and NaCl (17 g) was added. The mixture was then stirred for 45 minutes extracted three times with EtOAc (60 ml). To the remaining aqueous phase NaCl (6 g) was added and the mixture was additionally extracted three times with EtOAc (60 ml). The last extracted fraction had an acceptable product purity (HPLC) and was dried over $MgSO_4$, filtered through double GF/A filters and the solvent evaporated. The resulting residue was dissolved in $H_2O$ (30 ml) and the pH was adjusted to pH 8 with 0.8 M aqueous solution of $NaHCO_3$ (10 ml). The aqueous phase was extracted three times with DCM (11 mL). The organic phase was then dried over $MgSO_4$, filtered and evaporated to obtain the desired compound **13** (3.2 g, ≥ 35.6 % yield, 83 % purity (HPLC-ELSD)).
$^1$H NMR (400 MHz, $CDCl_3$) δ 5.80 (m, 2H), 5.02 (m, 4H), 3.81 (m, 4H), 3.70-3.60 (s, 875H), 3.38 (s, 6H), 3.23 (s, 4H), 2.05 (d, 4H).

*Example 5b: Compound **14,** 1,7-bis(triethoxysilyl)-4,4-bis(ω-methyl-(ethyleneoxy)$_{90}$-methyl)-hepotane:*

To the azeotropically dried solution of compound **13** from example 5a (3 g, 0.272 mmol) in toluene (23.25 ml), triethoxysilane (1.75 g, 10.88 mmol, 40 eq) was added at 22 °C under nitrogen. Karstedt's catalyst (40.06 μl, 2 % in xylenes, 0.00386 mmol, 0.0066 eq) was then added using a syringe over 30 minutes which resulted in an exotherm of ≤ 2 °C. The reaction mixture was left stirring at 22 °C under nitrogen overnight.

The reaction was monitored with $^1$H-NMR for the disappearance of the olefinic protons. The solvent was then evaporated and excess silane was removed by co-evaporating with anhydrous toluene (11.7 ml) for a total of four times. The residue was then redissolved in toluene (17.7 ml) degassed with 3 cycles of vacuum/nitrogen and stirred with activated SIR-200 resin (300 mg) for 3 days at 60 °C. The mixture was then filtered through two GF/A filters and the remaining SIR-200 resin was additionally stirred in anhydrous toluene (11.7 ml) to recover the product. This step was repeated for a total of three times. The collected fractions were filtered through two GF/A filters, pooled and the solvent evaporated to obtain the desired product **14** as a white solid (2.73 mg, 32.1 % yield, 27.1 % purity (HPLC-ELSD)).
$^1$H NMR (400 MHz, $C_6D_6$) δ 3.94 (q, 12H), 3.60-3.40 (s, 752H), 3.14 (s, 6H), 1.22 (m, 18H),

***Example 6 (prophetic example): Synthesis of Compound 16, 1,7-bis(triethoxysilyl)-4,4-bis(ω-methyl-(ethyleneoxy)$_{112}$-methyl)-heptane***

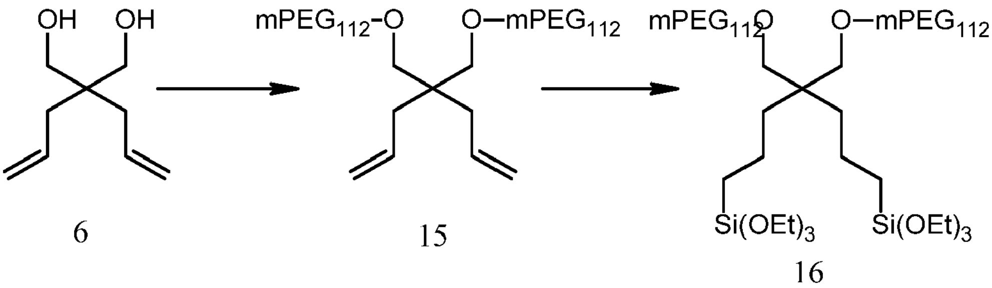

6 15 16

<u>*Example 6a (prophetic example): Compound **15,** 4,4-bis(ω-methyl-(ethyleneoxy)$_{112}$-methyl)-hepta-1,6-diene*</u>

Prior to starting the reaction, water from the starting material is removed using a Dean-Stark apparatus. The two alcohol groups of compound **6** are deprotonated with a strong base such as NaH and are coupled with an azeotropically dried solution of a PEGylating reagent carrying a suitable leaving group such as m-PEG$_{112}$-OTs to obtain **15**.

<u>*Example 6b (prophetic example): Compound **16,** 1,7-bis(triethoxysilyl)-4,4-bis(w-methyl-(ethyleneoxy)$_{112}$-methyl)-hepotane*</u>

Prior to starting the reaction, water from the starting material is removed using a Dean-Stark apparatus. The two allyl groups of compound **15** are hydrosilylated with a silane such as $(EtO)_3SiH$ and a catalyst, such as a platinum compound, such as Karstedt's catalyst. Any residual platinum is optionally removed by treatment with activated SIR-200 to obtain the final compound **16**.

***Example 7: Synthesis of compound 21, 1,7-bis(triethoxysilyl)-4-(2,2-bis(ω-methyl-(ethyleneoxy)$_{45}$-methyl)-ethyl)-heptane***

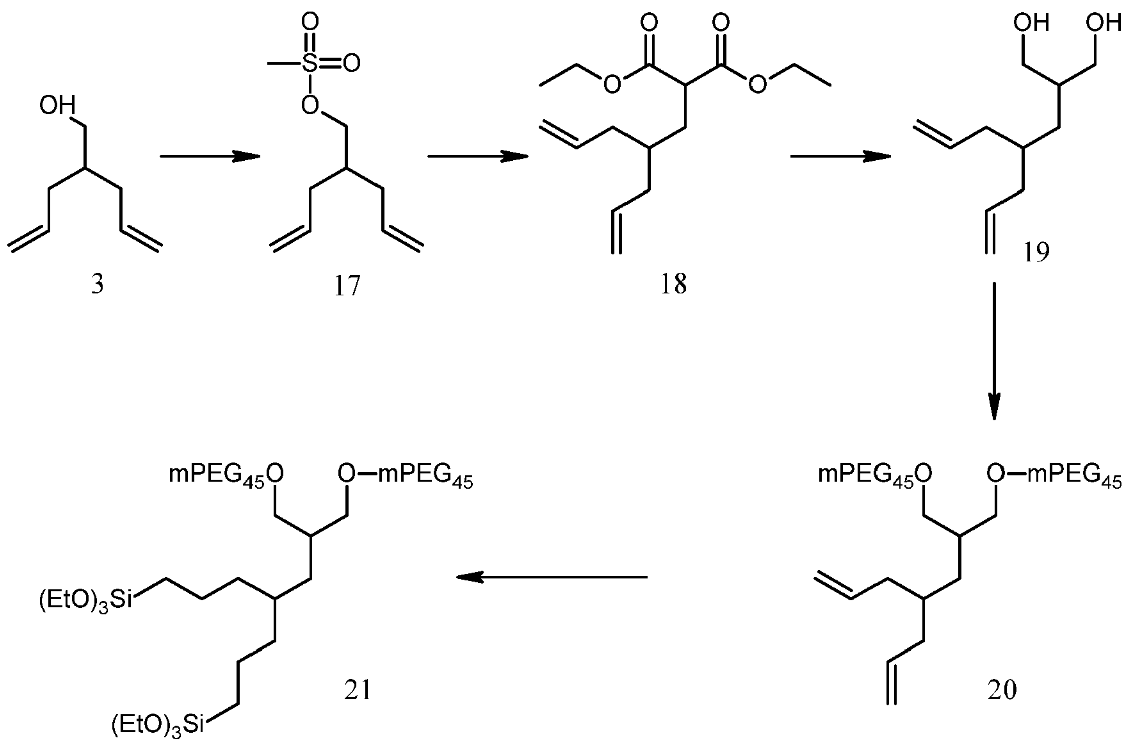

3 17 18 19 21 20

<u>*Example 7a: Compound **17,** methanesulfonic acid 2-allylpent-4-enyl ester*</u>

In a 100 ml round bottom diallyl alcohol **3** (1.00 g, 7.92 mmol) was dissolved in DCM (40 ml). The temperature

of the reaction was set to 0 °C and triethyl amine (1.1 ml, 11.9 mmol, 1.5 eq) was added. After stirring for 30 minutes MsCl (0.914, 9.5 mmol, 1.2 eq) was added in portions. The mixture was brought to room temperature and stirred for an additional 3 hours. The reaction was monitored by TLC until complete conversion of the starting material and quenched by the addition of 0.5 M HCl (36 ml). The two layers were left to separate and the organic phase was removed. The aqueous phase was extracted three times with DCM (36 ml). The combined organic phases were dried over anhydrous $MgSO_4$, filtered and evaporated under vacuum to obtain compound **17** as a colorless oil (1.387 g, 85.7 % yield).
$^1H$ NMR (400 MHz, $CDCl_3$) δ: 5.76 (m, 2H), 5.09 (d, 4H), 4.13 (d, *J* = 5.6 Hz, 2H), 3.00 (s, 3H), 2.16 (t, *J* = 7.0 Hz, 4H), 1.93 (hept, *J* = 6.4 Hz, 1H).

*Example 7b: Compound **18,** (2-allyl-pent-4-enyl)-diethyl malonate*

NaH (48.6 mg, 1.17 mmol, 60% dispersion in mineral) was suspended in dry DMF (1.7 ml) and THF (850 μl) and cooled to 0 °C. Diethyl diallyl malonate **1** (187 mg, 1.17 mmol) was added dropwise and the reaction was warmed to room temperature. The crude product from example 7a was added (80 mg, 0.392 mmol) as a solution in dry THF (850 μl) followed by the addition of KI (167 mg, 1.17 mmol). The reaction mixture was then stirred at 80 °C under reflux and nitrogen overnight. The reaction was monitored by TLC until complete conversion and allowed to cool at room temperature. It was quenched with saturated $NH_4Cl$ (3 ml) and extracted with EtOAc (3 × 2 ml). The organic phases were combined, dried over $MgSO_4$, filtered and the solvent was removed under vacuum. The resulting residue was purified by a short flash chromatography (heptane : EtOAc = 7 : 1) to obtain the desired compound **18** in quantitative yield as yellow oil.
$^1H$ NMR (400 MHz, $CDCl_3$) δ: 5.74 (m, 2H), 5.04 (m, 4H), 4.19 (m, *J* = 7.1, 4H), 3.46 (t, *J* = 7.7 Hz, 1H), 2.07 (m, 4H), 1.87 (t, *J* = 7.3 Hz, 2H), 1.54 (m, *J* = 13.0, 1H), 1.26 (t, *J* = 7.1 Hz, 6H).

*Example 7c: Compound **19,** 2-(2-allyl-pent-4-enyl)-diethyl-1.3-propane diol*

A solution of compound **18** (247 mg, 0.92 mmol) from example 7b in THF (1.5 ml) was added dropwise to a suspension of $LiAlH_4$ (88.24 mg, 2.4 eq) in THF (3 ml) at 0 °C, keeping the exotherm below 5 °C during the addition. The reaction was allowed to reach room temperature and was stirred for 6 hours. The reaction was monitored by TLC until complete conversion. The temperature was lowered to 0 °C and the reaction was quenched by the addition of $H_2O$ (1.0 ml) followed by 15 % NaOH (0.2 ml) and then diluted additionally with 1 ml of $H_2O$. A white precipitate was observed and the suspension was filtered through two GF/A filters and the filter cake washed with 2 ml THF. The volatiles were removed under vacuum and the resulting residue was purified by flash chromatography (Heptane: EtOAc 3:2, followed by Heptane : EtOAc 1:1) to obtain the desired product **19** (114 mg, 67.4 % yield).
$^1H$ NMR (400 MHz, $CDCl_3$) δ: 5.83 - 5.68 (m, 2H), 5.02 (d, 4H), 3.80 (dd, *J* = 10.6, 3.7 Hz, 2H), 3.63 (dd, *J* = 10.6, 7.6 Hz, 3H), 2.14 - 1.98 (m, 4H), 1.96 - 1.85 (m, 1H), 1.57 (dq, *J* = 12.9, 6.5 Hz, 1H), 1.16 (t, *J* = 7.0 Hz, 2H).

*Example 7d: Compound **20,** 2-(2-allyl-pent-4-enyl)-diethyl-1,3-bis(ω-methyl-(ethyleneoxy)$_{45}$-methyl)-propane*

Compound **19** (110 mg, 0.6 mmol) from the example 7c was dissolved in anhydrous toluene (15 ml) and cooled in an ice bath. When the inner temperature reached below 10 °C, NaH (111 mg, 4.2 mmol, 90 % in mineral oil, 7 eq) was added in portions. The mixture was then stirred at room temperature for 30 minutes and an azeotropically dried solution of m-$PEG_{45}$-OTs (5.43 g) in toluene (25 ml) was added under $N_2$ at 0 °C. The reaction mixture was heated to reflux overnight and stirred under $N_2$. The reaction was monitored by HPLC and upon completion the temperature was lowered to 15 °C and the reaction was quenched by a slow addition of $H_2O$ (0.3 ml). The solvent was evaporated and the residue dissolved in $H_2O$ (72 ml). NaCl (5.42 g) was added at 60 °C and the reaction mixture was extracted once with EtOAc (25 ml). To the remaining aqueous phase NaCl (1.80 g) was added and the mixture was extracted with EtOAc (25 ml) at 60 °C. The organic phases were combined, dried over $MgSO_4$, filtered and the solvent was evaporated. The resulting residue was purified by flash chromatography (C18 column $H_2O$ : acetonitrile 63 : 47) to obtain the desired product **20** (485 mg, 19 % yield).
$^1H$ NMR (400 MHz, $CDCl_3$) δ 5.74 (m, 2H), 5.00 (m, 4H), 3.71-3.60 (s, 225H), 3.39 (m, 6H), 2.20 (m, 4H), 1.25 (s, 4H)

*Example 7d: Compound **21,** 1,7-bis(triethoxysilyl)-4-(2,2-bis(ω-methyl-(ethyleneoxy)$_{45}$-methyl)-ethyl)-hepotane*

To the azeotropically dried solution of the compound **20** (0.13 g, 0.035 mmol) from the example 7d in dry toluene (2 ml) triethoxysilane (0.22 g, 1.3 mmol, 40 eq) was added. The bath temperature was set to 30 °C and Karstedt's catalyst (5 μl, 2% in xylenes) was added. The reaction mixture was left stirring at room temperature overnight. The reaction was monitored with $^1H$-NMR for the disappearance of the olefinic protons. The solvent was then evaporated and excess silane was removed by co-evaporating with anhydrous toluene (2 ml). The product was dissolved in toluene (2 ml)

degassed with 3 cycles of vacuum/nitrogen and stirred with activated SIR-200 (50 mg) for 2 days at 60 °C. Filtration and evaporation of the solvent gave the final compound **21** as a colorless solid.

***Example 8: Synthesis of compound 34, 1,9-bis(triethoxysilyl)-5,5-bis(ω-methyl-(ethyleneoxy)$_{45}$-methyl)-nonane***

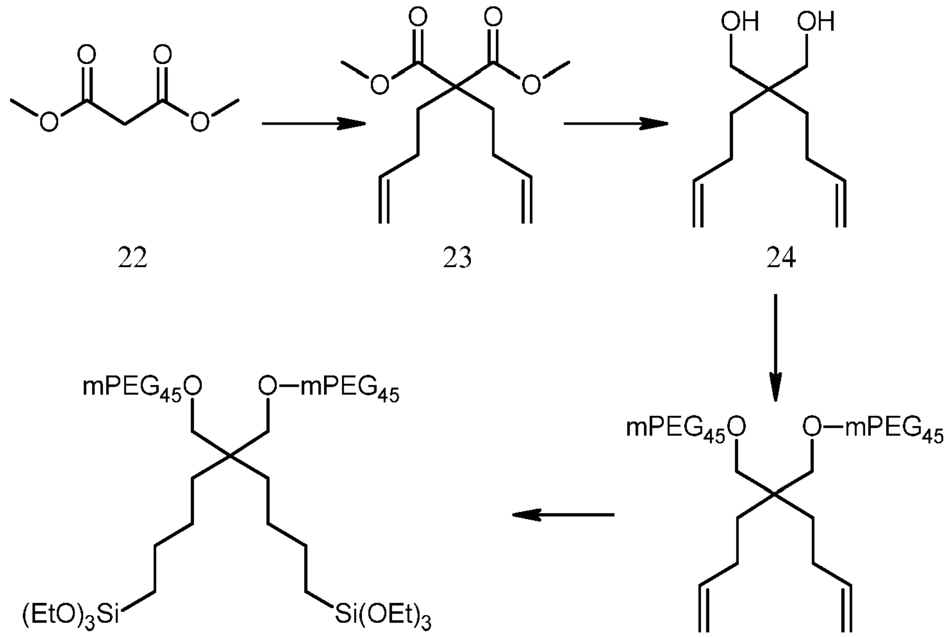

22 23 24

25

26

*Example 8a: Compound **23**, 2,2-Di-but-3-enyl-malonic acid diethyl ester*

In a 100 ml round bottom flask NaH (2.4 g, 0.1 mol, 60% dispersion in mineral) was suspended in 50 ml dry DMF at 0 °C under nitrogen. Dimethyl malonate **22** (2.3 ml, 0.02 mmol) was added to the mixture at 0 °C followed by the addition of 4-bromo-1-butene (5.20 ml, 0.052 mmol, 2.6 eq). The suspension was then stirred at room temperature for 2 hours and then gradually heated to 70 °C and left stirring overnight. The next day the reaction was allowed to cool down to room temperature and was quenched with $NH_4Cl$ (50 ml, sat. aq.). The reaction mixture was extracted three times with 100 ml diethyl ether. The organic phases were combined, dried over $MgSO_4$, filtered and the solvent was removed under vacuum. The resulting residue was purified by flash chromatography (heptane : EtOAc = 9 : 1) to obtain the desired product **23** as a yellow oil (4.06 g, 84.7% yield).
$^1H$ NMR (400 MHz, $CDCl_3$) δ: 5.77 (m, 2H), 5.00 (m, 4H), 3.72 (s, 6H), 1.98 (m, 8H).

*Example 8b: Compound **24**, 2,2-Di(but-3-enyl)propane-1,3-diol*

A solution of compound **23** (3.59 g, 0.015 mmol) from example 8a in THF (15 ml) was added dropwise to a suspension of $LiAlH_4$ in THF (30 ml) at 0 °C, keeping the exotherm below 5 °C during the addition. The reaction was stirred overnight at room temperature and monitored by TLC until complete conversion. The reaction was quenched by the addition of $H_2O$ (4.0 ml) followed by 15 % NaOH (4.0 ml) and finally 20 ml of $H_2O$. A white precipitate was observed and the suspension was filtered through two GF/A filters and the filter cake washed twice with 20 ml of THF. The suspension was extracted three times with DCM (50 ml). The combined organic phases were washed with brine (150 ml), dried over $MgSO_4$, filtered and the solvent was removed under vacuum. The resulting residue was purified by flash chromatography (heptane : EtOAc = 7 : 3) to obtain the desired product **24** (2.0 g, 70 % yield).
$^1H$ NMR (400 MHz, $CDCl_3$) δ: 5.82 (m, $J$ = 16.8 Hz, 2H), 5.04 (d, $J$ = 17.26 Hz, 2H), 4.96 (d, $J$ = 10.15 Hz, 2H), 3.60 (s, 4H), 2.01 (m, 4H), 1.40 (m, 4H).

*Example 8c: Compound **25**, 5,5-bis(ω-methyl-(ethyleneoxy)$_{45}$-methyl)-nona-1,8-diene*

A mixture of NaH (272 mg, 10.8 mmol, 95 % in mineral oil, 2.9 eq), compound **24** (1.0 g, 3.73 mmol) from example 8b and m-PEG$_{45}$-OTs (19.41 g, 8.9 mmol, 2.4 eq) in 100 ml anhydrous THF was heated to 40 °C and stirred overnight under $N_2$. The reaction was monitored by TLC and upon completion it was quenched by a slow addition of $H_2O$ (5 ml). The reaction mixture was dried over $MgSO_4$, filtered and the solvent was evaporated to obtain a white residue. To remove the impurities from the residue the crude was first dissolved in DCM (50 ml) and then precipitated by the addition of diethyl ether (200 ml). White solids were filtered off and the precipitation process was repeated for a total of three times to obtain the desired product **25**.
$^1$H NMR (400 MHz, $CDCl_3$) δ: 5.80 (m, 2H), 4.98 (m, 4H), 3.70-3.60 (s, 659H), 3.54 (m, 4H), 3.37 (s, 6H), 2.03 (s, 4H), 1.36 (m, 4H).

*Example 8d: Compound **26**, 1,9-bis(triethoxysilyl)-5,5-bis(ω-methyl-(ethyleneoxy)$_{45}$-methyl)-nonane.*

To the azeotropically dried solution of compound **25** from example 8c (3.0 g, 0.724 mmol) in anhydrous toluene (25 ml), triethoxysilane (550 μL, 2.94 mmol, 4.0 eq) was added. The bath temperature was set to 30 °C and Karstedt's catalyst (100 μl, 2% in xylenes) was added. The reaction mixture was left stirring at room temperature overnight. The reaction was monitored with $^1$H-NMR for the disappearance of the olefinic protons. The solvent was then evaporated and excess silane was removed by co-evaporating with anhydrous toluene (40 ml). The product was dissolved in toluene (50 ml) degassed with 3 cycles of vacuum/nitrogen and stirred with activated SIR-200 (2.28 g) for 4 days at 60 °C. After filtering and washing three times with toluene (50 ml) the solvent was evaporated and compound **26** (2.419 g, 75.6 % yield) was obtained as a colorless solid.
$^1$H NMR (400 MHz, $CDCl_3$) δ: 3.88 - 3.73 (m, 12H), 3.62 (s, 365H), 3.52 (m, 4H), 3.35 (s, 6H), 1.20 (q, $J$ = 6.9 Hz, 18H).

***Example 9: Synthesis of compound 30, 1,11-bis(triethoxysilyl)-6,6-bis(ω-methyl-(ethyleneoxy)$_{45}$-methyl)-undecane***

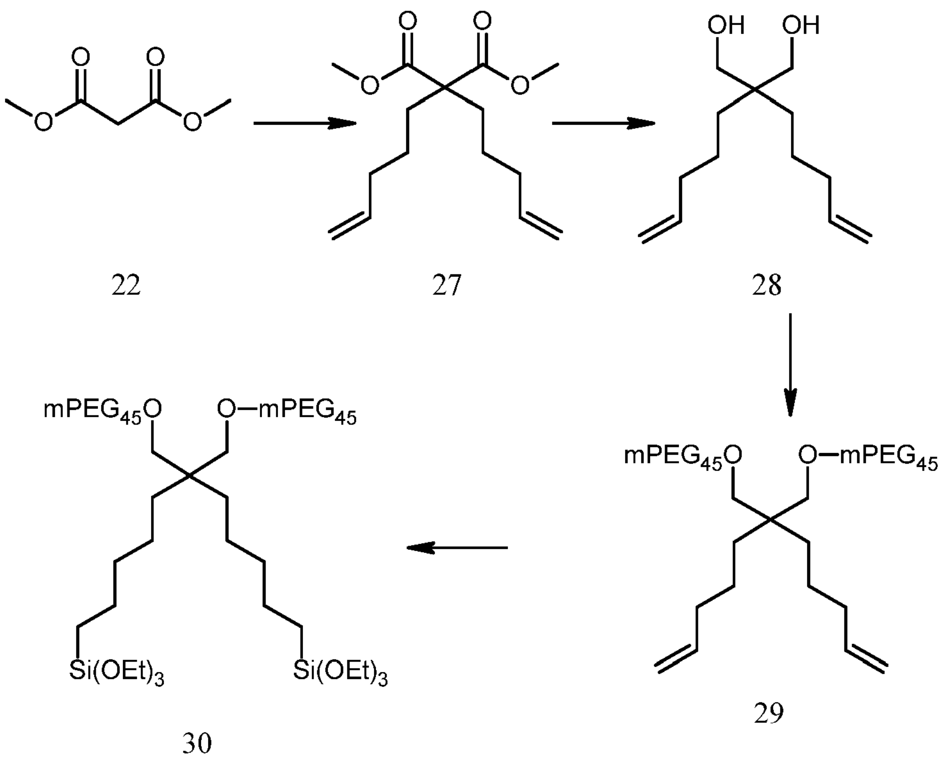

22 27 28

29

30

*Example 9a: Compound **27**, 2,2-Di-pent-4-enyl-malonic acid diethyl ester*

In a 100 ml round bottom flask NaH (2.4 g, 0.1 mol, 60% dispersion in mineral) was suspended in 50 ml dry DMF at 0 °C under nitrogen. Dimethyl malonate **22** (2.3 ml, 0.02 mmol) was added to the mixture at 0 °C followed by

the addition of 1-bromo-4-pentene (6.2 ml, 0.1 mmol). The suspension was then stirred at room temperature for 2 hours and then gradually heated to 50 °C and left stirring overnight. The next day the reaction was allowed to cool down to room temperature and was quenched with $NH_4Cl$ (50 ml, sat. aq.). The reaction mixture was extracted three times with diethyl ether (100 ml). The organic phases were combined, dried over $MgSO_4$, filtered and the solvent was removed under vacuum. The resulting residue was purified by flash chromatography to obtain the desired product **27** (4.0 g, 56 % yield).
$^1$H NMR (400 MHz, $CDCl_3$) δ: 5.78 (m, 2H), 4.99 (m, 4H), 3.72 (s, 6H), 2.06 (m, *J* = 7.50 Hz, 4H), 1.89 (m, 4H), 1.26 (m, *J* = 7.50 Hz, 4H).

*Example 9b: Compound **28,** 2,2-Di(pent-4-enyl)-propane-1,3-diol*

A solution of compound **27** (3.59 g, 0.015 mmol) from example 9a in THF (15 ml) was added dropwise to a suspension of $LiAlH_4$ in THF (30 ml) at 0 °C, keeping the exotherm below 5 °C during the addition. The reaction was stirred overnight and monitored by TLC until complete conversion. The reaction was quenched by the addition of $H_2O$ (4 ml) followed by 15 % NaOH (4 ml) and white precipitate was observed. The suspension was filtered through two GF/A filters and the filter cake washed twice with 20 ml THF. The suspension was then transferred to a separation funnel and extracted three times with DCM (50 ml). The combined organic phases were washed with brine (150 ml), dried over $MgSO_4$, filtered and the solvent was removed under vacuum. The resulting residue was purified by flash chromatography (heptane : EtOAc = 9 : 1) to obtain the desired product **28** (2.0 g, 70 % yield).
$^1$H NMR (400 MHz, $CDCl_3$) δ: 5.80 (m, 2H), 4.98 (d, 4H), 3.57 (s, 4H), 2.04 (m, 4H), 1.30 (m, 8H).

*Example 9c: Compound **29,** 6,6-bis(ω-methyl-(ethyleneoxy)$_{45}$-methyl)-undec-1,10-diene*

Compound **28** (1 g, 3.75 mmol) from the example 9b was dissolved in anhydrous toluene (80 ml) and cooled in an ice bath. When the inner temperature reached below 10 °C, NaH (176 mg, 7 mmol, 95 % in mineral oil, 7 eq) was added in portions. The mixture was then stirred at room temperature for 30 minutes and an azeotropically dried solution of m-PEG$_{45}$-OTs (13.2 g, 6.08 mmol, 6 eq) in toluene (100 ml) was added under $N_2$ at 0 °C. The reaction mixture was heated to reflux overnight and stirred under $N_2$. The reaction was monitored by HPLC and upon completion the temperature was lowered to 15 °C and the reaction was quenched by a slow addition of $H_2O$ (20 ml). The solvent was evaporated and the residue was purified by flash chromatography (C18 column $H_2O$ : acetonitrile 60 : 40) to obtain the desired product **29** (2.0 g, 70 % yield).
$^1$H NMR (500 MHz, $CDCl_3$) δ: 5.78 (m, *J* = 16.9 Hz, 2H), 4.94 (m, 4H), 3.69-3.60 (s, 391H), 3.53 (m, 4H), 3.37 (s, 6H), 1.99 (m, 4H), 1.25 (m, 8H).

*Example 9d: Compound **30,** 1,11-bis(triethoxysilyl)-6,6-bis(ω-methyl-(ethyleneoxy)$_{45}$-methyl)-undecane*

To the azeotropically dried solution of compound **29** from example 9c (0.91 g, 0.216 mmol) in toluene (7 ml), triethoxysilane (1.47, 7.9 mmol, 40 eq) was added. The reaction temperature was set to 30 °C and Karstedt's catalyst (32 μl, 2 % in xylene) was added. The reaction mixture was left stirring at room temperature overnight. The reaction was monitored with $^1$H-NMR for the disappearance of the olefinic protons. The solvent was then evaporated and excess triethoxysilane was removed by co-evaporating with anhydrous toluene (4 ml). The product was dissolved in toluene (6 ml) degassed with 3 cycles of vacuum/nitrogen and stirred with activated SIR-200 (300 mg) for 4 days at 60 °C. After filtering and washing three times with toluene (10 ml) the solvent was evaporated and compound **30** was obtained as a colorless solid (85,3 % purity (HPLC-ELSD)).

***Example 10: Synthesis of compound 43, 1,5-bis(triethoxysilyl)-3,3-bis(ω-methyl-(ethyleneoxy)$_{45}$-methyl)-pentane***

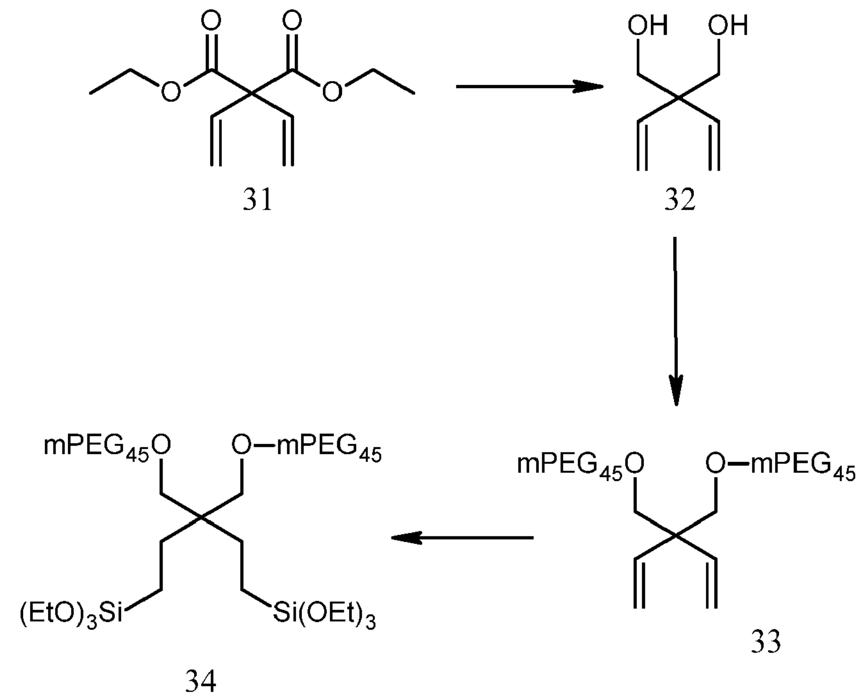

31 32 34 33

*Example 10a: Compound **32**, 2,2-divinly-propan-1,3-diol*

A solution of compound **31** (214 mg, 1.01 mmol) in THF (2 ml) was added dropwise to a suspension of $LiAlH_4$ in THF (5 ml) at 0 °C, keeping the exotherm below 5 °C during the addition. The reaction was stirred overnight at room temperature and monitored by TLC until complete conversion. The reaction was quenched by the addition of $H_2O$ (1 ml) followed by 15 % NaOH (250 μl). The suspension was filtered and the filter cake washed with THF (25 ml). The suspension was then transferred to a separation funnel and extracted three times with DCM (2 ml). The combined organic phases were washed with brine (5 ml), dried over $MgSO_4$, filtered and the solvent was removed under vacuum to obtain the desired product **32** in a quantitative yield.

$^1H$ NMR (400 MHz, $CDCl_3$) δ: 5.79(m, 2H), 5.32 (d, *J* = 11.0 Hz, 2H), 5.22 (d, *J* = 17.6, Hz, 3H), 3.75 (s, 4H).

*Example 10b: Compound **33**, 3,3-bis(ω-methyl-(ethyleneoxy)$_{45}$-methyl)-penta-1,4-diene*

Divinyl propanediol **32** from example 10a (152 mg, 1.2 mmol) was dissolved in anhydrous toluene (6 ml) and cooled in an ice bath. When the inner temperature reached below 10 °C, NaH (149 mg, 5.9 mmol, 95 % in mineral oil, 5 eq) was added in three portions maintaining the temperature below 10 °C. The slurry was then stirred at room temperature for 60 minutes and then added to an azeotropically dried solution of m-$PEG_{45}$-OTs (10.78 g, 4.9 mmol, 4.2 eq) in anhydrous toluene (48 ml) under $N_2$ at 0 °C. The reaction mixture was heated to reflux overnight and stirred under $N_2$. The reaction was monitored by HPLC and upon completion the temperature was lowered to 15 °C and the reaction was quenched by a dropwise addition of $H_2O$ (3 ml). The solvent was evaporated and the residue dissolved in DCM (20 ml). $MgSO_4$ was added and the suspension was stirred for 20 minutes. Volatiles were then evaporated under vacuum and the residual $MgSO_4$ was filtered off to obtain the desired product **33** as a colorless solid in quantitative yield.

*Example 10c: Compound **34** (prophetic example), 1,5-bis(triethoxysilyl)-3,3-bis(ω-methyl-(ethyleneoxy)$_{45}$-methyl)-pentane*

Prior to starting the reaction, water from the starting material is removed using a Dean-Stark apparatus. The two vinyl groups of compound **33** are hydrosilylated with a silane such as $(EtO)_3SiH$ and a catalyst, such as a platinum compound, such as Karstedt's catalyst. Any residual platinum is optionally removed by treatment with activated SIR-200 to obtain the final compound **34**.

***Example 11: Synthesis of compound 37, 1,6-bis(triethoxysilyl)-3,4-bis(ω-methyl-(ethyleneoxy)$_{45}$-methyl)-hexane***

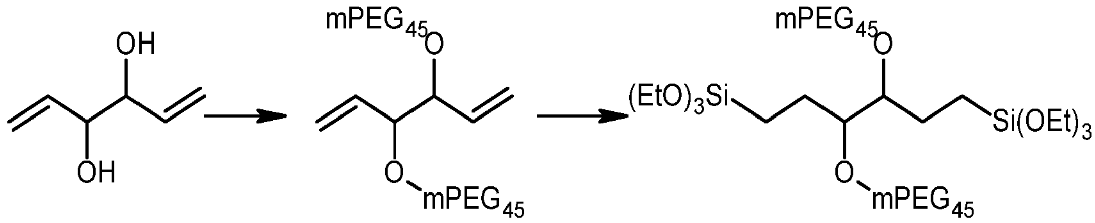

35 36 37

*Example 11a: Compound* ***36,*** *3,4-bis(ω-methyl-(ethyleneoxy)$_{45}$)-hexa-1,5-diene*

Divinyl glycol **35** (15 mg, 1.3 mmol) was dissolved in anhydrous toluene (20 ml) and cooled in an ice bath. When the inner temperature reached below 10 °C, NaH (232 mg, 9.2 mmol, 95 % in mineral oil, 7 eq) was added in three portions maintaining the temperature below 10 °C. The slurry was then stirred at room temperature for 60 minutes and then added to an azeotropically dried solution of m-PEG$_{45}$-OTs (8.28 g, 3.8 mmol, 3.2 eq) in anhydrous toluene (25 ml) under $N_2$ at 0 °C. The reaction mixture was heated to reflux overnight and stirred under $N_2$. The reaction was monitored by HPLC and upon completion the temperature was lowered to 15 °C and the reaction was quenched by a dropwise addition of $H_2O$ (3 ml). The solvent was evaporated and the residue dissolved in DCM (20 ml). $MgSO_4$ was added and the suspension was stirred for 20 minutes. Volatiles were then evaporated under vacuum and the residual $MgSO_4$ was filtered off to obtain the desired product **36** as a colorless solid in quantitative yield.

*Example 11b (prophetic example): Compound* ***37,*** *1,6-bis(triethoxysilyl)-3,4-bis(ω-methyl-(ethyleneoxy)$_{45}$)-hexane*

Prior to starting the reaction, water is removed from the starting material using a Dean-Stark apparatus. The two vinyl groups of compound **36** are hydrosilylated with a silane such as $(EtO)_3SiH$ and a catalyst, such as a platinum compound, such as Karstedt's catalyst. Any residual platinum is removed by treatment with activated SIR-200 to obtain the final compound **37**.

***Example 12 (prophetic example): Synthesis of compound 43***

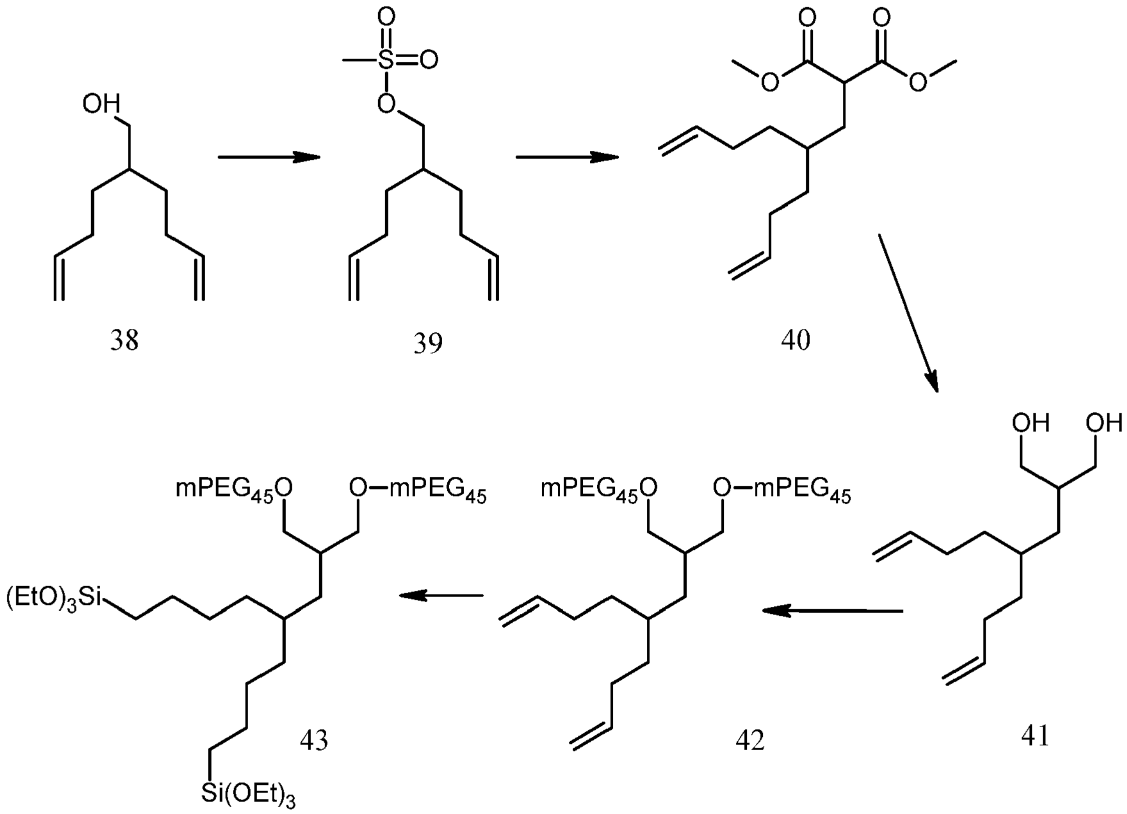

38 39 40 41 42 43

Compound **43** is synthetized by first activating the alcohol group in **38** as the mesylate **39** which is then allowed to undergo nucleophilic substitution reaction with dimethyl malonate to obtain **40**. The two ester groups are reduced with a reducing agent such as $LiAlH_4$ to obtain the diol **41**. The two alcohol groups are deprotonated with a strong base such as NaH and are coupled with a PEGylating reagent carrying a suitable leaving group such as m-$PEG_{45}$-OTs to obtain **42**. Finally, the two allyl groups are hydrosilylated with $(EtO)_3SiH$ and a catalyst, such as Karstedt's catalyst to obtain **43**.

***Example 13 (prophetic example): Synthesis of compound 49***

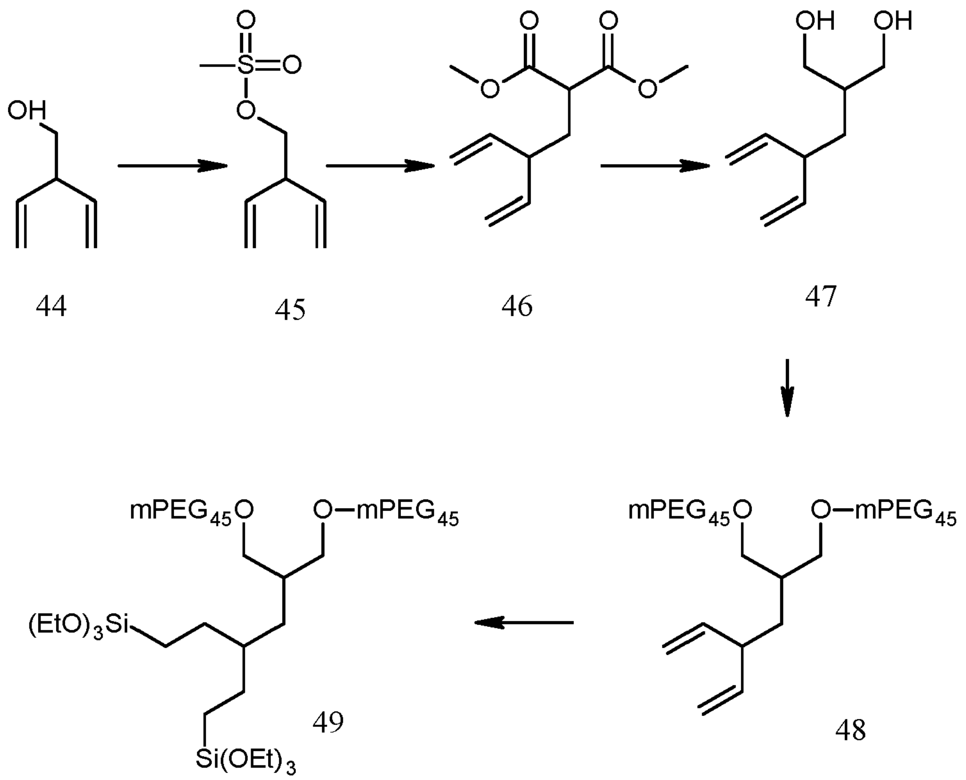

44 45 46 47 48 49

Compound **49** is synthetized by first activating the alcohol group in **44** as the mesylate **45** which is then allowed to undergo nucleophilic substitution reaction with dimethyl malonate to obtain **46**. The two ester groups are reduced with a reducing agent such as $LiAlH_4$ to obtain the diol **47**. The two alcohol groups are deprotonated with a strong base such as NaH and are coupled with a PEGylating reagent carrying a suitable leaving group such as m-$PEG_{45}$-OTs to obtain **48**. Finally, the two allyl groups are hydrosilylated with a $(EtO)_3SiH$ and a catalyst, such as Karstedt's catalyst to obtain **49**.

***Example 14: Use*** **of** ***compounds according to the present disclosure as a coating precursors***

Compounds **10**, **5**, **12** and **14** were used as coating precursors for coating nanostructures.

*Example 14a: Use of 1,7-bis(triethoxysilyl)-4,4-bis($\omega$-methyl-(ethyleneoxy)$_{45}$-methyl)-heptane, compound* ***10,*** *as a coating precursor*

Precursor nanostructures were synthesized by refluxing 93.60 g of 1,7-bis-(triethoxysilyl)-4,4-bis-(dimethylphosphonato)-heptane, **52**, in 4501 g of 90.0 % (v/v) aqueous ethylene glycol for 48 hours, resulting in a solution of precursor nanostructures with a hydrodynamic diameter of 17.6 nm and a phosphorus concentration of 69 mM.

Primed precursor nanostructures were synthesized by heating 4338 g of the solution of precursor nanostructures with 116.48 g of bis(triethoxysilyl)methane at 100 °C for 4 hours, resulting in a solution of primed nanostructures with a hydrodynamic diameter of 19.7 nm and a phosphorus concentration of 64 mM.

A 10-liter jacketed reactor equipped with a mechanical stirrer, a temperature probe, an addition funnel heated to 40 °C by a heating fan, and a reflux condenser topped with a connection to a $N_2$/vacuum manifold was charged with 6330 g of 90 % aqueous ethylene glycol and 3902 g of the solution of primed precursor nanostructures.

The jacket temperature was set to 125 °C for 1 hour before being lowered to 110 °C. A coating solution was prepared by dissolving 548.2 g of 1,7-bis(triethoxysilyl)-4,4-bis($\omega$-methyl-(ethyleneoxy)$_{45}$-methyl)-heptane in 548.2 g of anhydrous ethanol under heating to 40 °C. When the inner temperature in the reactor reached 105 °C, 250 ml of the coating solution was added. The remainder of the coating solution was added slowly via the addition funnel at 50 ml/h. The jacket temperature was kept at 110 °C until 48 hours after the initial addition of coating solution to the reactor before cooling down to 20 °C.

The reaction mixture was transferred to a 20-liter jug fitted with a 4-port lid. The reactor was rinsed with ultrapure water (5 + 4 liter) and the rinses added to the jug. The jug was shaken to mix the contents. The contents were filtered into a fresh 20-liter jug through an autoclaved filter assembly consisting of a 1 $\mu$m, a 0.4 $\mu$m, and a 0.2 $\mu$m filter connected in series. The filtered solution was purified by ultrafiltration using a 5400 $cm^2$ 300 kD TFF filter until the concentration

of ethylene glycol was below 500 ppm.

Average diameter (DLS) = 26.7 nm, Đd = 1.19, [P](ICP-OES) = 60 mM, [Si](ICP-OES) = 220 mM.

*Example 14b: Use of 4-[ω-methyl-(ethyleneoxy)$_{45}$-methyl]-1,7-bis(triethoxysilyl)heptane, Compound* ***5,*** as a *coating precursor*

A coating solution was prepared by dissolving 1056 mg of 4-[ω-methyl-(ethyleneoxy)$_{45}$-methyl]-1,7-Bis(triethoxysilyl)heptane in an equal mass of anhydrous dioxane at 40 °C. A 2-necked pear-shaped flask equipped with a reflux condenser topped with a connection to a $N_2$/vacuum manifold was charged with 5 ml of a solution of primed nanostructures with a hydrodynamic diameter of 20.1 nm and a phosphorus concentration of 150 mM, prepared similarly to the solution used in Example 14a, and heated for 10 minutes in a 110 °C oil bath. A 200 μl portion of the coating solution was added to the nanostructure solution. A further 1700 μl of the coating solution was added over 20 hours via a syringe pump, the syringe and needle being heated to ~ 40 °C by a heating fan. The reaction temperature was kept at 110 °C until 48 hours after the initial addition of coating solution before being allowed to cool down to ambient temperature. The solution was filtered through a 0.2 μm PES filter, diluted with water and purified by ultrafiltration using a 115 $cm^2$ 300 kD TFF filter.

Nanostructures synthesized in this manner do not aggregate on exposure to 20 mM $CaCl_2$.

Average diameter (DLS) = 29.4 nm, Đd = 1.14, [P](ICP-OES) = 59 mM, [Si](ICP-OES) = 263 mM.

*Example 14c: Use of 1,7-bis(triethoxysilyl)-4,4-bis(ω-methyl-(ethyleneoxy)$_{67}$-methyl)-heptane, compound* ***12,*** *as a coating precursor*

A 13.4 ml sample of a solution of primed nanostructures nanostructures with a hydrodynamic diameter of 19.2 nm and a phosphorus concentration of 64 mM, prepared similarly to the solution used in Example 14a, was diluted to 36.1 ml with 90 % (v/v) aqueous ethylene glycol in a 2-necked round bottom flask with the vertical neck equipped with a reflux condenser topped connection to a vacuum-nitrogen manifold and the side neck stoppered with a septum. The solution was heated for 20 minutes in a preheated 110 °C oil bath before 1500 μl of a 50 % (w/w) solution of 1,7-bis(triethoxysilyl)-4,4-bis(ω-methyl-(ethyleneoxy)$_{67}$-methyl)heptane in ethanol heated to 40 °C was added. A further 5200 μl of the warm 1,7-bis(triethoxysilyl)-4,4-bis(ω-methyl-(ethyleneoxy)$_{67}$-methyl)heptane solution was added by syringe pump at a speed of 266 μl/hour. After a total of 48 hours the solution was allowed to cool to ambient temperature.

The resulting nanostructure solutions was diluted to 150 ml with water and filtered through double glass fiber filters and subsequently through a 0.2 μm polyethersulfone (PES) syringe filter. The resulting solution was diluted to 800 ml with water and concentrated to approximately 20 ml using a tangential flow filtration setup with a 70 kD nominal cut off. The dilution - concentration procedure was repeated for a total of 4 times before the solution was further concentrated to 20 ml on a spin filter with a 300 kD nominal cut off.

Nanostructures synthesized in this manner do not aggregate on exposure to 20 mM $CaCl_2$.

Average diameter (DLS) = 32.5 nm, $Đ_d$ = 1.38, [P](ICP-OES) = 45 mM, [Si](ICP-OES) = 233 mM.

*Example 14d: Use of 1,7-bis(triethoxysilyl)-4,4-bis(ω-methyl-(ethyleneoxy)$_{90}$-methyl)-heptane, compound* ***14,*** as a *coating precursor*

A 4.1 ml sample of a solution of primed nanostructures with a hydrodynamic diameter of 19.2 nm and a phosphorus concentration of 64 mM, prepared similarly to the solution used in Example 14a, was diluted to 11 ml with 90 % (v/v) aqueous ethylene glycol in a 2-necked round bottom flask with the vertical neck equipped with a reflux condenser topped connection to a vacuum-nitrogen manifold and the side neck stoppered with a septum. The solution was heated for 15 minutes in a preheated 110 °C oil bath before 530 μl of a 50 % (w/w) solution of 1,7-bis(triethoxysilyl)-4,4-bis(ω-methyl-(ethyleneoxy)$_{91}$-methyl)heptane in ethanol heated to 40 °C was added. A further 2120 μl of the warm 1,7-bis(triethoxysilyl)-4,4-bis(ω-methyl-(ethyleneoxy)$_{90}$-methyl)heptane solution was added by syringe pump at a speed of 109 μl/hour. After a total of 48 hours the solution was allowed to cool to ambient temperature.

The resulting nanostructure solutions was diluted to 40 ml with water and filtered through double glass fiber filters and subsequently through a 0.2 μm polyethersulfone (PES) syringe filter. The resulting solution was diluted to 800 ml with water and concentrated to approximately 20 ml using a tangential flow filtration setup with a 70 kD nominal cut off. The dilution - concentration procedure was repeated for a total of 4 times before the solution was further concentrated to 10 ml on a spin filter with a 300 kD nominal cut off.

Nanostructures synthesized in this manner do not aggregate on exposure to 20 mM $CaCl_2$.

Average diameter (DLS) = 41.2 nm, $Đ_d$ = 1.59, [P](ICP-OES) = 24 mM, [Si](ICP-OES) = 116 mM.

***Example 15: Use of compounds outside the scope of the present disclosure as a coating precursors (Reference examples)***

Compounds **51** and **52** are compounds outside the scope of the present disclosure and used as reference examples.

*Example 15a: Use of 1-(ω-methy-(ethyleneoxy)$_{45}$-methyl)-3,5-bis(3-(triethoxysilyl)propyloxy)-benzene, compound **51**, as a coating precursor (Reference example)*

A coating solution was prepared by dissolving 2500 mg of 1-(ω-methyl-(ethyleneoxy)$_{45}$-methyl)-3,5-bis(3-(triethoxysilyl)propyloxy)-benzene, **51**, in an equal mass of anhydrous dioxane at 40 °C. A 2-necked pear-shaped flask equipped with a reflux condenser topped with a connection to a $N_2$/vacuum manifold was charged with 8 ml of a solution of primed nanostructures with a hydrodynamic diameter of 15.1 nm and a phosphorus concentration of 99 mM, prepared similarly to the solution used in Example 14a, and heated for 5 minutes in a 110 °C oil bath. A 300 μl portion of the coating solution was added to the nanostructure solution. A further 1970 μl of the coating solution was added over 20 hours via a syringe pump, the syringe and needle being heated to ~ 40 °C by a heating fan. The reaction temperature was kept at 110 °C until 48 hours after the initial addition of coating solution before being allowed to cool down to ambient temperature. The solution was filtered through a 0.2 μm PES filter, diluted with water and purified by ultrafiltration using a 115 $cm^2$ 300 kD TFF filter.

Average diameter (DLS) = 29.4 nm, Đd = 1.33, [P](ICP-OES) = 59 mM, [Si](ICP-OES) = 387 mM.

*Example 15b: Use of 1-(ω-methyl-(ethyleneoxy)$_{45}$)-10-triethoxysilyl-decane compound **52**, as a coating precursor (Reference example)*

A coating solution was prepared by dissolving 425 mg of 1-(ω-methyl-(ethyleneoxy)$_{45}$)-10-triethoxysilyl-decane, **52**, in an equal mass of anhydrous dioxane at 40 °C. A reaction vial was charged with 2.5 ml of a solution of primed nanostructures with a hydrodynamic diameter of 18.4 nm and a phosphorus concentration of 104 mM, prepared similarly to the solution used in Example 14a, and heated in a 110 °C oil bath. A 70 μl portion of the coating solution was added to the nanostructure solution. A further 700 μl of the coating solution was added over 20 hours via a syringe pump, the syringe and needle being heated to ~ 40 °C by a heating fan. The reaction temperature was kept at 110 °C until 94 hours after the initial addition of coating solution before being allowed to cool down to ambient temperature. The solution was filtered through a 0.2 μm PES filter, diluted with water and purified by ultrafiltration using a 115 $cm^2$ 300 kD TFF filter.

Average diameter (DLS) = 25.3 nm, Đd = 1.19, [P](ICP-OES) = 35 mM, [Si](ICP-OES) = 150 mM.

***Example 16: Comparison between coated nanostructures coated with coating precursors according to the present disclosure and nanostructures coated with reference coating precursors outside the scope of the present disclosure.***

Nanostructures coated with compounds **5** and 10 were compared to nanostructures coated with a compound (compound **51**) outside the scope of the present disclosure.

*Example 16a: Nanostructures coated with precursors according to the present disclosure have longer plasma half-lives than nanostructures coated with reference precursors*

Coated nanostructures coated with 1,7-bis(triethoxysilyl)-4,4-bis(ω-methyl-(ethyleneoxy)as-methyl)-heptane (compound **10**), (test item A); 4-[ω-methyl-(ethyleneoxy)$_{45}$-methyl]-1,7-bis(triethoxysilyl)heptane (compound **5**), (test item B); and reference compound 1-(ω-methyl-(ethyleneoxy)$_{45}$-methyl)-3,5-bis(3-(triethoxysilyl)propyloxy)-benzene (compound **51**), (test item C, reference compound outside the scope of the present disclosure), made according to examples 14a, 14b and 15a, respectively, were loaded with yttrium in the form of $YCl_3$ so that [P]/[Y] was 23 (±1). The test items were formulated for injection by adjusting [Ca], pH, and osmolality to physiological levels with $CaCl_2$, NaOH, and mannitol, respectively. *In vivo* behavior was investigated by injecting female BALB/c mice with the test items at a dose of 2 μmol Y/kg. Blood was sampled at 1, 6, and 24 hours after injection and plasma was analyzed for [Y] and [Si] by ICP-OES. Results in the form of circulation half-lives are shown in Table 1.

***Table 1.*** *Plasma half-lives of coated nanostructures coated with coating precursors according to the present disclosure (A and B) and a reference coating precursor (C), calculated from [Y] and from [Si] as measured by ICP-OES.*

| Test item | $t_{½}$ (Y) (h) | $t_{½}$ (Si) (h) |
|---|---|---|
| A | 9.4 | 4.5 |
| B | 7.3 | 4 |
| C (reference) | 3.5 | 2.1 |

As can be seen in Table 1, coated nanostructures coated with coating precursors according to the present disclosure (A) and (B) show significantly prolonged circulation times, as compared to nanostructures coated with a reference coating precursor (C).

*Example 16b: Nanostructures coated with precursors according to the present disclosure have better storage stability than nanostructures coated with reference precursors*

Nanostructures coated with compound **51**, i.e. coated nanostructures according to Example 15a were stored at 4 °C for 48 hours. Decomposition products related to the shedding of the coating layer were detected at a total concentration of >5% (area/area) as measured by SEC-ELSD.

Nanostructures coated with compound **10**, i.e. coated nanostructures according to Example 14a were stored at 4 °C for 106 days. Decomposition products related to the shedding of the coating layer were detected at a total concentration of <3% (area/area) as measured by SEC-ELSD. This indicates that the storage stability of coated nanostructures coated with coating precursors of the present disclosure is increased approximately 2 orders of magnitude as compared to coated nanostructures coated with the reference coating precursor.

Nanostructures coated with compound **10**, i.e. coated nanostructures according to Example 14a were stored at 4 °C for 76 days. DLS was measured before (27.0 nm) and after (26.9 nm) storage, and no significant change in the diameter of the nanostructures could be detected. This indicates that the storage stability of coated nanostructures coated with coating precursors of the present disclosure with respect to aggregation is good.

***Example 17: Use of a nanostructure coated with a coating precursor according to the current disclosure in a pharmaceutical composition***

Tumors were induced in 10 weeks old female BALB/c mice by inoculation with 4T1 cells ($1 \times 10^5$ cells) injected subcutaneously in the right flank.

Coated nanostructures according to Example 14a (nanostructures coated with compound **10**) were loaded with $^{177}Lu$ to a concentration of approximately one atom of $^{177}Lu$ per nanostructure and formulated for injection by addition of tris buffer, $CaCl_2$ and saline.

When the tumor volume reached 200-300 $mm^3$, at day 14 or 15 after inoculation, the treatment group (n=16) was injected with the $^{177}Lu$ loaded nanostructure at a dose of 218 MBq/kg and the control group (n=10) received saline. Anti-tumor efficacy was monitored by following tumor growth and overall survival.

The treatment group showed significant increased mean and median survival time as compared to the control group (see Table 2). Further, the tumor growth was significantly slowed down for the treatment group. See Fig. 12.

***Table 2.*** *Median and mean survival for 4T1 tumor bearing female BALB/c mice, treated with $^{177}Lu$ loaded coated nanoparticles and control.*

| | Median survival (days after treatment) | Mean survival (days after treatment) |
|---|---|---|
| Treatment group | 13 | 14.9 |
| Control group | 10 | 10.7 |

Thus, nanostructures coated with coating precursors according to the present disclosure may be used in a pharmaceutical composition. The pharmaceutical composition may be used in the treatment of cancer. Further, it has been demonstrated that nanostructures coated with coating precursors according to the present disclosure may be used as carrier for radionuclides.

***Example 18: Use* of a *nanostructure coated with a coating precursor according to the current disclosure* as a *carrier for* a *radionuclide***

Coated nanostructures according to Example 14a (nanostructures coated with compound 10) were loaded with $^{177}Lu$ as in example 17. No unbound $^{177}Lu$ could be detected by $\gamma$-counter-SEC. Less than 2.5% of $^{177}Lu$ was mobile on radio-TLC on silica eluted with citrate buffer, the remainder being bound to the nanostructures, which were completely retained at the baseline of the TLC plate.

This demonstrates that nanostructures coated with coating precursors according to the present disclosure may be used as carrier for radionuclides.

***Example 19: Hydrosilylation of 4,4-bis($\omega$-methyl-(ethyleneoxy)$_{45}$-methyl)-hepta-1,6-diene, compound 9, under standard hydrosilylation conditions, demonstrating superiority of the method of hydrosilylation of the current disclosure***

*Reference example outside the scope of the current disclosure.*

A 10 ml round bottom flask was charged with 600 mg of azeotropically dried compound **9** (0.15 mmol), 5 ml toluene, and 100 mg of freshly distilled triethoxysilane (0.6 mmol, 4 eq.). The solution was degassed and heated to 30 °C before 11 $\mu$l addition of a solution Karstedt's catalyst (2% Pt, 1.1 $\mu$mol, 0.07 eq). Heating to 40 °C overnight gave a reaction mixture containing only 6% (a/a) of compound **10** and 73 % (area/area) of side products containing only one silyl moiety.

Notably, the synthesis of compound **10** by the method of the present disclosure, resulted in compound **10** in a purity exceeding 90% as shown in Example 3. Thus, the method of the present disclosure is superior to standard methods used for hydrosilylation.

***Example 20: (prophetic example): Recalibration of salt concentrations for extractive purification of di-PEGylated dienes according to Formula (II) for the use* of *salts other than NaCl***

A recalibration of the concentrations of NaCl used for the extractive purification of di-PEGylated dienes to the salt $M_xA_y$ is realized through interpolation of representative test extractions.

A mixture comprising a di-PEGylated diene according to Formula (II) and at least 2 of the impurities generally found in the reaction mixture when the di-PEGylated diene is synthesized through PEGylation of a diene according to Formula (III) is dissolved in water to a concentration similar to the one used in the method of extractive purification of di-PEGylated dienes. The solution is split into 8 portions, A through H.

Portion A is treated with sodium chloride to a concentration, [NaCl]i, similar to the one used for the intermediate extraction step in the method of extractive purification of di-PEGylated dienes.

Portions B, C and D are treated with $M_xA_y$ to concentrations, $[M_xA_y]_B$, $[M_xA_y]_C$, and $[M_xA_y]_D$, that are 0.75, 1, and 1.25 times $[M_xA_y]_{ref\text{-}i}$, where $[M_xA_y]_{ref\text{-}i}$ is the concentration of $M_xA_y$ that would result in a solution having the same ionic strength as a solution of NaCl in pure water at the concentration $[NaCl]_i$, assuming B, C, and D contained pure water.

Portion E is treated with sodium chloride to a concentration, $[NaCl]_p$, similar to the one used for the product extraction step in the method of extractive purification of di-PEGylated dienes.

Portions F, G and H are treated with $M_xA_y$ to concentrations, $[M_xA_y]_F$, $[M_xA_y]_G$, and $[M_xA_y]_H$, that are 0.75, 1, and 1.25 times $[M_xA_y]_{ref\text{-}p}$, where $[M_xA_y]_{ref\text{-}p}$ is the concentration of $M_xA_y$ that would result in a solution having the same ionic strength as a solution of NaCl in pure water at the concentration $[NaCl]_p$, assuming F, G and H contained pure water.

Portions A through H are each subjected to one extraction with the primary extraction solvent at a temperature above 50 °C, generating the aqueous phases $A_{aq}$-$H_{aq}$ and the organic phases $A_{org}$-$H_{org}$.

The relative composition of $A_{aq}$-$H_{aq}$ and $A_{org}$-$H_{org}$ are evaluated using a suitable analytical method, such as HPLC.

For each fraction, Qi, and each impurity present, $I_j$, is calculated the ratio

$$r(Q_i, I_j) = \frac{[diPEGylateddiene]}{[I_j]}.$$

For each impurity is prepared a plot of $r(B_{org},I_j)$, $r(C_{org},I_j)$, and $r(D_{org},I_j)$ as a function of $[M_xA_y]$, and a linear regression is used to find $[M_xA_y]$ such that r is equal to $r(A_{org},I_j)$. The average of $[M_xA_y]$ calculated in this fashion for all impurities is calculated - [MxAy]average-intermediate-org.

$[M_xA_y]_{average\text{-}intermediate\text{-}aq}$ is calculated similarly and $[M_xA_y]_{average\text{-}intermediate}$ is calculated as the average of [MxAy]average-intermediate-org and $[M_xA_y]_{average\text{-}intermediate\text{-}aq.}$ $[M_xA_y]_{average\text{-}product}$ is calculated analogously using

fractions D through H. A linear model is fitted using the points {$[NaCl]_i$: $[M_xA_y]_{average-intermediate}$} and {$[NaCl]_p$: $[M_xA_y]_{average-product}$} and said model is used to recalibrate concentrations of NaCl as concentrations of $M_xA_y$.

***Example 21: Concentration* of a *solution* of *coated nanostructures, demonstrating the high solubility in water of nanostructures coated with coating according to the present disclosure.***

A 20ml sample of nanostructures according to Example 14a (nanostructures coated with compound **10**) was concentrated on a 300 kDa spin filter. The concentration of P was measured to 328 mM by ICP-OES, corresponding to ca 25% nanostructures in solution, w/v (weight/volume). The hydrodynamic diameter was measured to 27.4 nm and no aggregates were visible on ocular inspection, indicating the absence of aggregation.

This demonstrates the high solubility and resistance to aggregation of nanostructures coated with coating according to the present disclosure.

## Claims

**1.** A compound according to Formula (I):

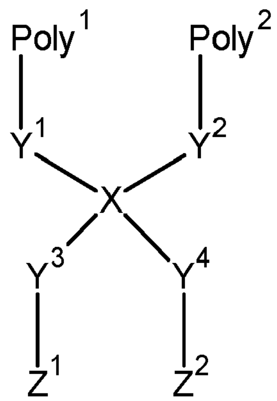

(I)

wherein

Poly$^1$ and Poly$^2$ independently are selected from the group consisting of a hydrogen and a hydrophilic polymer group having a molecular weight of 400 to 10 000 Da; wherein at least one of Poly$^1$ or Poly$^2$ is a hydrophilic polymer group having a molecular weight of 400 to 10 000 Da;
$Y^1$ and $Y^2$ independently are $-A(CH_2)_nB-$; wherein

A is bound to X;
B in $Y^1$ is bound to Poly$^1$;
B in $Y^2$ is bound to Poly$^2$;
n is an integer between 0 and 5;
A is chosen from the group consisting of -O-, -S-, -NH-, -C(=O)O-, -C(=O)NH-, -O(C=O)-, -NH(C=O)- and a covalent bond; and
B is chosen from the group consisting of -O-, -S-, -NH-, -C(=O)O-, -C(=O)NH-, -O(C=O)-, -NH(C=O)- and a covalent bond, provided that B is a covalent bond when the atom in Poly$^1$ or Poly$^2$ that is bound to $Y^1$ or $Y^2$ is not a carbon atom;

$Y^3$ and $Y^4$ independently are $-E(CH_2)_m-$; wherein

E is bound to X;
m is an integer between 2 and 5; and
E is independently chosen from the group consisting of -S-, -NH-, -C(=O)O-, -C(=O)NH-, -O(C=O)-, -NH(C=O)- and a covalent bond; and

X is chosen from the group consisting of structures $X^1$, $X^2$, $X^3$, $X^4$, and $X^5$

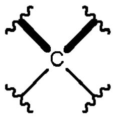
X¹

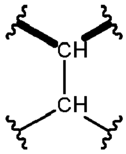
X²

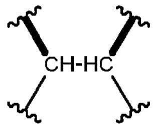
X³

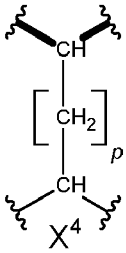
X⁴

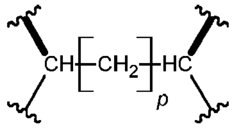
X⁵

wherein

bold bonds represent bonds to $Y^1$ or $Y^2$;
non-bold bonds represent bonds to $Y^3$ or $Y^4$; and
p is an integer between 1 and 5;
and

$Z^1$ and $Z^2$ independently are $-SiR^1R^2R^3$, wherein
$R^1$, $R^2$, and $R^3$ are independently chosen from the group consisting of chloride, bromide, iodide, lower alkoxy, aryloxy, carboxy, amino, and -NH-acyl.

**2.** The compound according to claim 1, wherein, in both $Y^1$ and $Y^2$, A is a covalent bond, B is -O-, and n is an integer between 1 and 3.

**3.** The compound according to claim 1 or 2, wherein, in both $Y^3$ and $Y^4$, E is a covalent bond.

**4.** The compound according to any one of claims 1 to 3, wherein the hydrophilic polymer group comprises a PEG chain, optionally wherein the PEG chain is terminated with a lower alkyl.

**5.** The compound according to claim 4, wherein each hydrophilic polymer group comprises 20 to 150 ethylene glycol residues.

**6.** The compound according to any one of the claims 1 to 5, wherein

$Poly^1$ and $Poly^2$ are ω-methyl-$(ethyleneoxy)_w$, wherein w is 20 to 150;
in $Y^1$ and $Y^2$, A is a covalent bond, B is -O-, and n is 1;
$Y^3$ and $Y^4$ are both -CH2-CH2-CH2-;
X is $X^1$; and
$Z^1$ and $Z^2$ are independently chosen from the group consisting of triethoxysilyl and trimethoxysilyl.

**7.** A compound according to Formula (II):

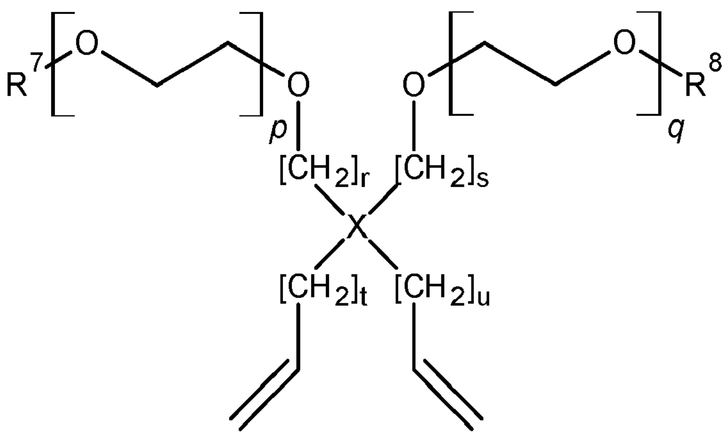

(II)

wherein

$R^7$ and $R^8$ are independently chosen from the group consisting of lower alkyls;
p is an integer between 20 and 150;
q is an integer between 20 and 150;
r is an integer between 1 and 3;
s is an integer between 1 and 3;
t is an integer between 0 and 3;
u is an integer between 0 and 3; and
X is chosen from the group consisting of $X^1$, $X^2$, $X^3$, $X^4$, and $X^5$:

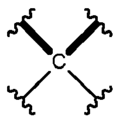
$X^1$

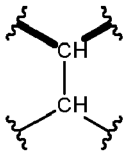
$X^2$

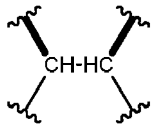
$X^3$

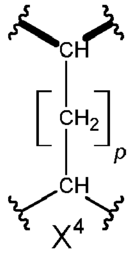
$X^4$

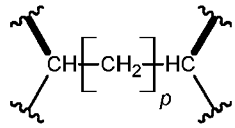
$X^5$

wherein
bold bonds represent bonds to $[CH_2]_r$ and $[CH_2]_q$; non-bold bonds represent bonds to $[CH_2]_t$ and $[CH_2]_u$; and p is an integer between 1 and 5.

**8.** Use of a compound according to claim 7, in the production of a compound according to any one of claims 4 to 6.

**9.** A method of purification of a compound according to claim 7, comprising the steps of:

a) providing an aqueous solution of an impure compound according to claim 7, wherein the aqueous solution comprises:

water in an amount of 7.5 to 16.5 times the total mass of the impure compound according to claim 7; and
NaCl in an amount of 6% to 9% (w/v) of the amount of water;

b) subjecting the aqueous solution of step a) to between 2 and 5 intermediate extractions, performed at a temperature between 40 °C and 70 °C, wherein each intermediate extraction comprises the steps of:

b1) optionally; adding a further portion of NaCl so that the total amount of NaCl added corresponds to an amount of NaCl less than 9% (w/v) of the amount of water in the aqueous solution of step a);
b2) extracting the aqueous solution with a carboxylate ester solvent; and
b3) removing the organic phase, thereby providing an aqueous phase;

c) adding NaCl to the aqueous phase from step b3) in an amount of at least 1 % of the amount of water in step a), so that the total amount of NaCl corresponds to an amount of NaCl between 8% and 12% and subjecting the aqueous phase to between 2 and 5 product extractions, performed at a temperature between 40 °C and 70 °C, wherein each product extraction comprises the steps of:

c1) extracting the aqueous phase with a carboxylate ester solvent; and
c2) removing the organic phase;

d) pooling the organic phases from each step c2);
e) concentrating the pooled organic phases from step d), thereby obtaining a residue;
f) dissolving the residue from step e) in an aqueous buffer having a pH of between 6 and 9 to provide an aqueous phase;
g) subjecting the aqueous phase from step f) to 2 to 4 polishing extractions, wherein each polishing extraction comprises the steps of:

g1) extracting the aqueous phase from step f) with a chlorinated solvent; and
g2) removing the organic phase;

h) pooling the organic phases from each step g2); and
i) concentrating the pooled organic phases from step h), thereby obtaining a residue, comprising a diPEGylated diene according to claim 7, comprising less than 10 % (w/w) impurities.

**10.** A method for producing the compound according to any one of claims 4 to 6, wherein both $Poly^1$ and $Poly^2$ comprise a PEG chain and wherein each PEG chain is terminated with a lower alkyl, the method comprising the steps of:

i) providing a diene according to claim 7, optionally purified according to the method of claim 9;
ii) contacting the diene with at least 30 equivalents of a hydrosilylation reagent having the structure $HSiR^1R^2R^3$, wherein $R^1$, $R^2$, and $R^3$ are independently chosen from the group consisting of lower alkoxy-groups, in the presence of a platinum catalyst and an aromatic hydrocarbon solvent at a temperature of between 10 and 35 °C;
iii) removing the excess of the hydrosilation reagent; and
iv) removing platinum from the product.

**11.** Use of a compound according to any one of claims 1 to 6 or claim 7, or a product of a method according to any one of claims 8, 9 or 10, as an intermediate in the production of coated nanostructures.

**12.** A globular nanostructure having a hydrodynamic diameter between 10 and 100 nm, wherein the nanostructure has a coating derived from a compound according to any one of claims 1 to 6 or claim 7, or a product of a method according to any one of claims 8, 9 or 10.

**13.** The globular nanostructure according to claim 12, further comprising a radionuclide.

**14.** A pharmaceutical composition comprising a plurality of globular nanostructures according to claim 12 or 13.

**15.** A pharmaceutical composition for use in the treatment of cancer or in imaging, wherein the pharmaceutical composition comprises a plurality of globular nanostructures according to claim 13.

**16.** Use of a globular nanostructure according to claim 12 as a carrier of radionuclides.

Fig. 1

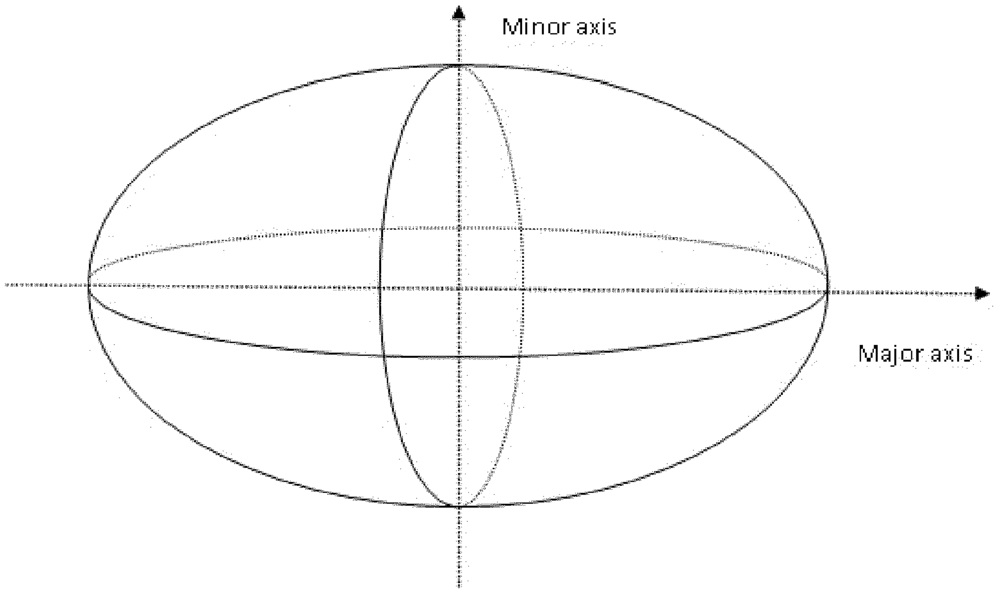


Fig. 2

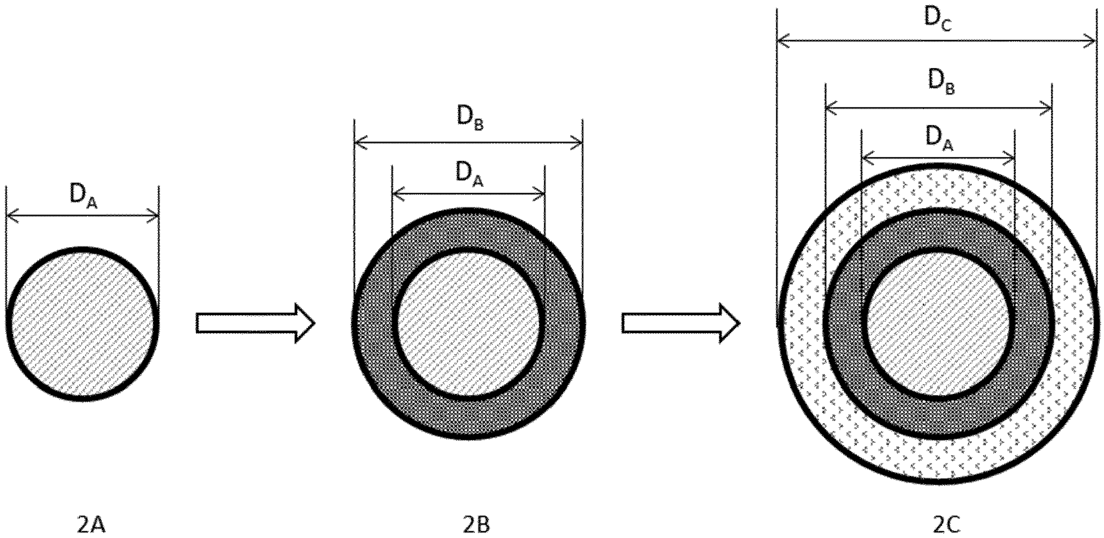

Fig. 3

stepwise extractive purification process, overview

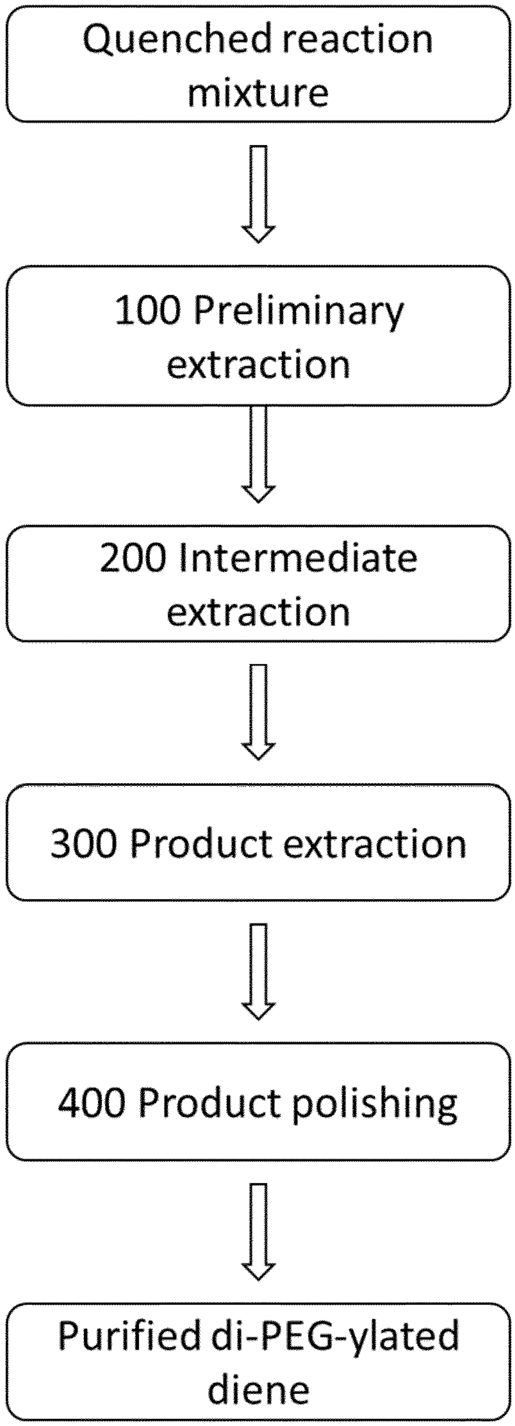

Fig. 4

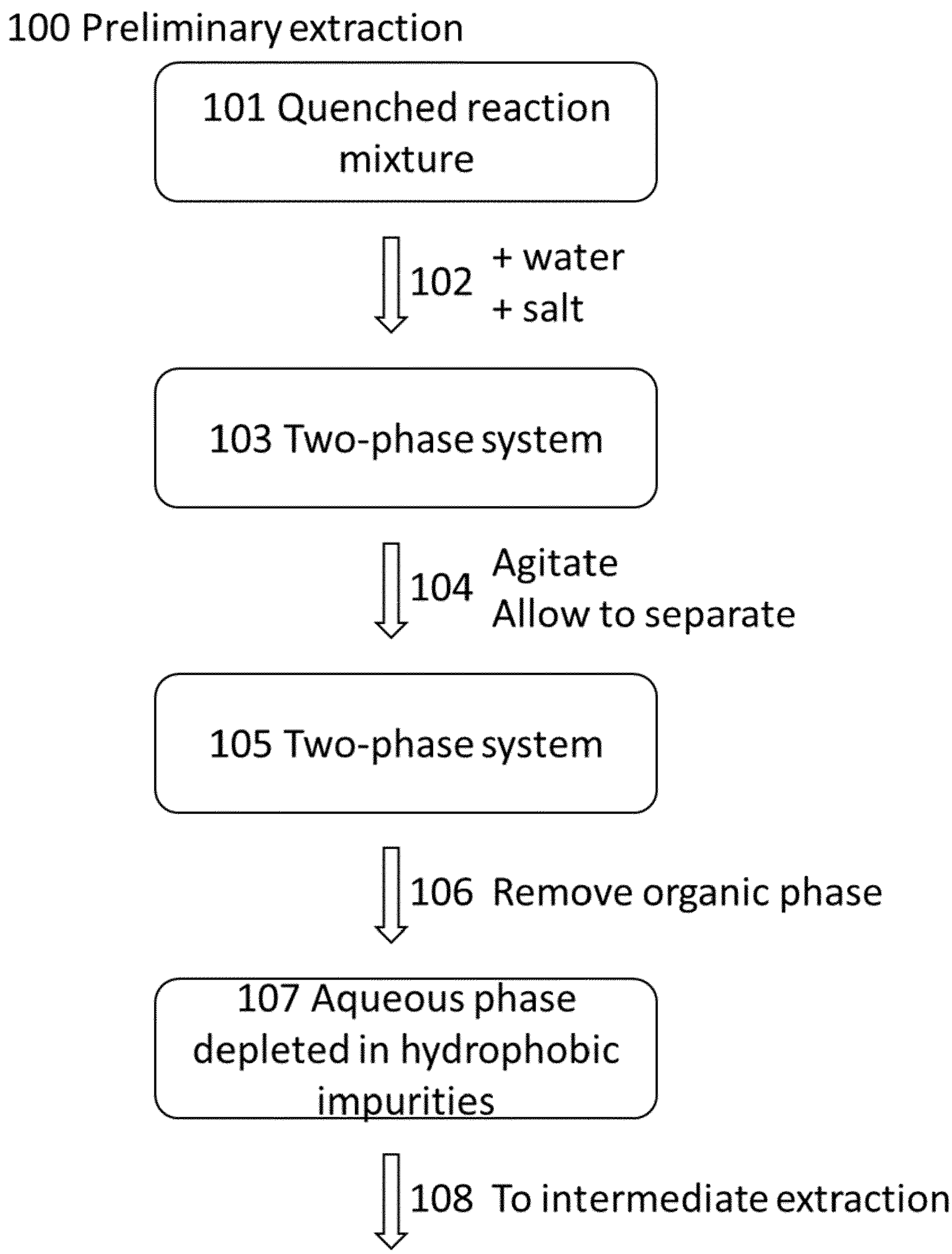
100 Preliminary extraction
101 Quenched reaction mixture
102
+ water
+ salt
103 Two-phase system
104
Agitate
Allow to separate
105 Two-phase system
106 Remove organic phase
107 Aqueous phase depleted in hydrophobic impurities
108 To intermediate extraction

Fig. 5

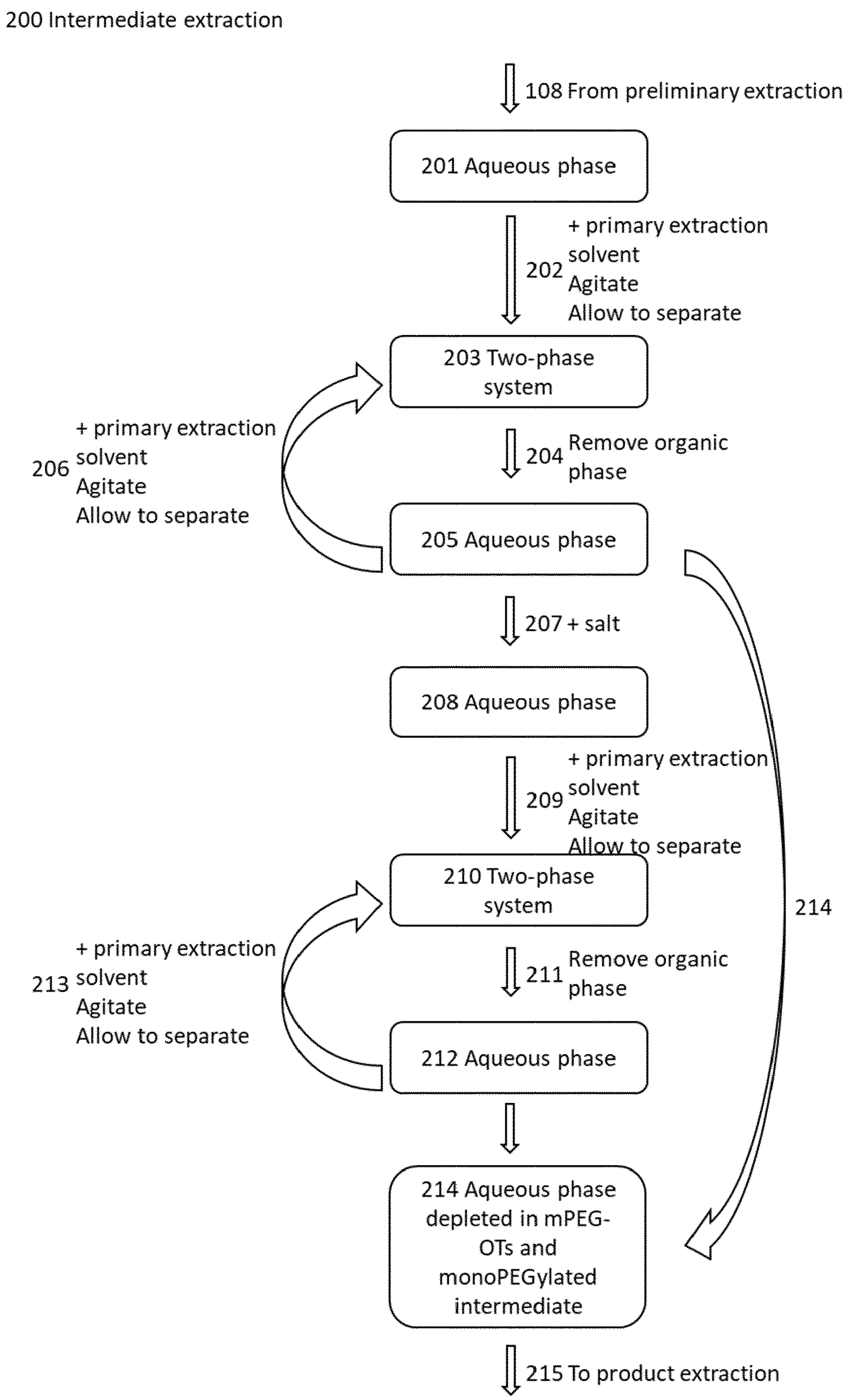
200 Intermediate extraction
108 From preliminary extraction
201 Aqueous phase
202
+ primary extraction solvent
Agitate
Allow to separate
203 Two-phase system
204 Remove organic phase
206
+ primary extraction solvent
Agitate
Allow to separate
205 Aqueous phase
207 + salt
208 Aqueous phase
209
+ primary extraction solvent
Agitate
Allow to separate
210 Two-phase system
211 Remove organic phase
213
+ primary extraction solvent
Agitate
Allow to separate
212 Aqueous phase
214
214 Aqueous phase depleted in mPEG-OTs and monoPEGylated intermediate
215 To product extraction

Fig. 6

300 Product extraction

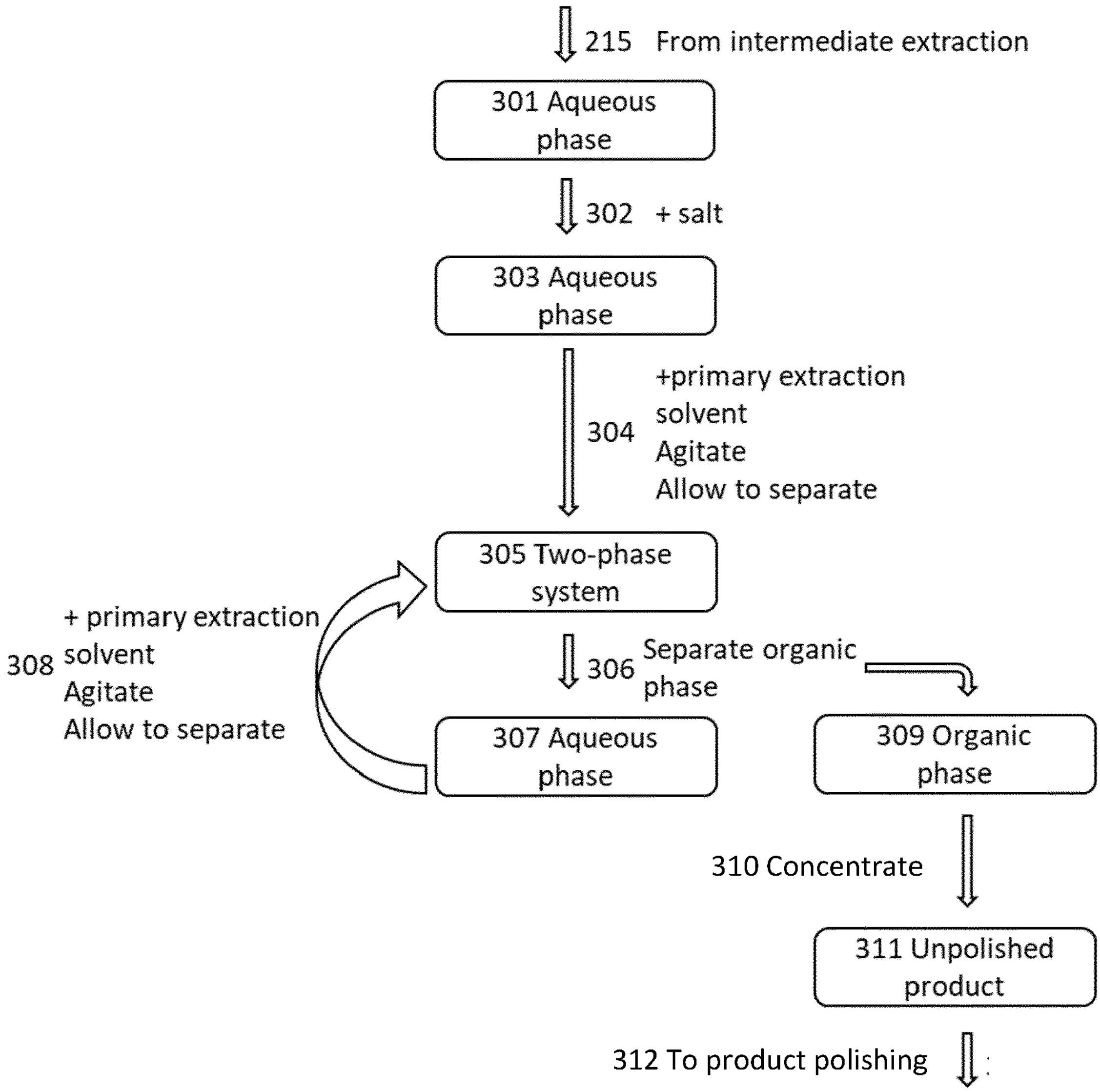

Fig. 7

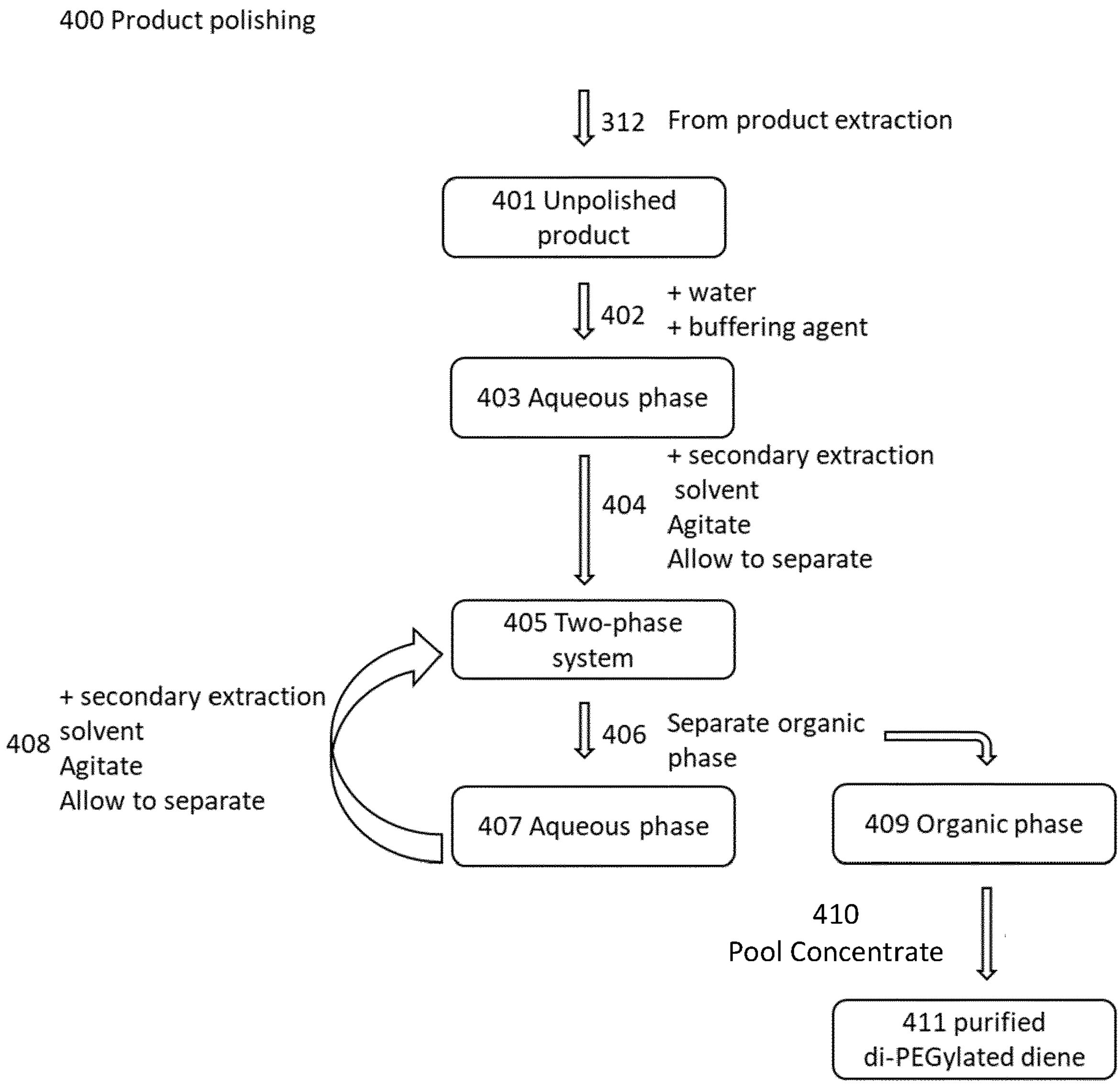
400 Product polishing
312 From product extraction
401 Unpolished product
402
+ water
+ buffering agent
403 Aqueous phase
404
+ secondary extraction solvent
Agitate
Allow to separate
405 Two-phase system
408
+ secondary extraction solvent
Agitate
Allow to separate
406
Separate organic phase
407 Aqueous phase
409 Organic phase
410
Pool Concentrate
411 purified di-PEGylated diene

Fig. 8

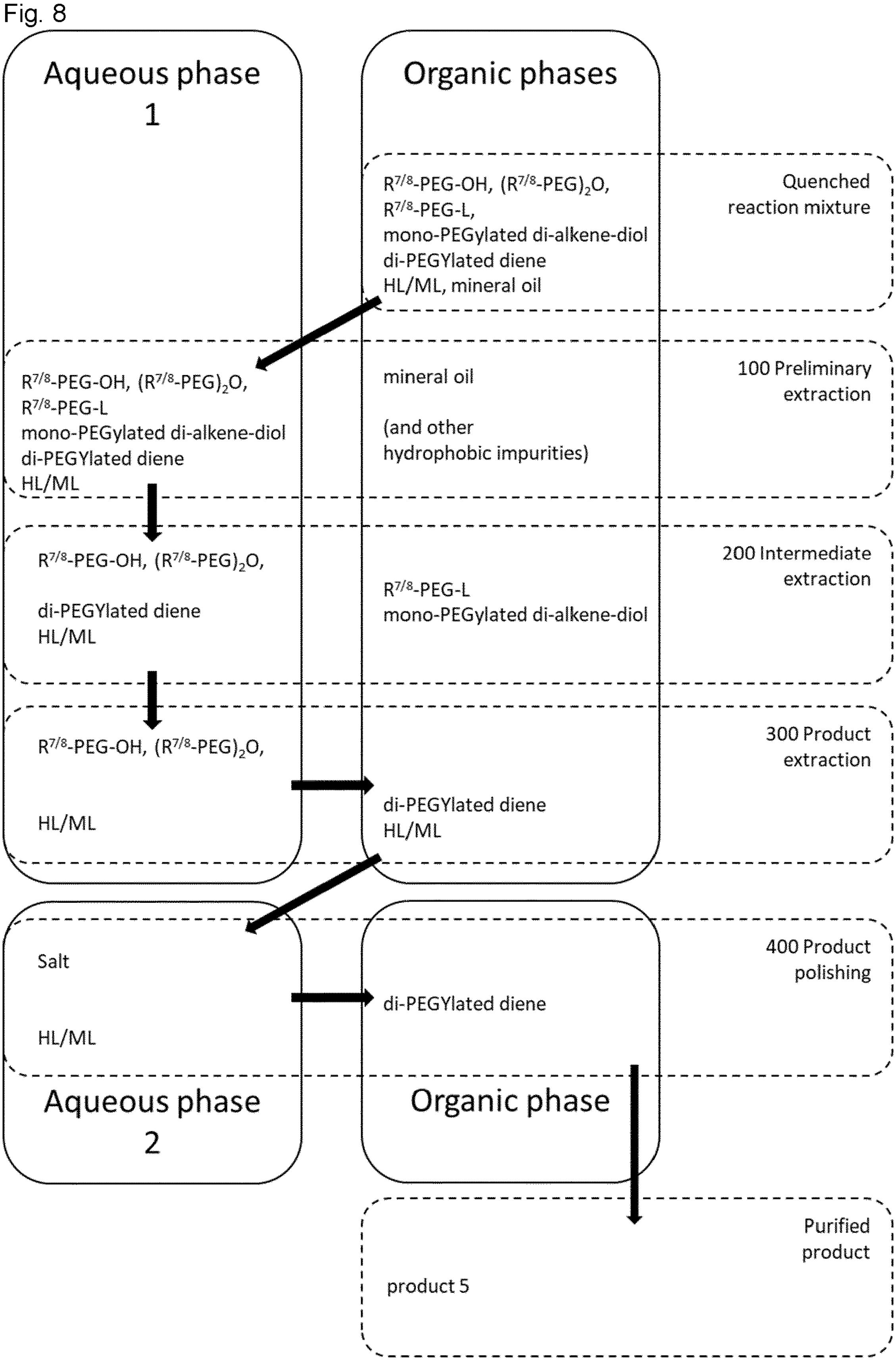
Aqueous phase 1
Organic phases
R7/8-PEG-OH, (R7/8-PEG)2O,
R7/8-PEG-L,
mono-PEGylated di-alkene-diol
di-PEGYlated diene
HL/ML, mineral oil
Quenched reaction mixture
R7/8-PEG-OH, (R7/8-PEG)2O,
R7/8-PEG-L
mono-PEGylated di-alkene-diol
di-PEGYlated diene
HL/ML
mineral oil
(and other hydrophobic impurities)
100 Preliminary extraction
R7/8-PEG-OH, (R7/8-PEG)2O,
di-PEGYlated diene
HL/ML
R7/8-PEG-L
mono-PEGylated di-alkene-diol
200 Intermediate extraction
R7/8-PEG-OH, (R7/8-PEG)2O,
HL/ML
di-PEGYlated diene
HL/ML
300 Product extraction
Salt
HL/ML
di-PEGYlated diene
400 Product polishing
Aqueous phase 2
Organic phase
product 5
Purified product

Fig. 9

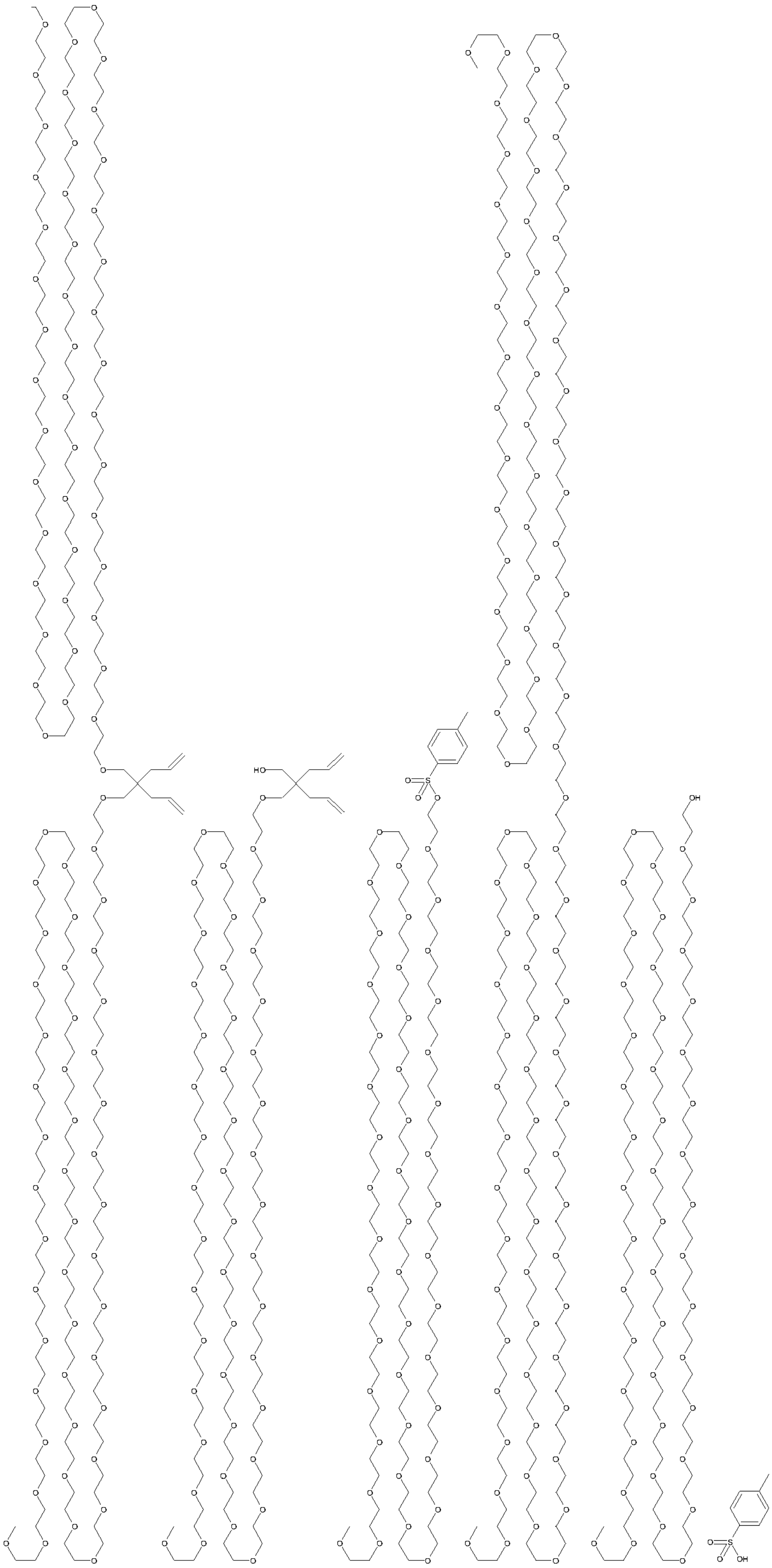

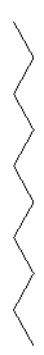

Fig. 10

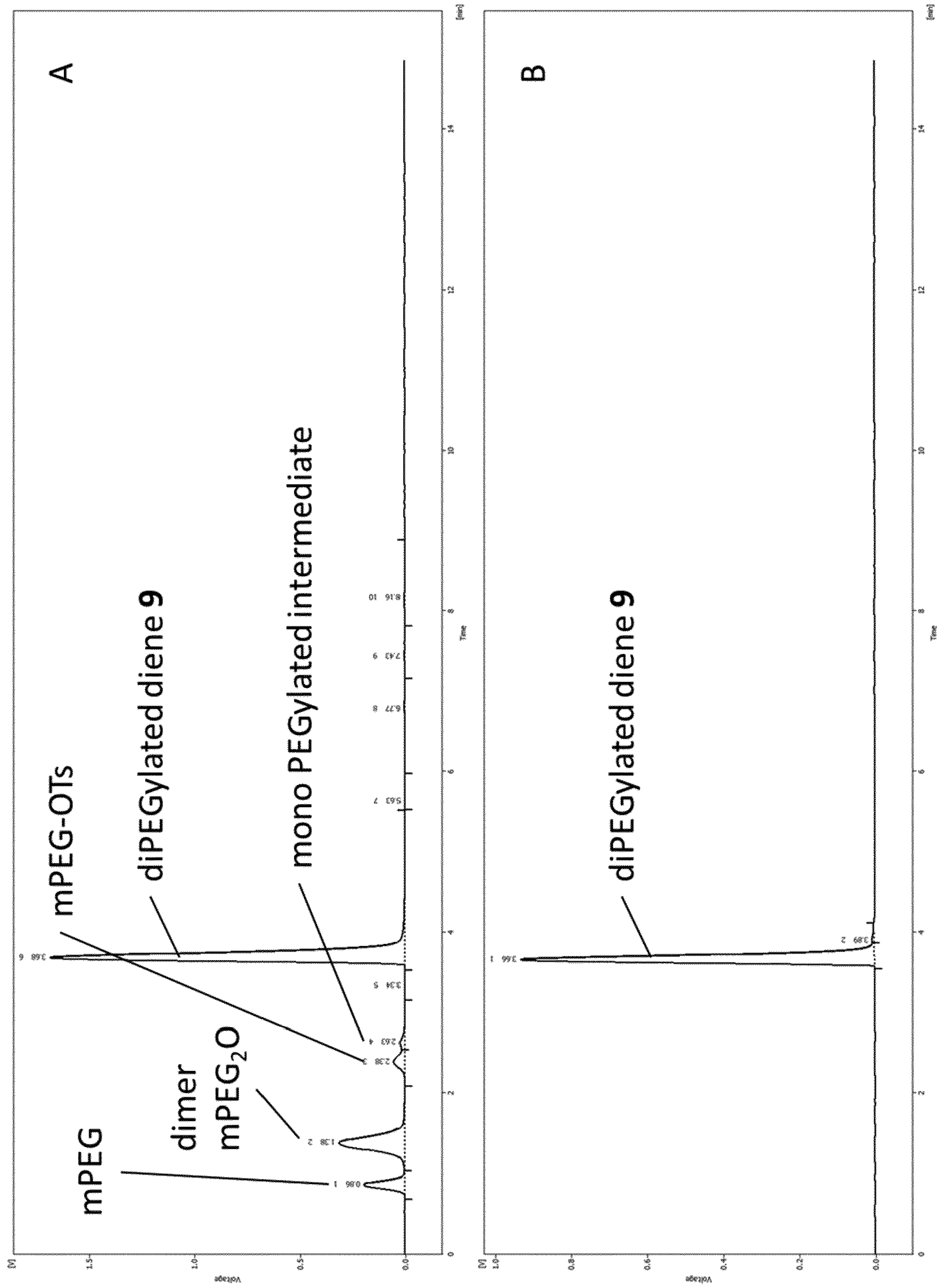
A
mPEG
dimer
$mPEG_2O$
mPEG-OTs
diPEGylated diene 9
mono PEGylated intermediate
B
diPEGylated diene 9
Voltage
Time

Fig. 11

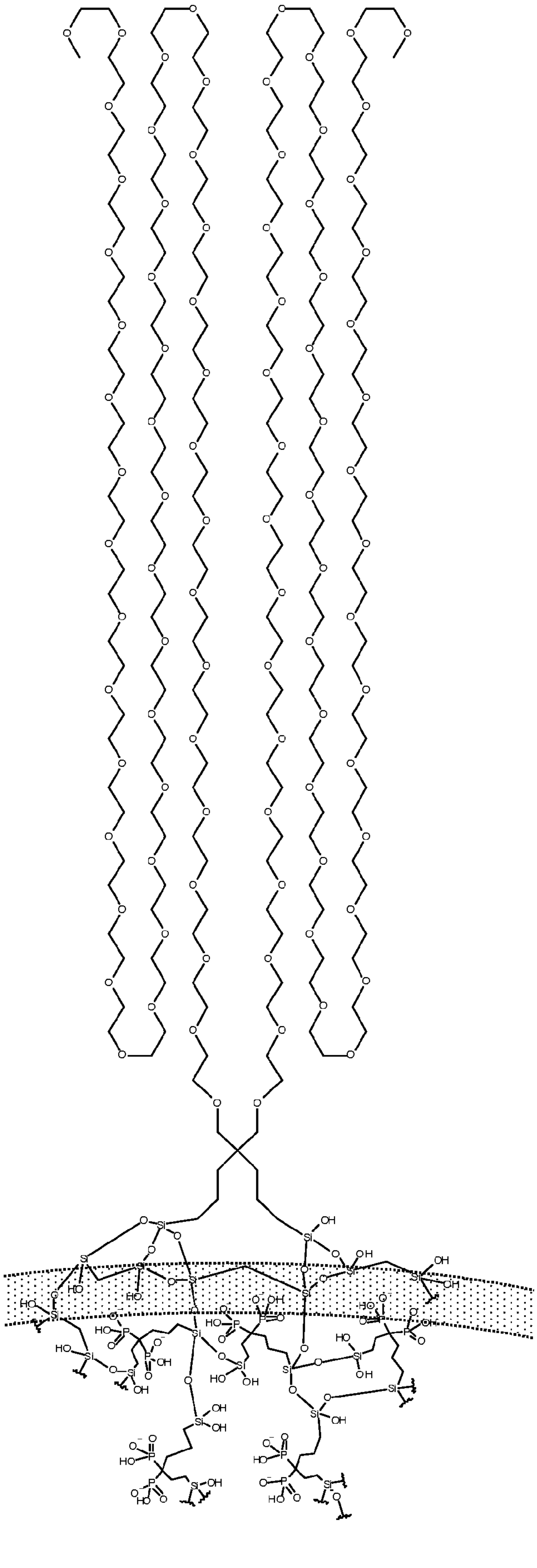

Fig. 12

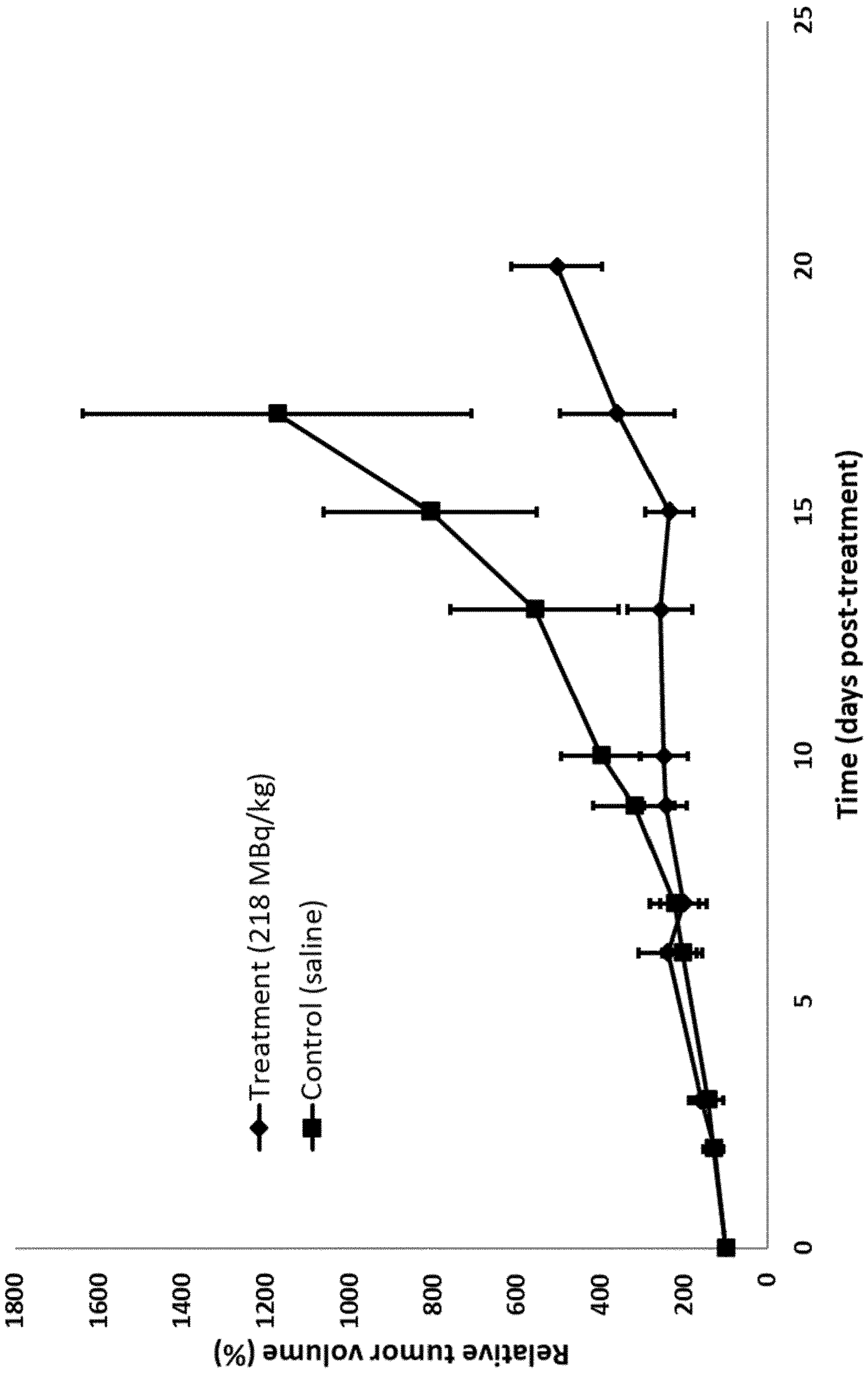
Treatment (218 MBq/kg)
Control (saline)
Relative tumor volume (%)
0
200
400
600
800
1000
1200
1400
1600
1800
Time (days post-treatment)
0
5
10
15
20
25

Fig. 13

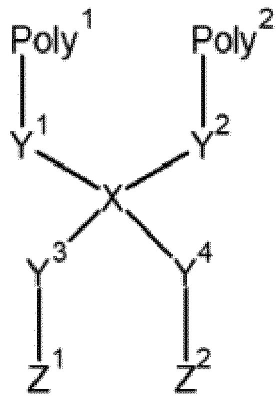

(I)

Europäisches Patentamt
European Patent Office
Office européen des brevets

# EUROPEAN SEARCH REPORT

Application Number
EP 22 16 0866

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/352459 A1 (AXELSSON OSKAR [SE] ET AL) 21 November 2019 (2019-11-21) * example 8; compound 44 * * claims 1-16 * ----- | 1-16 | INV. C08G65/336 A61K49/18 A61K51/06 C09D171/02 |
| A | US 2017/106105 A1 (AXELSSON OSKAR [SE] ET AL) 20 April 2017 (2017-04-20) * examples 1-21 * * figures 1-8 * ----- | 1-16 | |
| A | US 2005/255514 A1 (DE PALMA RANDY [BE] ET AL) 17 November 2005 (2005-11-17) * examples 1-8 * * figures 1-13 * ----- | 1-15 | |

TECHNICAL FIELDS SEARCHED (IPC)

C08G
A61K
C09D

The present search report has been drawn up for all claims

1

| Place of search | Date of completion of the search | Examiner |
|---|---|---|
| The Hague | 25 July 2022 | Popescu, Teodora |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 22 16 0866

This annex lists the patent family members relating to the patent documents cited in the above-mentioned European search report. The members are as contained in the European Patent Office EDP file on
The European Patent Office is in no way liable for these particulars which are merely given for the purpose of information.

25-07-2022

| Patent document cited in search report | Publication date | Patent family member(s) | Publication date |
|---|---|---|---|
| US 2019352459 A1 | 21-11-2019 | AU 2018208538 A1 | 18-07-2019 |
| | | BR 112019014483 A2 | 11-02-2020 |
| | | CA 3048552 A1 | 19-07-2018 |
| | | CN 110191890 A | 30-08-2019 |
| | | DK 3568403 T3 | 12-07-2021 |
| | | EP 3348560 A1 | 18-07-2018 |
| | | EP 3568403 A1 | 20-11-2019 |
| | | ES 2880228 T3 | 24-11-2021 |
| | | IL 267895 A | 26-09-2019 |
| | | JP 7078274 B2 | 31-05-2022 |
| | | JP 2020507559 A | 12-03-2020 |
| | | KR 20190104598 A | 10-09-2019 |
| | | PL 3568403 T3 | 08-11-2021 |
| | | RU 2019125812 A | 16-02-2021 |
| | | US 2019352459 A1 | 21-11-2019 |
| | | WO 2018130713 A1 | 19-07-2018 |
| US 2017106105 A1 | 20-04-2017 | AU 2015238208 A1 | 29-09-2016 |
| | | CA 2943852 A1 | 01-10-2015 |
| | | CN 106232146 A | 14-12-2016 |
| | | DK 3122383 T3 | 08-04-2019 |
| | | EP 2923712 A1 | 30-09-2015 |
| | | EP 3122383 A1 | 01-02-2017 |
| | | ES 2715885 T3 | 06-06-2019 |
| | | JP 6707074 B2 | 10-06-2020 |
| | | JP 2017509708 A | 06-04-2017 |
| | | KR 20160137634 A | 30-11-2016 |
| | | PL 3122383 T3 | 30-08-2019 |
| | | TR 201903815 T4 | 22-04-2019 |
| | | US 2017106105 A1 | 20-04-2017 |
| | | WO 2015144891 A1 | 01-10-2015 |
| US 2005255514 A1 | 17-11-2005 | AT 477490 T | 15-08-2010 |
| | | EP 1607743 A1 | 21-12-2005 |
| | | US 2005255514 A1 | 17-11-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018130713 A
- WO 2021122859 A
- EP 2572736 A
- US 2005255514 A


### Non-patent literature cited in the description


- **TANIKAGA,R. et al.** *Synthesis,* 1977, 299-301